(19) Europäisches Patentamt
European Patent Office
Office européen des brevets

(11) EP 1 438 962 A1

(12) **EUROPEAN PATENT APPLICATION**
published in accordance with Art. 158(3) EPC

(43) Date of publication:
**21.07.2004 Bulletin 2004/30**

(21) Application number: **02705299.2**

(22) Date of filing: **18.03.2002**

(51) Int Cl.7: **A61K 31/499**, A61K 31/5377, A61K 45/00, C07D 471/10, A61P 43/00

(86) International application number:
**PCT/JP2002/002552**

(87) International publication number:
**WO 2003/035074 (01.05.2003 Gazette 2003/18)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **23.10.2001 JP 2001324435**

(71) Applicant: **ONO PHARMACEUTICAL CO., LTD.**
**Osaka-shi, Osaka 541-8526 (JP)**

(72) Inventors:
- **IMAWAKA, Haruo,**
  **c/o Ono Pharmaceutical Co., Ltd.**
  **Mishima-gun, Osaka 618-8585 (JP)**
- **SHIBAYAMA, Shiro,**
  **c/o Ono Pharmaceutical Co., Ltd.**
  **Mishima-gun, Osaka 618-8585 (JP)**
- **TAKAOKA, Y., c/o Ono Pharmaceutical Co., Ltd.**
  **Mishima-gun, Osaka 618-8585 (JP)**

(74) Representative: **Henkel, Feiler & Hänzel**
**Möhlstrasse 37**
**81675 München (DE)**

(54) **DRUGS COMPRISING COMBINATION OF TRIAZASPIRO 5,5 U NDECANE DERIVATIVE WITH CYTOCHROME P450 ISOZYME 3A4 INHIBITOR AND/OR P−GLYCOPROTEIN INHIBITOR**

(57) A pharmaceutical composition which comprises a combination of a triazaspiro[5.5]undecane derivative of the formula (I):

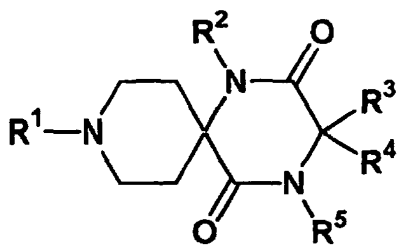

(I)

(wherein all the symbols have the same meanings as defined hereinafter), a quaternary ammonium salt thereof, or an N-oxide thereof, or a non-toxic salt thereof with at least one cytochrome P450 isozyme 3A4 inhibitor and/or at least one P-glycoprotein inhibitor. As a result the pharmaceutical composition comprising the above-described combination, bioavailability of the compound of the formula (I) is enhanced and is able to be effectively utilized as an oral agent in the treatment of various diseases.

EP 1 438 962 A1

**Description**

TECHNICAL FIELD

The present invention relates to

(1) a pharmaceutical composition which comprises, as an active ingredient, a combination of a triazaspiro[5.5] undecane derivative of the formula (I):

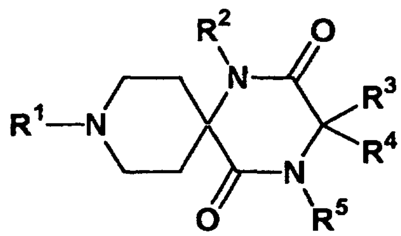

(I)

(wherein all the symbols have the same meanings as defined hereinafter), a quaternary ammonium salt thereof, or an N-oxide thereof, or a non-toxic salt thereof with a cytochrome P450 isozyme 3A4 inhibitor and/or a P-glycoprotein inhibitor; and

(2) a agent for preventing and/or treating various inflammatory diseases, asthma, atopic dermatitis, urticaria, allergic diseases (allergic bronchopulmonary aspergillosis, allergic eosinophilic gastroenteritis), nephritis, nephropathy, hepatitis, arthritis, chronic articular rheumatism, psoriasis, rhinitis, conjunctivitis, ischemia-reperfusion injury, multiple sclerosis, ulcerative colitis, acute respiratory distress syndrome, shock accompanied by bacterial infection, diabetes mellitus, autoimmune disease, transplant rejection, immunosuppression, metastasis of cancer, acquired immune deficiency syndrome and human immune deficiency virus infection, which comprises the combined medicament as an active ingredient.

BACKGROUND ART

Bioavailability (hereinafter referred to as BA) is defined as "relative amount and speed of the drug delivered to systemic circulation blood to the administered drug". Generally, when a drug is orally administered, it is important to evaluate BA and efficacy and safety of the drug are greatly governed by BA.

The orally administered drug is absorbed via epithelial cells from intestinal lumen and comes into systemic circulation blood after passing through the liver. Accordingly, factors for determining BA of the oral preparation include an absorption rate from intestinal lumen to epithelial cells, an avoiding rate of metabolic loss in epithelial cells of intestinal tract and a utilizing rate in the liver. Therefore, in order to increase BA of the oral preparation, it is important to enhance the absorption of the drug and to avoid the first-pass effect in the small intestine and the liver.

In recent years, as the factor which affects during the process where an orally administered drug is absorbed into systemic circulation blood from digestive tracts, participation of metabolism by isozyme 3A4 of cytochrome P450 (hereinafter referred to as CYP 3A4) and taking-out of the drug from epithelial cells to lumen by P-glycoprotein (hereinafter referred to as P-gp) which is a membrane transportation protein has attracted attention. The proteins have similar substrate specificity and are likely to act cooperatively as a barrier for absorption of the drug.

The CYP3A subfamily which is considered to participate in metabolism of more than one half of the medicaments used at present has the biggest expression amount among human P450 and occupies about 30% of P450 of liver and 70% or more of small intestine. Most of the CYP3A subfamily expressed in the liver and small intestine of adults is CYP 3A4. An expressed amount of CYP3A in the small intestine is about 10 to 50% of that in the liver, and both expressed amount and activity lower in the order of upper to lower areas of the digestive tracts.

It is known that, in general, when a plurality of drugs which inhibit CYP 3A4 or are to be substrates for CYP 3A4 are administered, concentration of a specific drug significantly increases due to drug interaction.

In addition, P-gp is an ATP-depending transportation carrier expressing in plasma membrane and, in cancer cells acquiring multidrug resistance, it participates in taking-out of an antitumor agent to the outside of the cells. Furthermore, P-gp is expressed not only in cancer cells but also in many normal tissues such as digestive tracts, kidney and blood-brain barrier and, in the small intestine, it is expressed in membrane of the lumen side of epithelial cells and plays a role of a taking-out of the drug from epithelial cells to the lumen. Therefore, it is suggested that the drug which is to be a substrate for P-gp is excreted from the inner side of cells due to a drug taking-out mechanism by P-gp and prevents the absorption.

From the above facts, JP-A-9-508623 discloses, as a method for enhancing the bioavailability of oral pharmaceutical preparations, a method where a biopotentiating substance containing a pharmaceutical compound and a suppressor for CYP 3A4 or a suppressor for membrane transport mediated by P-gp is cooperatively administered. The specification shows an example where metabolism of cyclosporine A in a human is suppressed by its use in combination with ketoconazole which is a CYP 3A4 inhibitor. However, no other specific example is described therein.

On the other hand, the specification of WO 01/40227 discloses that a compound of the formula (I), a quaternary ammonium salt thereof, or an N-oxide thereof, or a non-toxic salt thereof suppresses the action of chemokine/chemokine receptor (CCR) whereby it is useful for prevention and/or treatment of various inflammatory diseases, asthma, atopic dermatitis, urticaria, allergic diseases (allergic bronchopulmonary aspergillosis, allergic eosinophilic gastroenteritis), nephritis, nephropathy, hepatitis, arthritis, chronic articular rheumatism, psoriasis, rhinitis, conjunctivitis, ischemia-reperfusion injury, multiple sclerosis, ulcerative colitis, acute respiratory distress syndrome, shock accompanied by bacterial infection, diabetes mellitus, autoimmune disease, transplant rejection, immunosuppression, metastasis of cancer and acquired immune deficiency syndrome.

Enhancement of bioavailability of medicaments in clinical applications is very highly significant in both of medical viewpoint and economical viewpoint. The compound of the formula (I) has an oral activity and is a quite useful compound as a preventive and therapeutic agent for various diseases by suppressing the action of CCR. In view of the above, investigation has been carried out for enhancing the bioavailability of the compound of the formula (I) in oral administration.

DISCLOSURE OF THE INVENTION

The present inventors have carried out intensive investigations and confirmed that the compound of the formula (I) is mainly metabolized by CYP 3A4 and is used as a substrate for P-gp. Also, it has been confirmed that, when the compound of the formula (I) is used together with ketoconazole which is a P-gp inhibitor and a CYP 3A4 inhibitor, the plasma concentration of the compound of the formula (I) increases. Furthermore, it has been confirmed that, when the compound of the formula (I) is used together with cyclosporine A or d-α-tocopheryl polyethylene glycol 1000 succinate (hereinafter referred to as TPGS) which is a P-gp inhibitor, a drug permeability of the compound of the formula (I) increases.

Thus, it has been confirmed that, when the compound of the formula (I) is administered in combination with at least one CYP 3A4 inhibitor and/or at least one P-gp inhibitor, the bioavailability of the compound of the formula (I) can be enhanced, and thus the present invention has been completed. These facts have been confirmed by the present inventors for the first time. Thus, the compound of the formula (I) has been able to be effectively utilized as an oral preparation in the treatment of the above-described various diseases.

Particularly, AIDS which is caused by HIV infection is one of diseases for which the treating method has been most briskly demanded in recent years. In the anti-HIV drugs which have been commercially available at present, there are three kinds, i.e. HIV protease inhibitors, nucleoside reverse transcriptase inhibitors and non-nucleoside reverse transcriptase inhibitors. In the anti-HIV therapy, single-drug therapy turned to combination therapy using two preparations and, further, there has been a change to combination therapy using three or four preparations. The principle of the anti-HIV therapy at present is that, in an early stage before virus acquires a drug resistance, combination therapy is carried out on a continuous basis. It is the recent finding that, although it is not possible to suppress the virus completely by that therapy, growth of the virus is suppressed as much as possible whereby it can be expected that decrease of CD4-positive cells is prevented and that immunosuppression function is maintained. in addition, a strong suppression of growth of the virus is important for the prevention of expression of the virus having a drug-resisting mutation.

The compound of the present invention of the formula (I), a quaternary ammonium salt thereof, or an N-oxide thereof, or a non-toxic salt thereof controls the action of CCR to thereby treat and/or HIV infection. At present, no anti-HIV drug which suppresses HIV infection by the above-described mechanism has been commercially available yet. Accordingly, it also can be used for the patients suffering from HIV to whom other preventive and/or treating agent for HIV infection is no longer effective.

The present inventors have confirmed that, when the compound of the formula (I) and an HIV protease inhibitor are used together, the plasma concentration of the compound of the formula (I) increases. Thus, the concomitant medicament of the compound of the formula (I) and an HIV protease inhibitor can be expected to have an additional/synergistic effect to HIV infection.

From the above, provision of the drug in accordance with the present invention is a very useful fact for establishing an effective method for treating HIV infection.

The present invention relates to

(1) a pharmaceutical composition which comprises, as an active ingredient, a combination of a triazaspiro[5.5]undecane derivative of the formula (I):

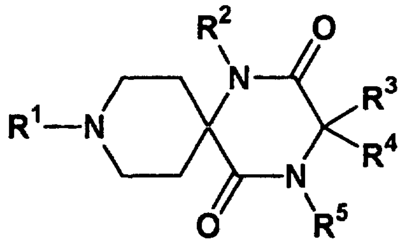

(I)

wherein $R^1$ is

(1) hydrogen,
(2) C1-18 alkyl,
(3) C2-18 alkenyl,
(4) C2-18 alkynyl,
(5) $-COR^6$,
(6) $-CONR^7R^8$,
(7) $-COOR^9$,
(8) $-SO_2R^{10}$,
(9) $-COCOOR^{11}$,
(10) $-CONR^{12}COR^{13}$,
(11) Cyc1, or
(12) C1-18 alkyl, C2-18 alkenyl or C2-18 alkynyl substituted by 1-5 of optionally selected from (a) halogen, (b) $-CONR^7R^8$, (c) $-COOR^9$, (d) $-OR^{14}$, (e) $-SR^{15}$, (f) $-NR^{16}R^{17}$, (g) $-NR^{18}BCOR^{19}$, (h) $-SO_2NR^{20}R^{21}$, (i) $-OCOR^{22}$, (j) $-NR^{23}SO_2R^{24}$, (k) $-NR^{25}COOR^{26}$, (l) $-NR^{27}CONR^{28}R^{29}$, (m) Cyc1, (n) keto or (o) $-N(SO_2R^{24})_2$,

$R^6$-$R^9$, $R^{11}$-$R^{21}$, $R^{23}$, $R^{25}$ and $R^{27}$-$R^{29}$ are each independently,

(1) hydrogen,
(2) C1-8 alkyl,
(3) C2-8 alkenyl,
(4) C2-8 alkynyl,
(5) Cyc1, or
(6) C1-8 alkyl, C2-8 alkenyl or C2-8 alkynyl substituted by 1-5 of optionally selected from (a) Cyc1, (b) halogen, (c) $-OR^{30}$, (d) $-SR^{31}$, (e) $-NR^{32}R^{33}$, (f) $-COOR^{34}$, (g) $-CONR^{35}R^{36}$, (h) $-NR^{37}$ $COR^{38}$, (i) $-NR^{39}SO_2R^{40}$ or (j) $-N(SO_2R^{40})2$, or

$R^7$ and $R^8$, $R^{20}$ and $R^{21}$, $R^{28}$ and $R^{29}$, taken together, are 1) C2-6 alkylene, 2) - (C2-6 alkylene)-O-(C2-6 alkylene)-, 3) -(C2-6 alkylene)-S-(C2-6 alkylene)-, or 4) -(C2-6 alkylene)-$NR^{195}$-(C2-6 alkylene)-, wherein $R^{195}$ is hydrogen, C1-8 alkyl, phenyl or C1-8 alkyl substituted by phenyl,
$R^{10}$, $R^{22}$, $R^{24}$ and $R^{26}$ are each independently,

(1) C1-8 alkyl,
(2) C2-8 alkenyl,
(3) C2-8 alkynyl,
(4) Cyc1, or
(5) C1-8 alkyl, C2-8 alkenyl or C2-8 alkynyl substituted by 1-5 of optionally selected from (a) Cyc1, (b) halogen, (c) $-OR^{30}$, (d) $-SR^{31}$, (e) $-NR^{32}R^{33}$, (f) $-COOR^{34}$, (g) $-CONR^{35}R^{36}$, (h) $-NR^{37}COR^{38}$, (i) $-NR^{39}SO_2R^{40}$ or (j) $-N(SO_2R^{40})_2$,

$R^{30}$-$R^{37}$ and $R^{39}$ are each independently, hydrogen, C1-8 alkyl, Cyc1 or C1-8 alkyl substituted by Cyc1, or
$R^{35}$ and $R^{36}$, taken together, are 1) C2-6 alkylene, 2) -(C2-6 alkylene)-O-(C2-6 alkylene)-, 3) -(C2-6 alkylene) -S-(C2-6 alkylene)- or 4) -(C2-6 alkylene)-$NR^{196}$-(C2-6 alkylene)-, wherein $R^{196}$ is hydrogen, C1-8 alkyl, phenyl or C1-8 alkyl substituted by phenyl,
$R^{38}$ and $R^{40}$ are each independently C1-8 alkyl, Cyc1 or C1-8 alkyl substituted by Cyc1,
Cyc1 is C3-15 mono-, bi- or tri-(fused or spiro)carbocyclic ring or 3-15 membered mono-, bi- or tri-(fused or spiro)cyclic hetero ring containing 1-4 nitrogen atoms, 1-3 oxygen atoms and/or 1-3 sulfur atoms, wherein Cyc1 may be optionally substituted by 1-5 of $R^{51}$,

$R^{51}$ is

(1) C1-8 alkyl,
(2) C2-8 alkenyl,
(3) C2-8 alkynyl,
(4) halogen,
(5) nitro,
(6) trifluoromethyl,
(7) trifluoromethoxy,
(8) nitrile,
(9) keto,
(10) Cyc2
(11) $-OR^{52}$,
(12) $-SR^{53}$,
(13)$-NR^{54}R^{55}$,
(14) $-COOR^{56}$,
(15)$-CONR^{57}R^{58}$,
(16)$-NR^{59}COR^{60}$,
(17)$-SO_2NR^{61}R^{62}$,
(18) $-OCOR^{63}$,
(19)$-NR^{64}SO_2R^{65}$,
(20) $-NR^{66}COOR^{67}$,
(21)$-NR^{68}CONR^{69}R^{70}$,
(22) $-B(OR^{71})_2$,
(23) $-SO_2R^{72}$,
(24) $-N(SO_2R^{72})_2$,
(25) $-S(O)R^{72}$, or
(26) C1-8 alkyl, C2-8 alkenyl or C2-8 alkynyl substituted by 1-5 of optionally selected from (a) halogen, (b) Cyc2, (c) $-OR^{52}$, (d) $-SR^{53}$, (e) $-NR^{54}R^{55}$, (f) $-COOR^{56}$, (g) $-CONR^{57}R^{58}$, (h) $-NR^{59}COR^{60}$, (i) $-SO_2NR^{61}R^{62}$, (j) $-OCOR^{63}$, (k) $-NR^{64}SO_2R^{65}$, (l) $-NR^{66}COOR^{67}$, (m) $-NR^{68}CONR^{69}R^{70}$, (n) $-B(OR^{71})_2$, (o) $-SO_2R^{72}$, (p) $-N(SO_2R^{72})_2$, (q) $-S(O)R^{72}$, or (r) keto,

$R^{52}$-$R^{62}$, $R^{64}$, $R^{66}$ and $R^{68}$-$R^{71}$ are each independently 1) hydrogen, 2) C1-8 alkyl, 3) C2-8 alkenyl, 4) C2-8 alkynyl, 5) Cyc2, 6) $-OR^{73}$, or 7) C1-8 alkyl, C2-8 alkenyl or C2-8 alkynyl substituted by Cyc2, $-OR^{73}$, $-COOR^{74}$ or $-NR^{75}R^{76}$, or

$R^{57}$ and $R^{58}$, $R^{61}$ and $R^{62}$, $R^{69}$ and $R^{70}$, taken together, are 1) C2-6 alkylene, 2) -(C2-6 alkylene)-O-(C2-6 alkylene)-, 3) -(C2-6 alkylene)-S-(C2-6 alkylene)-, or 4) -(C2-6 alkylene)-$NR^{197}$-(C2-6 alkylene)-, wherein $R^{197}$ is hydrogen, C1-8 alkyl, phenyl or C1-8 alkyl substituted by phenyl,

$R^{63}$, $R^{65}$, $R^{67}$ and $R^{72}$ are each independently 1) C1-8 alkyl, 2) C2-8 alkenyl, 3) C2-8 alkynyl, 4) Cyc2 or 5) C1-8 alkyl, C2-8 alkenyl or C2-8 alkynyl substituted by Cyc2, $-OR^{73}$, $-COOR^{74}$ or $-NR^{75}R^{76}$,

$R^{73}$-$R^{76}$ are independently hydrogen, C1-8 alkyl, Cyc2 or C1-8 alkyl substituted by Cyc2,

Cyc2 has the same meaning as Cyc1, wherein Cyc2 may be optionally substituted by 1-5 of $R^{77}$,

$R^{77}$ is

1) C1-8 alkyl,
2) halogen,
3) nitro,
4) trifluoromethyl,
5) trifluoromethoxy,
6) nitrile,
7) $-OR^{78}$,
8) $-NR^{79}R^{80}$,
9) $-COOR^{81}$,
10) $-SR^{82}$,
11) $-CONR^{83}R^{84}$,
12) C2-8 alkenyl,
13) C2-8 alkynyl,
14) keto,

15) Cyc6,
16) $-NR^{161}COR^{162}$,
17) $-SO_2NR^{163}R^{164}$,
18) $-OCOR^{165}$,
19) $-NR^{166}SO_2R^{167}$,
20) $-NR^{168}COOR^{169}$,
21) $-NR^{170}COR^{171}R^{172}$,
22) $-SO_2R^{173}$,
23) $-N(SO_2R^{167})_2$,
24) $-S(O)R^{173}$, or
25) C1-8 alkyl, C2-8 alkenyl or C2-8 alkynyl substituted by 1-5 of optionally selected from (a) halogen, (b) $-OR^{78}$, (c) $-NR^{79}R^{80}$, (d) $-COOR^{81}$, (e) $-SR^{82}$, (f) $-CONR^{83}R^{84}$, (g) keto, (h) Cyc6, (i) $-NR^{161}COR^{162}$, (j) $-SO_2NR^{163}R^{164}$, (k) $-OCOR^{165}$, (l) $-NR^{166}SO_2R^{167}$, (m) $-NR^{168}COOR^{169}$, (n) $-NR^{170}COR^{171}R^{172}$, (o) $-SO_2R^{173}$, (p) $-N(SO_2R^{167})_2$, or (q) $-S(O)R^{173}$,

$R^{78}$-$R^{84}$, $R^{161}$-$R^{164}$, $R^{166}$, $R^{168}$ and $R^{170}$-$R^{172}$ are each independently (a) hydrogen, (b) C1-8 alkyl, (c) C2-8 alkenyl, (d) C2-8 alkynyl, (e) Cyc6, (f) $-OR^{174}$, or (g) C1-8 alkyl, C2-8 alkenyl or C2-8 alkynyl substituted by Cyc6, $-OR^{174}$, $-COOR^{175}$, $-NR^{176}R^{177}$or-$CONR^{178}R^{179}$, or

$R^{83}$ and $R^{84}$, $R^{163}$ and $R^{164}$, $R^{171}$ and $R^{172}$, taken together, are 1) C2-6 alkylene, 2) -(C2-6 alkylene)-O-(C2-6 alkylene)-, 3) -(C2-6 alkylene)-S-(C2-6 alkylene)-or 4) -(C2-6 alkylene)-$NR^{198}$-(C2-6 alkylene)-, wherein $R^{198}$ is hydrogen, C1-8 alkyl, phenyl or C1-8 alkyl substituted by phenyl,

$R^{165}$ , $R^{167}$ , $R^{169}$ and $R^{173}$ are each independently (a) C1-8 alkyl, (b) C2-8 alkenyl, (c) C2-8 alkynyl, (d) Cyc6 or (e) C1-8 alkyl, C2-8 alkenyl, C2-8 alkynyl substituted by Cyc6, $-OR^{174}$, $-COOR^{175}$, $-NR^{176}R^{177}$ or $-CONR^{178}R^{179}$,

$R^{174}$-$R^{177}$ are each independently 1) hydrogen, 2) C1-8 alkyl, 3) Cyc6 or 4) C1-8 alkyl substituted by Cyc6, or

$R^{178}$ and $R^{179}$, taken together, are 1) C2-6 alkylene, 2) -(C2-6 alkylene)-O-(C2-6 alkylene)-, 3) -(C2-6 alkylene)-S-(C2-6 alkylene)- or 4) -(C2-6 alkylene)-$NR^{199}$-(C2-6 alkylene)-, wherein $R^{199}$ is hydrogen, C1-8 alkyl, phenyl or C1-8 alkyl substituted by phenyl,

Cyc6 is C3-8 mono-carbocyclic ring or 3-8 membered mono-cyclic hetero ring containing 1-4 nitrogen atoms, 1-2 oxygen atoms and/or 1-2 sulfur atoms, wherein Cyc6 may be optionally substituted by 1-5 of $R^{180}$,

$R^{180}$ is,

(1) C1-8 alkyl,
(2) halogen,
(3) nitro,
(4) trifluoromethyl,
(5) trifluoromethoxy,
(6) nitrile,
(7) $-OR^{181}$,
(8) $-NR^{182}R^{183}$,
(9) $-COOR^{184}$,
(10) $-SR^{185}$, or
(11) $-CONR^{186}R^{187}$,

$R^{181}$-$R^{187}$ are each independently 1) hydrogen, 2) C1-8 alkyl, 3) phenyl or 4) C1-8 alkyl substituted by phenyl,

$R^{182}$ and $R^{183}$, $R^{186}$ and $R^{187}$, taken together, are 1) C2-6 alkylene, 2) -(C2-6 alkylene)-O-(C2-6 alkylene)-, 3) -(C2-6 alkylene)-S-(C2-6 alkylene)- or 4)-(C2-6 alkylene)-$NR^{200}$-(C2-6 alkylene)-, wherein $R^{200}$ is hydrogen, C1-8 alkyl, phenyl or C1-8 alkyl substituted by phenyl;

$R^2$ is

(1) hydrogen,
(2) C1-8 alkyl,
(3) C2-8 alkenyl,
(4) C2-8 alkynyl,
(5) $-OR^{90}$,
(6) Cyc3, or
(7) C1-8 alkyl, C2-8 alkenyl or C2-8 alkynyl substituted by 1-5 of optionally selected from (a) halogen, (b) $-OR^{90}$, (c) $-SR^{91}$, (d) $-NR^{92}R^{93}$, (e) $-COOR^{94}$, (f) $-CONR^{95}R^{96}$, (g) $-NR^{97}COR^{98}$, (h) $-SO_2NR^{99}R^{100}$, (i) $-OCOR^{101}$, (j) $-NR^{102}SO_2R^{103}$, (k) $-NR^{104}COOR^{105}$, (l) $-NR^{106}CONR^{107}R^{108}$, (m) Cyc3, (n) keto or (o) -N

$(SO_2R^{103})_2$,

$R^{90}$-$R^{100}$, $R^{102}$, $R^{104}$ and $R^{106}$-$R^{108}$ are each independently 1) hydrogen, 2) C1-8 alkyl, 3) C2-8 alkenyl, 4) C2-8 alkynyl, 5) Cyc3 or 6) C1-8 alkyl, C2-8 alkenyl or C2-8 alkynyl substituted by Cyc3, or

$R^{95}$ and $R^{96}$, $R^{99}$ and $R^{100}$, $R^{107}$ and $R^{108}$, taken together, are 1) C2-6 alkylene, 2) -(C2-6 alkylene)-O-(C2-6 alkylene)-, 3) -(C2-6 alkylene)-S-(C2-6 alkylene)-or 4) -(C2-6 alkylene)-$NR^{201}$-(C2-6 alkylene)-,

$R^{201}$ is hydrogen, C1-8 alkyl, phenyl or C1-8 alkyl substituted by phenyl,

$R^{101}$, $R^{103}$ and $R^{105}$ are each independently 1) C1-8 alkyl, 2) C2-8 alkenyl, 3) C2-8 alkynyl or 4) Cyc3, or C1-8 alkyl, C2-8 alkenyl or C2-8 alkynyl substituted by Cyc3,

Cyc3 has the same meaning as Cyc1, wherein Cyc3 may be optionally substituted by 1-5 of $R^{109}$,

$R^{109}$ has the same meaning as $R^{51}$;

$R^3$ and $R^4$ are each independently

(1) hydrogen,
(2) C1-8 alkyl,
(3) C2-8 alkenyl,
(4) C2-8 alkynyl,
(5) $-COOR^{120}$,
(6) $-CONR^{121}R^{122}$,
(7) Cyc4, or
(8) C1-8 alkyl, C2-8 alkenyl or C2-8 alkynyl substituted by 1-5 of optionally selected from (a) halogen, (b) nitrile, (c) Cyc4, (d) $-COOR^{120}$, (e) $-CONR^{121}R^{122}$, (f) $-OR^{123}$ (g) $-SR^{124}$, (h) $-NR^{125}R^{126}$, (i) $-NR^{127}COR^{128}$, (j) $-SO_2NR^{129}R^{130}$, (k) $-OCOR^{131}$, (l) $-NR^{132}SO_2R^{133}$, (m) $-NR^{134}COOR^{135}$, (n) $-NR^{136}CONR^{137}R^{138}$, (o) $-S-SR^{139}$, (p) $-NHC(=NH)NHR^{140}$, (q) keto, (r) $-NR^{145}CONR^{146}COR^{147}$ or (s) $-N(SO_2R^{133})_2$,

$R^{120}$-$R^{130}$, $R^{132}$, $R^{134}$, $R^{136}$-$R^{138}$, $R^{145}$ and $R^{146}$ are each independently 1) hydrogen, 2) C1-8 alkyl, 3) C2-8 alkenyl, 4) C2-8 alkynyl, 5) Cyc4 or 6) C1-8 alkyl, C2-8 alkenyl or C2-8 alkynyl substituted by Cyc4, halogen, $-OR^{148}$, $-SR^{149}$, $-COOR^{150}$ or $-NHCOR^{141}$,

$R^{121}$ and $R^{122}$, $R^{129}$ and $R^{130}$, $R^{137}$ and $R^{138}$, taken together, are 1) C2-6 alkylene, 2) -(C2-6 alkylene) -O-(C2-6 alkylene)-, 3) -(C2-6 alkylene)-S-(C2-6 alkylene)-or 4) -(C2-6 alkylene)-$NR^{202}$-(C2-6 alkylene)-, wherein $R^{202}$ is hydrogen, C1-8 alkyl, phenyl or C1-8 alkyl substituted by phenyl,

$R^{131}$, $R^{133}$, $R^{135}$, $R^{139}$ and $R^{147}$ are each independently 1) C1-8 alkyl, 2) C2-8 alkenyl, 3) C2-8 alkynyl, 4) Cyc4 or 5) C1-8 alkyl, C2-8 alkenyl or C2-8 alkynyl substituted by Cyc4, halogen, $-OR^{148}$, $-SR^{149}$, $-COOR^{150}$ or $-NHCOR^{141}$,

$R^{140}$ is hydrogen, $-COOR^{142}$ or $-SO_2R^{143}$,

$R^{141}$-$R^{143}$ are each independently 1) C1-8 alkyl, 2) C2-8 alkenyl, 3) C2-8 alkynyl, 4) Cyc4 or 5) C1-8 alkyl, C2-8 alkenyl or C2-8 alkynyl substituted by Cyc4,

$R^{148}$-$R^{150}$ are each independently 1) hydrogen, 2) C1-8 alkyl, 3) C2-8 alkenyl, 4) C2-8 alkynyl, 5) Cyc4 or 6) C1-8 alkyl, C2-8 alkenyl or C2-8 alkynyl substituted by Cyc4,

Cyc4 has the same meaning as Cyc1, wherein Cyc4 may be optionally substituted by 1-5 of $R^{144}$,

$R^{144}$ has the same meaning as $R^{51}$, or

$R^3$ and $R^4$, taken together, are

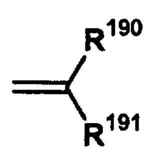

wherein $R^{190}$ and $R^{191}$ are each independently the same meaning as $R^3$ or $R^4$;

$R^5$ is

(1) hydrogen,
(2) C1-8 alkyl,
(3) Cyc5, or
(4) C1-8 alkyl substituted by Cyc5

wherein Cyc5 has the same meaning as Cyc1, and wherein Cyc5 is optionally substituted by 1-5 of $R^{160}$,

$R^{160}$ has the same meaning as $R^{51}$,
a quaternary ammonium salt thereof, an N-oxide thereof, or a non-toxic salt thereof, and
at least one of cytochrome P 450 isozyme inhibitors and / or at least one of P glycoproteins,

(2) the pharmaceutical composition comprising the combination wherein the triazaspiro[5.5]undecane derivative, a quaternary ammonium salt thereof, or an N-oxide thereof, or a non-toxic salt thereof and at least one cytochrome P450 isozyme 3A4 inhibitor and/or at least one P-glycoprotein inhibitor as active ingredients are administered as separate pharmaceutical agents,

(3) the pharmaceutical composition comprising the combination according to (1), wherein the triazaspiro[5.5]undecane derivative, a quaternary ammonium salt thereof, or an N-oxide thereof, or a non-toxic salt thereof and at least one cytochrome P450 isozyme 3A4 inhibitor and/or at least one P-glycoprotein inhibitor as active ingredients are contained in a single pharmaceutical preparation, and

(4) an agent for preventing and/or treating various inflammatory diseases, asthma, atopic dermatitis, urticaria, allergic diseases (allergic bronchopulmonary aspergillosis, allergic eosinophilic gastroenteritis), nephritis, nephropathy, hepatitis, arthritis, chronic articular rheumatism, psoriasis, rhinitis, conjunctivitis, ischemia-reperfusion injury, multiple sclerosis, ulcerative colitis, acute respiratory distress syndrome, shock accompanied by bacterial infection, diabetes mellitus, autoimmune disease, transplant rejection, immunosuppression, metastasis of cancer and acquired immune deficiency syndrome, which comprises the pharmaceutical composition described in above (1).

Among the compounds of the present invention of the formula (I), preferred compounds are those which are concretely specified (as specific examples or Examples) in each of the specifications of WO 01/40227, Japanese Patent Application No. 2001-079610 and Japanese Patent Application No. 2001-160251.

Particularly preferred compounds are the following compounds from Compound 1 to Compound 96, quaternary ammonium salts thereof, and N-oxides thereof, and non-toxic salts thereof. In the following compound names, R* and S* do not mean absolute positions but just mean relative positions.

The particularly preferred compounds of the present invention are

<u>Compound 1</u>

9-(3,5-dimethyl-1-phenylpyrazol-4-ylmethyl)-2,5-dioxo-3-(2-methyl-1-propyl)-1-propyl-1,4,9-triazaspiro[5.5] undecane

<u>Compound 2(1)</u>

9-(1,4-benzodioxan-6-ylmethyl)-1-butyl-3-cyclohexylmethyl-2,5-dioxo-1,4,9-triazaspiro[5.5]undecane

<u>Compound 2(2)</u>

1-butyl-3-cyclohexylmethyl-2,5-dioxo-9-(2-phenylimidazol-4-ylmethyl)-1,4,9-triazaspiro[5.5]undecane

<u>Compound 2(3)</u>

1-butyl-3-(2-methyl-1-propyl)-2,5-dioxo-9-(4-phenyloxyphenylmethyl)-1,4,9-triazaspiro[5.5]undecane

<u>Compound 2(4)</u>

(3S)-2,5-dioxo-3-(2-methylpropyl)-9-(6-phenylhexyl)-1-propyl-1,4,9-triazaspiro[5.5]undecane

<u>Compound 2(5)</u>

(3R)-2,5-dioxo-3-(2-methylpropyl)-9-(6-phenylhexyl)-1-propyl-1,4,9-triazaspiro[5.5]undecane

<u>Compound 3(1)</u>

1-butyl-9-((3,5-dimethyl-1-phenyl)-4-pyrazolyl)methyl)-2,5-dioxo-3-(2-methyl-1-propyl)-1,4,9-triazaspiro[5.5] undecane

Compound 3(2)

1-butyl-3-cyclohexylmethyl-2,5-dioxo-9-(4-phenyloxyphenylmethyl)-1,4,9-triazaspiro[5.5]undecane

Compound 3(3)

9-(1,4-benzodioxan-6-ylmethyl)-1-butyl-3-(2-methyl-1-propyl)-2,5-dioxo-1,4,9-triazaspiro[5.5]undecane

Compound 3(4)

9-(4-benzyloxyphenylmethyl)-1-butyl-2, 5-dioxo-3-(2-methyl-1-propyl)-1,4,9-triazaspiro[5.5]undecane

Compound 4

1-butyl-2,5-dioxo-3-(2-methylpropyl)-9-(6-phenylhexyl)-1,4,9-triazaspiro[5.5]undecane

Compound 5(1)

(3S)-1-(2-methylpropyl)-2,5-dioxo-3-(4-(N-benzyloxycarbonyl)aminobutyl)-9-(2-phenylethyl)-1,4,9-triazaspiro[5.5]undecane

Compound 5(2)

(3S)-1-propyl-2,5-dioxo-3-(4-(N-benzyloxycarbonyl)aminobutyl)-9-(2-phenylethyl)-1,4,9-triazaspiro[5.5]undecane

Compound 5(3)

(3R)-1-propyl-2,5-dioxo-3-(4-(N-benzyloxycarbonyl)aminobutyl)-9-(2-phenylethyl)-1,4,9-triazaspiro[5.5]undecane

Compound 5(4)

(3S)-1-propyl-2,5-dioxo-3-(4-(N-benzyloxycarbonyl)aminobutyl)-9-(3-phenylpropyl)-1,4,9-triazaspiro[5.5]undecane

Compound 5(5)

(3R)-1-propyl-2,5-dioxo-3-(4-(N-benzyloxycarbonyl)aminobutyl)-9-(3-phenylpropyl)-1,4,9-triazaspiro[5.5]undecane

Compound 5(6)

(3S)-1-propyl-2,5-dioxo-3-(4-(N-benzyloxycarbonyl)aminobutyl)-9-(4-phenylbutyl)-1,4,9-triazaspiro[5.5]undecane

Compound 5(7)

(3R)-1-propyl-2,5-dioxo-3-(4-(N-benzyloxycarbonyl)aminobutyl)-9-(4-phenylbutyl)-1,4,9-triazaspiro[5.5]undecane

Compound 5(8)

(3S)-1-benzyl-2,5-dioxo-3-(2-methylpropyl)-9-benzyl-1,4,9-triazaspiro[5.5]undecane

Compound 5(9)

(3R)-1-benzyl-2,5-dioxo-3-(2-methylpropyl)-9-benzyl-1,4,9-triazaspiro[5.5]undecane

Compound 5(10)

(3S)-1-propyl-2,5-dioxo-3-(4-(N-benzyloxycarbonyl)aminobutyl)-9-(2-(2-phenyl-5-methyloxazol-4-yl)ethyl)-1,4,9-triazaspiro[5.5]undecane

Compound 5(11)

(3S)-1-propyl-2,5-dioxo-3-(4-(N-(2-chlorophenylmethyl)oxycarbonyl)aminobutyl)-9-(2-phenylethyl)-1,4,9-triazaspiro[5.5]undecane

Compound 5(12)

(3S)-1-propyl-2,5-dioxo-3-[3-(3-(2,4,6-trimethyl phenylsulfonyl)guanidino) propyl]-9-(2-phenylethyl)-1,4,9-triazaspiro[5.5]undecane

Compound 6(1)

1-propyl-2,5-dioxo-3-(2-methylpropyl)-9-(4-phenyloxyphenylmethyl)-1,4,9-triazaspiro[5.5]undecane

Compound 6(2)

1-butyl-2,5-dioxo-3-cyclohexylmethyl-9-(4-methoxyphenylmethyl)-1,4,9-triazaspiro[5.5]undecane

Compound 6(3)

1-butyl-2,5-dioxo-3-cyclohexylmethyl-9-(4-allyloxyphenylmethyl)-1,4,9-triazaspiro[5.5]undecane

Compound 6(4)

(3S)-1-propyl-9-(3,5-dimethyl-1-phenylpyrazol-4-ylmethyl)-2,5-dioxo-3-(2-methyl-1-propyl)-1,4,9-triazaspiro[5.5]undecane

Compound 6(5)

(3R)-1-propyl-9-(3,5-dimethyl-1-phenylpyrazol-4-ylmethyl)-2,5-dioxo-3-(2-methyl-1-propyl)-1,4,9-triazaspiro[5.5]undecane

Compound 6(6)

1-butyl-2,5-dioxo-3-(2-methylpropyl)-9-phenylmethyl-1,4,9-triazaspiro[5.5]undecane

Compound 6(7)

1-butyl-2, 5-dioxo-3-(2-methylpropyl)-9-benzyloxycarbonyl-1,4,9-triazaspiro[5.5]undecane

Compound 6(8)

1-butyl-2,5-dioxo-3-cyclohexylmethyl-9-benzyloxycarbonyl-1,4,9-triazaspiro[5.5]undecane

Compound 6(9)

1-benzyl-2,5-dioxo-3-(2-methylpropyl)-9-(4-phenyloxyphenylmethyl)-1,4,9-triazaspiro[5.5]undecane

Compound 6(10)

1-butyl-2, 5-dioxo-3-propyl-9-(4-phenyloxyphenylmethyl)-1,4,9-triazaspiro[5.5]undecane

Compound 6(11)

1-butyl-2,5-dioxo-3-methoxymethyl-9-(4-phenyloxyphenylmethyl)-1,4,9-triazaspiro[5.5]undecane

Compound 6(12)

1-(1-methylpropyl)-2,5-dioxo-3-(2-methylpropyl)-9-(4-phenyloxyphenylmethyl)-1,4,9-triazaspiro[5.5]undecane

Compound 6(13)

1-(2-methylbutyl)-2,5-dioxo-3-(2-methylpropyl)-9-(4-phenyloxyphenylmethyl)-1,4,9-triazaspiro[5.5]undecane

Compound 6(14)

1-(2-methylpropyl)-2,5-dioxo-3-(2-methylpropyl)-9-(4-phenyloxyphenylmethyl)-1,4,9-triazaspiro[5.5]undecane

Compound 6(15)

1-(2-dimethylaminoethyl)-2,5-dioxo-3-(2-methylpropyl)-9-(4-phenyloxyphenylmethyl)-1,4,9-triazaspiro[5.5]undecane

Compound 6(16)

1-(2-methoxyethyl)-2, 5-dioxo-3-(2-methylpropyl)-9-(4-phenyloxyphenylmethyl)-1,4,9-triazaspiro[5.5]undecane

Compound 6(17)

1-(2-methylthioethyl)-2,5-dioxo-3-(2-methylpropyl)-9-(4-phenyloxyphenylmethyl)-1,4,9-triazaspiro[5.5]undecane

Compound 6(18)

1-benzyl-2,5-dioxo-3-(2-methylpropyl)-9-(1,4-benzodioxan-6-ylmethyl)-1,4,9-triazaspiro[5.5]undecane

Compound 6(19)

1-benzyl-2,5-dioxo-3-(2-methylpropyl)-9-(4-benzyloxyphenylmethyl)-1,4, 9-triazaspiro[5.5]undecane

Compound 6(20)

1-benzyl-2,5-dioxo-3-(2-methylpropyl)-9-(3,5-dimethyl-1-phenylpyrazol-4-ylmethyl)-1,4,9-triazaspiro[5.5]undecane

Compound 6(21)

1-(3-methylphenylmethyl)-2,5-dioxo-3-(2-methylpropyl)-9-(1,4-benzodioxan-6-ylmethyl)-1,4,9-triazaspiro[5.5]undecane

Compound 6(22)

1-(3-methylphenylmethyl)-2,5-dioxo-3-(2-methylpropyl)-9-(3,5-dimethyl-1-phenylpyrazol-4-ylmethyl)-1,4,9-triazaspiro[5.5]undecane

Compound 6(23)

1-(1-methylbutyl)-2,5-dioxo-3-(2-methylpropyl)-9-(4-phenyloxyphenylmethyl)-1,4,9-triazaspiro[5.5]undecane

Compound 6(24)

1-(3-methylbutyl)-2, 5-dioxo-3-(2-methylpropyl)-9-(4-phenyloxyphenylmethyl)-1,4,9-triazaspiro[5.5]undecane

Compound 6(25)

1-(2-methoxyphenylmethyl)-2,5-dioxo-3-(2-methylpropyl)-9-((3,5-dimethyl-1-phenyl)-4-pyrazolyl)methyl)-1,4,9-triazaspiro[5.5]undecane

Compound 6(26)

1-(3-methoxyphenylmethyl)-2,5-dioxo-3-(2-methylpropyl)-9-((3,5-dimethyl-1-phenyl)-4-pyrazolyl)methyl)-1,4,9-triazaspiro[5.5]undecane

Compound 6(27)

1-(2-methylphenylmethyl)-2,5-dioxo-3-(2-methylpropyl)-9-(3,5-dimethyl-1-phenylpyrazol-4-ylmethyl)-1,4,9-triazaspiro[5.5]undecane

Compound 6(28)

1-(3-methylphenylmethyl)-2,5-dioxo-3-(2-methylpropyl)-9-(4-phenyloxyphenylrnethyl)-1,4,9-triazaspiro[5.5]undecane

Compound 6(29)

1-butyl-2,5-dioxo-3-cyclohexylmethyl-9-(5-ethylthiophen-2-ylmethyl)-1,4,9-triazaspiro[5.5]undecane

Compound 6(30)

1-butyl-2,5-dioxo-3-cyclohexylmethyl-9-(5-ethylfuran-2-ylmethyl)-1,4,9-triazaspiro[5.5]undecane

Compound 6(31)

(3S)-1-butyl-2,5-dioxo-3-((1R)-1-hydroxy-2-methylpropyl)-9-(4-phenyloxyphenylmethyl)-1,4,9-tnazaspiro[5.5]undecane

Compound 6(32)

(3R)-1-butyl-2,5-dioxo-3-((1S)-1-hydroxy-2-methylpropyl)-9-(4-phenyloxyphenylmethyl)-1,4,9-triazaspiro[5.5]undecane

Compound 7

(3S)-1-propyl-2,5-dioxo-3-(4-(N-benzyloxycarbonyl)aminobutyl)-9-allyloxycarbonyl-1,4,9-triazaspiro[5.5]undecane

Compound 8

(3S)-1-propyl-2,5-dioxo-3-(4-(N-benzyloxycarbonyl)aminobutyl)-1,4,9-triazaspiro[5.5]undecane

Compound 8(1)

1-propyl-2,5-dioxo-3-(2-methylpropyl)-1,4,9-triazaspiro[5.5]undecane

Compound 9

1-butyl-2,5-dioxo-3-(2-methylpropyl)-1,4, 9-triazaspiro[5.5]undecane

Compound 9(1)

1-butyl-2,5-dioxo-3-cyclohexylmethyl-1,4,9-triazaspiro[5.5]undecane

Compound 10

(3S)-1-propyl-2,5-dioxo-3-(4-(N-benzyloxycarbonyl)aminobutyl)-9-(4-dihydroxyboranephenylmethyl)-1,4,9-triazaspiro[5.5]undecane

Compound 10(1)

(3S)-1-propyl-2,5-dioxo-3-(4-(N-benzyloxycarbonyl)aminobutyl)-9-(1,3-benzodioxolan-4-ylmethyl)-1,4,9-triazaspiro[5.5]undecane

Compound 11

1-butyl-2,5-dioxo-3-(2-methylpropyl)-9-(1-(1,4-benzodioxan-6-yl)ethyl)-1,4,9-triazaspiro[5.5]undecane

Compound 11(1)

1-butyl-2,5-dioxo-3-(2-methylpropyl)-9-(1-(4-phenyloxyphenyl)ethyl)-1,4,9-triazaspiro[5.5]undecane

Compound 12

1-butyl-2,5-dioxo-3-cyclohexytmethyl-9-(1-(1,4-benzodioxan-6-yl)ethyl)-1,4,9-triazaspiro[5.5]undecane

Compound 13

(3S)-1-propyl-2,5-dioxo-3-(4-(N-benzyloxycarbonyl)aminobutyl)-9-allyl-1,4,9-triazaspiro[5.5]undecane

Compound 14

(3S)-1-propyl-2,5-dioxo-3-(4-aminobutyl)-9-phenylethyl-1,4,9-triazaspiro[5.5]undecane

Compound 15

(3S)-1-propyl-2,5-dioxo-3-(4-(N-(4-phenyl)phenylcarbonyl)aminobutyl)-9-phenylethyl-1,4,9-triazaspiro[5.5]undecane

Compound 16

1-butyl-2,5-dioxo-3-cyclohexylmethyl-9-methyl-9-(1-(1,4-benzodioxan-6-yl)ethyl)-1,4,-diaza-9-azoniaspiro[5.5]undecane iodide

Compound 17

(3S)-3-(4-(N-benzyloxycarbonyl)aminobutyl)-2,5-dioxo-9-(2-hydroxy-2-phenylethyl)-1-propyl-1,4,9-triazaspiro[5,5]undecane

Compound 18

(3S)-3-(4-(N-benzyloxycarbonyl)aminobutyl)-2,5-dioxo-9-(2-oxo-2-phenylethyl)-1-propyl-1,4,9-triazaspiro [5.5]undecane

Compound 19

(3S)-1-(2-methylpropyl)-2, 5-dioxo-3-methyl-9-allyloxycarbonyl-1,4,9-triazaspiro[5.5]undecane

Compound 19(1)

(3S)-1-(2-methylpropyl)-2, 5-dioxo-3-methyl-9-(2-phenylethyl)-1,4,9-triazaspiro[5.5]undecane

Compound 19(2)

(3S)-1-(2-methylpropyl)-2,5-dioxo-3-(4-N-benzyloxycarbonyl)aminobutyl)-9-(2-phenylethyl)-1,4,9-triazaspiro [5.5]undecane

Compound 19(3)

(3S)-1-(1-benzylpiperidin-4-yl)-2,5-dioxo-3-methyl-9-(2-phenylethyl)-1,4,9-triazaspiro[5.5]undecane

Compound 19(4)

(3S)-1-(1-benzylpiperidin-4-yl)-2,5-dioxo-3-(4-(N-benzyloxycarbonyl)aminobutyl)-9-(2-phenylethyl)-1,4,9-triazaspiro[5.5]undecane

Compound 19(5)

(3S)-1-(2,2-diphenylpropyl)-2,5-dioxo-3-methyl-9-(2-phenylethyl)-1,4,9-triazaspiro[5.5]undecane

Compound 19(6)

(3S)-1-(2,2-diphenylpropyl)-2,5-dioxo-3-(4-(N-benzyloxycarbonyl)aminobutyl)-9-(2-phenylethyl)-1,4,9-triazaspiro[5.5]undecane

Compound 19(7)

(3S)-1-propyl-2,5-dioxo-3-(4-benzyloxyphenylmethyl)-9-(2-phenylethyl)-1,4,9-triazaspiro[5.5]undecane

Compound 20

(3S)-1-propyl-2,5-dioxo-3-(4-(benzylcarbonylamino)butyl)-9-(2,4,6-trimethoxybenzyl)-1,4,9-triazaspiro[5.5] undecane

Compound 20(1)

(3S)-1-propyl-2,5-dioxo-3-(4-(benzylcarbonylamino)butyl)-9-(2,2-dimethylpropyl)-1,4,9-triazaspiro[5.5]undecane

Compound 21

(3S)-1-propyl-2,5-dioxo-3-(4-(benzylcarbonylamino)butyl)-9-(3-phenylpropanoyl)-1,4,9-triazaspiro[5.5]undecane

Compound 21(1)

(3S)-1-propyl-2,5-dioxo-3-(4-(benzylcarbonylamino)butyl)-9-benzenesulfonyl-1,4,9-triazaspiro[5.5]undecane

Compound 21(2)

(3S)-1-propyl-2,5-dioxo-3-(4-(benzylcarbonylamino)butyl)-9-benzylaminocarbonyl-1,4,9-triazaspiro[5.5]undecane

Compound 22

(3S)-1-propyl-2,5-dioxo-3-(4-(3-phenylpropanoyl)aminobutyl)-9-(2-phenylethyl)-1,4,9-triazaspiro[5.5]undecane

Compound 22(1)

(3S)-1-propyl-2,5-dioxo-3-(4-benzenesulfonylaminobutyl)-9-(2-phenylethyl)-1,4,9-triazaspiro[5.5]undecane

Compound 22(2)

(3S)-1-propyl-2,5-dioxo-3-(4-(N-benzylcarbamoyl)aminobutyl)-9-(2-phenylethyl)-1,4,9-triazaspiro[5.5]undecane

Compound 23

1-cyclopropylmethyl-2,5-dioxo-3-(2-methylpropyl)-9-(4-phenoxyphenyl)-1,3,9-triazaspiro[5.5]undecane

Compound 23(1)

1-(thiophen-2-ylmethyl)-2,5-dioxo-3-(2-methylpropyl)-9-(4-phenoxyphenyl)-1,3,9-triazaspiro[5.5]undecane

Compound 24(1)

(3S)-1-butyl-2,5-dioxo-3-(4-methoxyphenylmethyl)-9-cyclohexylmethyl-1,4, 9-triazaspiro[5.5]undecane

Compound 24(2)

1-butyl-2,5-dioxo-3-(2-methylpropyl)-9-(2-(4-chlorophenyl)thiophen-5-ylmethyl)-1,4, 9-triazaspiro[5.5]undecane

Compound 24(3)

1-butyl-2,5-dioxo-3-(2-methylpropyl)-9-(2-(4-methoxyphenyl)thiophen-5-ylmethyl)-1,4,9-triazaspiro[5.5]undecane

Compound 24(4)

1-((2E)-2-butenyl)-2,5-dioxo-3-(2-methylpropyl)-9-(4-phenyloxyphenylmethyl)-1,4,9-triazaspiro[5.5]undecane

Compound 24(5)

1-(furan-2-ylmethyl)-2,5-dioxo-3-(2-methylpropyl)-9-(4-phenyloxyphenylmethyl)-1,4,9-triazaspiro[5.5]undecane

Compound 24(6)

1-(thiophen-2-ylmethyl)-2,5-dioxo-3-(2-methylpropyl)-9-(4-phenyloxyphenylmethyl)-1,4,9-triazaspiro[5.5]undecane

Compound 24(7)

1-cyclopropylmethyl-2,5-dioxo-3-(2-methylpropyl)-9-(4-phenyloxyphenylmethyl)-1,4,9-triazaspiro[5.5]undecane

Compound 24(8)

1-(2-fluorophenylmethyl)-2,5-dioxo-3-{2-methylpropyl)-9-(4-phenyloxyphenylmethyl)-1,4,9-triazaspiro[5.5] undecane

Compound 24(9)

1-(3-methyl-2-butenyl)-2,5-dioxo-3-(2-methylpropyl)-9-(4-phenyloxyphenylmethyl)-1,4,9-triazaspiro[5.5]undecane

Compound 24(10)

1-butyl-2,5-dioxo-3-(2-methylpropyf)-9-(quinolin-3-ylmethyl)-1,4,9-triazaspiro[5.5]undecane

Compound 24(11)

1-butyl-2, 5-dioxo-3-(benzyloxycarbonylmethyl)-9-(4-phenyloxypheny(methyl)-1,4, 9-triazaspiro[5.5]undecane

Compound 24(12)

1-(3-methy)-2-buteny))-2,5-dioxo-3-cyclohexylmethy)-9-(1,4-benzodioxan-6-ylmethy))-1,4,9-triazaspiro[5.5] undecane

Compound 24(13)

1-butyl-2,5-dioxo-3-cyclohexylmethyl-9-((2E)-3-phenyl-2-propenyl)-1,4,9-triazaspiro[5.5]undecane

Compound 24(14)

(3S)-1-butyl-2,5-dioxo-3-(1,1-dimethylethyl)-9-(4-phenyloxyphenylmethyl)-1,4,9-triazaspiro[5.5]undecane

Compound 24(15)

(3S)-1-butyl-2,5-dioxo-3-(1,1-dimethylethyl)-9-(1,4-benzodioxan-6-ylmethyl)-1,4, 9-triazaspiro[5.5]undecane

Compound 24(16)

1-butyl-2,5-dioxo-3-(2-methylpropyl)-9-(4-methylthiazol-2-y(methyl)-1,4,9-triazaspiro[5.5]undecane

Compound 24(17)

1 -butyl-2,5-dioxo-3-cyclohexylmethyl-9-(5-methylthiazol-2-ylmethyl)- 1,4,9-triazaspiro[5.5]undecane

Compound 24(18)

1-butyl-2, 5-dioxo-3-cyclohexylmethyl-9-(4-methyithiazol-2-ylmethyl)-1,4,9-triazaspiro[5.5]undecane

Compound 24(19)

1-butyl-2,5-dioxo-3-(2-methylpropyl)-9-(5-methylthiazol-2-ylmethyl)-1,4,9-triazaspiro[5.5]undecane

Compound 24(20)

(3R)-1-butyl-2,5-dioxo-3-cyclohexytmethyl-9-(1,4-benzodioxan-6-ylmethyl)-1,4,9-thazaspiro[5.5]undecane

Compound 24(21)

(3S)-1-butyl-2,5-dioxo-3-cyclohexylmethyl-9-(1,4-benzodioxan-6-ylmethyl)-1,4,9-triazaspiro[5.5]undecane

Compound 24(22)

(3R)-1-butyl-2,5-dioxo-3-((1R)-1-methylpropyl)-9-(4-phenyloxyphenylmethyl)-1,4,9-triazaspiro[5.5]undecane

Compound 24(23)

(3S)-1-butyl-2,5-dioxo-3-((1S)-1-methylpropyl)-9-(4-phenyloxyphenylmethyl)-1,4,9-triazaspiro[5.5]undecane

Compound 24(24)

1-(2-butynyl)-2,5-dioxo-3-(2-methylpropyl)-9-(4-phenyloxyphenylmethyl)-1,4,9-triazaspiro[5.5]undecane

Compound 24(25)

1-(2-butynyl)-2,5-dioxo-3-cyctohexylmethyl-9-(1,4-benzodioxan-6-ylmethyl)-1,4, 9-triazaspiro[5.5]undecane

Compound 24(26)

1-pentyl-2,5-dioxo-3-cyclohexylmethyl-9-(1,4-benzodioxan-6-ylmethyl)-1,4,9-triazaspiro[5.5]undecane

Compound 24(27)

1-(3-methoxyphenylmethyl)-2,5-dioxo-3-(benzyloxymethyl)-9-(3,5-dimethyl-1-phenylpyrazol-4-ylmethyl)-1,4,9-triazaspiro[5.5]undecane

Compound 24(28)

(3R)-1-butyl-2,5-dioxo-3-(2-methylpropyl)-9-(4-phenyloxyphenylmethyl)-1,4,9-triazaspiro[5.5]undecane

Compound 24(29)

(3S)-1-butyl-2,5-dioxo-3-(2-methylpropyl)-9-(4-phenyloxyphenylmethyl)-1,4,9-triazaspiro[5.5]undecane

Compound 24(30)

1-butyl-2,5-dioxo-3-cyclopentylmethyl-9-(1,4-benzodioxan-6-ylmethyl)-1,4,9-triazaspiro[5.5]undecane

Compound 24(31)

1 -propyl-2,5-dioxo-3-(cyclohexylmethyloxymethyl)-9-(3,5-dimethyl-1 -phenylpyrazol-4-ylmethyl)-1,4,9-triazaspiro[5.5]undecane

Compound 24(32)

(3S)-1-butyl-2,5-dioxo-3-(1-methylpropyl)-9-(1,4-benzodioxan-6-ylmethyl)-1,4,9-triazaspiro[5.5]undecane

Compound 24(33)

(3R)-1-butyl-2,5-dioxo-3-(1-methylpropyl)-9-(1,4-benzodioxan-6-ylmethyl)-1,4,9-triazaspiro[5.5]undecane

Compound 24(34)

1-butyl-2,5-dioxo-3-(2-methylpropyl)-9-(5-phenylmethylthiophen-2-ylmethyl)-1,4,9-triazaspiro[5.5]undecane

Compound 24(35)

1-butyl-2,5-dioxo-3-cyclohexylmethyl-9-(2-phenylmethylthiophen-5-ylmethyl)-1,4,9-triazaspiro[5.5]undecane

Compound 24(36)

(3R)-1-butyl-2,5-dioxo-3-(2,2-dimethylpropyl)-9-(1,4-benzodioxan-6-ylmethyl)-1,4,9-triazaspiro[5.5]undecane

Compound 24(37)

(3S)-1-butyl-2,5-dioxo-3-(2,2-dimethylpropyl)-9-(1,4-benzodioxan-6-ylmethyl)-1,4,9-triazaspiro[5.5]undecane

Compound 24(38)

(3R)-1-(2-butynyl)-2,5-dioxo-3-(2,2-dimethylpropyl)-9-(4-phenyloxyphenylmethyl)-1,4,9-triazaspiro[5.5]undecane

Compound 24(39)

(3S)-1-(2-butynyl)-2,5-dioxo-3-(2,2-dimethylpropyl)-9-(4-phenyloxyphenylmethyl)-1,4,9-triazaspiro[5.5]undecane

Compound 24(40)

1-butyl-2,5-dioxo-3-cycloheptylmethyl-9-(1,4-benzodioxan-6-ylmethyl)-1,4,9-triazaspiro[5.5]undecane

Compound 24(41)

1-butyl-2,5-dioxo-3-cyclohexylmethyl-9-(2,4,6-trimethoxyphenylmethyl)-1,4,9-triazaspiro[5.5]undecane

Compound 24(42)

1-butyl-2,5-dioxo-3-(3-cyclohexylpropyl)-9-(1,4-benzodioxan-6-ylmethyl)-1,4,9-triazaspiro[5.5]undecane

Compound 24(43)

1-butyl-2,5-dioxo-3-(3-cyclohexylpropyl)-9-(4-phenyloxyphenylmethyl)-1,4,9-triazaspiro[5.5]undecane

Compound 24(44)

1-butyl-2,5-dioxo-3-(3-cyclohexylpropyl)-9-(3,5-dimethyl-1-phenylpyrazol-4-ylmethyl)-1,4,9-triazaspiro[5.5] undecane

Compound 24(45)

1-butyl-2,5-dioxo-3-(2-hydroxy-2-methylpropyl)-9-(4-phenyloxyphenylmethyl)-1,4, 9-triazaspiro[5.5]undecane

Compound 24(46)

1-(2-butynyl)-2,5-dioxo-3-(2-methylpropyl)-9-(3,5-dimethyl-1-phenylpyrazol-4-ylmethyl)-1,4,9-triazaspiro

[5.5]undecane

Compound 24(47)

1-(2-butynyl)-2,5-dioxo-3-cyclohexylmethyl-9-(3,5-dimethyl-1-phenylpyrazol-4-ylmethyl)-1,4,9-triazaspiro [5.5]undecane

Compound 24(48)

1-butyl-2,5-dioxo-3-cyclohexylmethyl-9-(3,5-dimethyl-1-phenylpyrazol-4-ylmethyl)-1,4,9-triazaspiro[5.5]undecane

Compound 24(49)

1-butyl-2,5-dioxo-3-(2-methylpropyl)-9-(2-phenyloxypyridin-3-ylmethyl)-1,4,9-triazaspiro[5.5]undecane

Compound 24(50)

1-butyl-2,5-dioxo-3-cyclohexylmethyl-9-(2-phenyloxypyridin-3-ylmethyl)-1,4,9-triazaspiro[5.5]undecane

Compound 24(51)

1-butyl-2,5-dioxo-3-cyclohexylmethyl-9-(4-methylbenzomorpholin-7-ylmethyl)-1,4,9-triazaspiro[5.5]undecane

Compound 24(53)

1-butyl-2,5-dioxo-3-(2-methylpropyl)-9-(4-(N-methyl-N-phenylamino)phenylmethyl)-1,4,9-triazaspiro[5.5]undecane

Compound 24(54)

1-butyl-2,5-dioxo-3-cyclohexylmethyl-9-(4-(N-methyl-N-phenylamino)phenylmethyl)-1,4,9-triazaspiro[5.5] undecane

Compound 24(55)

1-butyl-2,5-dioxo-3-(2-methylpropyl)-9-(2-(3,5-dimethylpyrazol-1-yl)-5-methoxyphenylmethyl)-1,4,9-triazaspiro[5.5]undecane

Compound 24(56)

1-butyl-2, 5-dioxo-3-cyclohexylmethyl-9-(2-(3, 5-dimethylpyrazol-1-yl)-5-methoxyphenylmethyl)-1,4,9-triazaspiro[5.5]undecane

Compound 24(57)

1-buty)-2,5-dioxo-3-(2-methylpropyl)-9-(3,5-diethyl-1-(4-chlorophenyl)pyrazol-4-ylmethyl)-1,4,9-triazaspiro [5.5]undecane

Compound 24(58)

1-butyl-2,5-dioxo-3-cyclohexylmethyl-9-(3,5-diethyl-1-(4-chlorophenyl)pyrazol-4-ylmethyl)-1,4,9-triazaspiro [5.5]undecane

Compound 24(59)

1-butyl-2,5-dioxo-3-(2-methylpropyl)-9-(6-phenyloxypyridin-3-ylmethyl)-1,4,9-triazaspiro[5.5]undecane

Compound 24(60)

1-butyl-2,5-dioxo-3-cyclohexylmethyl-9-(6-phenyloxypyridin-3-ylmethyl)-1,4,9-triazaspiro[5.5]undecane

Compound 24(61)

1-butyl-2,5-dioxo-3-(2-methylpropyl)-9-(1,3-benzodioxolan-5-ylmethyl)-1,4,9-triazaspiro[5.5]undecane

Compound 24(62)

1-butyl-2,5-dioxo-3-cyclohexylmethyl-9-(1,3-benzodioxolan-5-ylmethyl)-1,4,9-triazaspiro[5.5]undecane

Compound 24(63)

1-butyl-2,5-dioxo-3-cyclohexylmethyl-9-(2-hydroxy 4-methoxyphenyimethyl)-1,4,9-triazaspiro[5.5]undecane

Compound 24(64)

1-butyl-2,5-dioxo-3-cyclohexylmethyl-9-(4-methylthiophenylmethyl)-1,4,9-triazaspiro[5.5]undecane

Compound 24(65)

1-butyl-2,5-dioxo-3-(2-methylpropyl)-9-(4-(N,N-diphenylamino)phenylmethyl)-1,4,9-triazaspiro[5.5]undecane

Compound 24(66)

1-butyl-2,5-dioxo-3-cyclohexylmethyl-9-(4-(N, N-diphenylamino)phenylmethyl)-1,4,9-triazaspiro[5.5]undecane

Compound 24(67)

(3S)-1-(2-butynyl)-2,5-dioxo-3-cyclohexytmethyl-9-(3,5-dimethy)-1-pheny)pyrazo)-4-ylmethyl)-1,4,9-triazaspiro[5.5]undecane

Compound 24(68)

(3S)-1-(2-butynyl)-2,5-dioxo-3-(2-methylpropyl)-9-(3,5-dimethyl-1-phenylpyrazol-4-ylmethyl)-1,4,9-triazaspiro[5.5]undecane

Compound 24(69)

(3S)-1-butyl-2,5-dioxo-3-cyclohexylmethyl-9-(3,5-dimethyl-1-phenylpyrazol-4-ylmethyl)-1,4,9-triazaspiro [5.5]undecane

Compound 24(70)

1-butyl-2,5-dioxo-3-(2-methylpropyl)-9-(3,5-dimethyl-1-(4-methylphenyl)pyrazol-4-ylmethyl)-1,4,9-triazaspiro[5.5]undecane

Compound 24(71)

1-butyl-2,5-dioxo-3-cyclohexylmethyl-9-(3,5-dimethyl-1-(4-methylphenyl)pyrazol-4-ylmethyl)-1,4,9-triazaspiro[5.5]undecane

Compound 24(72)

1-butyl-2,5-dioxo-3-(2-methylpropyl)-9-(3,5-dimethyl-1-(4-chlorophenyl)pyrazol-4-ylmethyl)-1,4,9-triazaspiro

[5.5]undecane

Compound 24(73)

1-butyl-2,5-dioxo-3-cyclohexylmethyl-9-(3,5-dimethyl-1-(4-chlorophenyl)pyrazol-4-ylmethyl)-1,4,9-triazaspiro[5.5]undecane

Compound 24(74)

1-butyl-2,5-dioxo-3-(2-methylpropyl)-9-(3,5-dimethyl-1-(4-trifluoromethylphenyl)pyrazol-4-ylmethyl)-1,4,9-triazaspiro[5.5]undecane

Compound 24(75)

1-butyl-2,5-dioxo-3-cyclohexylmethyl-9-(3,5-dimethyl-1-(4-trifluoromethylphenyl)pyrazol-4-ylmethyl)-1,4,9-triazaspiro[5.5]undecane

Compound 24(76)

1-butyl-2,5-dioxo-3-(2-methylpropyl)-9-(3,5-diethyl-1-phenylpyrazol-4-ylmethyl)-1,4,9-triazaspiro[5.5]undecane

Compound 24(77)

1-butyl-2,5-dioxo-3-cyclohexylmethyl-9-(3,5-diethyl-1-phenylpyrazol-4-ylmethyl)-1,4,9-triazaspiro[5.5]undecane

Compound 24(78)

1-butyl-2, 5-dioxo-3-cyclohexylmethyl-9-(2-phenylthiazol-4-ylmethyl)-1,4,9-triazaspiro[5.5]undecane

Compound 24(79)

1 -butyl-2,5-dioxo-3-(2-methylpropyl)-9-(2-phenylthiazol-4-ylmethyl)-1,4,9-triazaspiro[5.5]undecane

Compound 24(80)

1 -butyl-2,5-dioxo-3-(2-methylpropyl)-9-(2-(1,4-benzodioxan-2-yl)thiazol-4-ylmethyl)-1,4,9-triazaspiro[5.5] undecane

Compound 24(81)

1-butyl-2,5-dioxo-3-(2-methylpropyl)-9-(4-trifluoromethyl-2-(morpholin-1-yl)thiazol-5-ylmethyl)-1,4,9-triazaspiro[5.5]undecane

Compound 24(82)

1-butyl-2,5-dioxo-3-(tetrahydropyran-4-ylmethyl)-9-(3,5-dimethyl-1-phenylpyrazol-4-ylmethyl)-1,4,9-triazaspiro[5.5]undecane

Compound 24(83)

1-butyl-2,5-dioxo-3-(tetrahydropyran-4-ylmethyl)-9-(1,4-benzodioxan-6-ylmethyl)-1,4, 9-triazaspiro[5.5]undecane

Compound 24(84)

1-butyl-2,5-dioxo-3-cyclohexylmethyl-9-(4-carboxyphenylmethyl)-1,4,9-triazaspiro[5.5]undecane

Compound 24(85)

1-butyl-2,5-dioxo-3-(2-cyclohexylethyl)-9-(3,5-dimethyl-1-phenylpyrazol-4-ylmethyl)-1,4,9-triazaspiro[5.5]undecane

Compound 24(86)

1-butyl-2,5-dioxo-3-(2-cyclohexylethyl)-9-(1,4-benzodioxan-6-ylmethyl)-1,4,9-triazaspiro[5.5]undecane

Compound 24(87)

(3R)-1-butyl-2,5-dioxo-3-cyclohexylmethyl-9-(3,5-dimethyl-1 -phenylpyrazol-4-ylmethyl)-1,4,9-triazaspiro[5.5]undecane

Compound 24(88)

1-butyl-2, 5-dioxo-3-(2-methylpropyl)-9-(4-methyl-2-phenylthiazol-5-ylmethyl)-1,4,9-triazaspiro[5.5]undecane

Compound 24(89)

1-butyl-2,5-dioxo-3-(2-methylpropyl)-9-(2-(thiophen-1-yl)thiazol-4-ylmethyl)-1,4,9-triazaspiro[5.5]undecane

Compound 24(90)

1-butyl-2,5-dioxo-3-(2-methylpropyl)-9-(2-(pyridin-4-yl)thiazol-4-ylmethyl)-1,4,9-triazaspiro[5.5]undecane

Compound 24(91)

1-butyl-2,5-dioxo-3-cyclohexylmethyl-9-(3,4-dimethaxyphenylmethyl)-1,4,9-triazaspiro[5.5]undecane

Compound 24(92)

1-butyl-2, 5-dioxo-3-cyclohexylmethyl-9-(3, 5-dimethoxyphenylmethyl)-1,4,9-triazaspiro[5.5]undecane

Compound 24(93)

1-butyl-2,5-dioxo-3-(2-methylpropyl)-9-(5-(pyridin-2-yl)furan-2-ylmethyl)-1,4,9-triazaspiro[5.5]undecane

Compound 24(94)

1-butyl-2,5-dioxo-3-(2-methylpropyl)-9-(5-(pyridin-3-yl)furan-2-ylmethyl)-1,4,9-triazaspiro[5.5]undecane

Compound 24(95)

1-butyl-2,5-dioxo-3-(2-methylpropyl)-9-(4-(3,5-dimethylpyrazol-1-yl)phenylmethyl)-1,4,9-triazaspiro[5.5]undecane

Compound 24(96)

1-butyl-2,5-dioxo-3-(2-methylpropyl)-9-(4-(5-chloropyridin-3-yloxy)phenylmethyl)-1,4,9-triazaspiro[5.5]undecane

Compound 24(97)

1-butyl-2, 5-dioxo-3-(2-methylpropyl)-9-(4-(pyrimidin-2-yloxy)phenylmethyl)-1,4,9-triazaspiro[5.5]undecane

Compound 24(98)

1-butyl-2, 5-dioxo-3-(2-methylpropyl)-9-(4-(pyridin-3-yloxy)phenylmethyl)-1,4,9-triazaspiro[5.5]undecane

Compound 24(99)

1-(2-butynyl)-2,5-dioxo-3-(2-methylpropyt)-9-(3,5-dimethyl-1-(4-methylphenyl)pyrazot-4-ylmethyl)-1,4,9-triazaspiro[5.5]undecane

Compound 24(100)

(3R)-1-(2-butynyl)-2,5-dioxo-3-cyclohexylmethyl-9-(3,5-dimethyl-1-phenylpyrazol-4-ylmethyl)-1,4,9-triazaspiro[5.5]undecane

Compound 24(101)

1-butyl-2,5-dioxo-3-(2-methylpropyl)-9-(4-(4-hydroxyphenyloxy)phenylmethyl)-1,4,9-triazaspiro[5.5]undecane

Compound 24(102)

1-butyl-2,5-dioxo-3-(2-methylpropyl)-9-(4-(pyridin-2-yl)phenylmethyl)-1,4,9-triazaspiro[5.5]undecane

Compound 24(103)

1-butyl-2, 5-dioxo-3-(2-methylpropyl)-9-(4-(pyridin-3-yl)phenylmethyl)-1,4, 9-triazaspiro[5.5]undecane

Compound 24(104)

1-butyl-2,5-dioxo-3-(2-methylpropyl)-9-(3,5-dimethyl-1-(4-carboxyphenyl)pyrazo)-4-ylmethyl)-1,4,9-triazaspiro[5.5]undecane

Compound 24(105)

1-butyl-2,5-dioxo-3-(2-methylpropyl)-9-(4-(pyrazin-2-yloxy)phenylmethyl)-1,4, 9-triazaspiro[5.5]undecane

Compound 24(106)

1-butyl-2,5-dioxo-3-(2-methylpropyl)-9-(4-(4-carboxyphenyl)phenylmethyl)-1,4, 9-triazaspiro[5.5]undecane

Compound 24(107)

1-butyl-2,5-dioxo-3-(2-methylpropyl)-9-(4-(pyridin-4-yl)phenylmethyl)-1,4,9-triazaspiro[5.5]undecane

Compound 24(108)

1-butyl-2, 5-dioxo-3-(2-methylpropyl)-9-(4-(pyridin-2-yloxy)phenylmethyl)-1,4,9-triazaspiro[5.5]undecane

Compound 24(109)

1-butyl-2,5-dioxo-3-cyclohexylmethyl-9-(naphthalen-2-ylmethyl)-1,4,9-triazaspiro[5.5]undecane

Compound 24(110)

1-butyl-2,5-dioxo-3-cyclohexylmethyl-9-(6-methoxynaphthalen-2-ylmethyl)-1,4,9-triazaspiro[5.5]undecane

Compound 24(111)

1-butyl-2,5-dioxo-3-(2-methylpropyl)-9-(4-(4-carboxyphenyloxy)phenylmethyl)-1,4,9-triazaspiro[5.5]undecane

Compound 24(112)

1-butyl-2,5-dioxo-3-(2-methylpropyl)-9-(5-(pyridin-4-yl)furan-2-ylmethyl)-1,4,9-triazaspiro[5.5]undecane

Compound 24(113)

1-butyl-2.5-dioxo-3-cyclopentylmethyl-9-(4-phenyloxyphenylmethyl)-1,4,9-triazaspiro[5.5]undecane

Compound 24(114)

(3R)-1-butyl-2,5-dioxo-3-(2,2-dimethylpropyl)-9-(4-phenyloxyphenylmethyl)-1,4,9-triazaspiro[5.5]undecane

Compound 24(115)

(3S)-1-butyl-2,5-dioxo-3-(2,2-dimethylpropyl)-9-(4-phenyloxyphenylmethyl)-1,4,9-triazaspiro[5.5]undecane

Compound 24(116)

1-butyl-2,5-dioxo-3-cyclohexylmethyl-9-(4-nitrophenylmethyl)-1,4,9-triazaspiro[5.5]undecane

Compound 24(117)

(3R)-1-(tetrahydrofuran-2-ylmethyl)-2,5-dioxo-3-phenylmethyl-9-{4-phenylbutyl)-1,4,9-triazaspiro[5.5]undecane

Compound 24(118)

(3S)-1-(tetrahydrofuran-2-ylmethyl)-2,5-dioxo-3-phenylmethyl-9-(4-phenylbutyl)-1,4,9-triazaspiro[5.5]undecane

Compound 24(119)

(3S)-1-propyl-2,5-dioxo-3-(3-(benzyloxycarbonylamino)propyl)-9-(2-phenylethyl)-1,4, 9-triazaspiro[5.5]undecane

Compound 25

1-butyl-2,5-dioxo-3-(carboxymethyl)-9-(4-phenyloxyphenylmethyl)-1,4,9-triazaspiro[5.5]undecane

Compound 26(1)

1-(3-hydroxybutyl)-2,5-dioxo-3-(2-methylpropyl)-9-(4-phenyloxyphenylmethyl)-1,4,9-triazaspiro[5.5]undecane

Compound 26(2)

1-(3-hydroxypropyl)-2,5-dioxo-3-(2-methylpropyl)-9-(4-phenyloxyphenylmethyl)-1,4,9-triazaspiro[5.5]undecane

Compound 26(3)

1-(2-hydroxybutyl)-2,5-dioxo-3-(2-methylpropyl)-9-(4-phenyloxyphenylmethyl)-1,4,9-triazaspiro[5.5]undecane

Compound 27

1-butyl-2,5-dioxo-3-cyclohexylmethyl-9-(4-aminophenylmethyl)-1,4,9-triazaspiro[5.5]undecane

Compound 28

1-butyl-2,5-dioxo-3-cyclohexylmethyl-9-(4-((4-methylphenyl)sulfonylamino)phenylmethyl)-1,4,9-triazaspiro [5.5]undecane

Compound 28(1)

1-butyl-2,5-dioxo-3-cyclohexylmethyl-9-(4-(phenylcarbonylamino)phenylmethyl)-1,4,9-triazaspiro[5.5]undecane

Compound 29

(3S)-1-butyl-2,5-dioxo-3-benzyloxymethyl-9-benzyloxycarbonyl-1,4,9-triazaspiro[5.5]undecane

Compound 30

(3S)-1-butyl-2,5-dioxo-3-hydroxymethyl-9-benzyloxycarbonyl-1,4,9-triazaspiro[5.5]undecane

Compound 31

(3S)-1-butyl-2,5-dioxo-3-hydroxymethyl-1,4,9-triazaspiro[5.5]undecane

Compound 32(1)

(3S)-1-butyl-2,5-dioxo-3-hydroxymethyl-9-(4-phenyloxyphenylmethyl)-1,4,9-triazaspiro[5.5]undecane

Compound 32(2)

(3S)-1-butyl-2,5-dioxo-3-hydroxymethyl-9-(3,5-dimethyl-1-phenylpyrazol-4-ylmethyl)-1,4,9-triazaspiro[5.5] undecane

Compound 33

1-butyl-2, 5-dioxo-3-(2-methylpropyl)-9-(4-cyclopentyloxyphenylmethyl)-1,4,9-triazaspiro[5.5]undecane

Compound 33(1)

1-butyl-2,5-dioxo-3-cyclohexylmethyl-9-(4-(2-diethylaminoethyloxy)phenylmethyl)-1,4,9-triazaspiro[5.5]undecane

Compound 33(2)

1-butyl-2,5-dioxo-3-cyclohexylmethyl-9-(4-(2-dimethylaminoethyloxy)phenylmethyl)-1,4,9-triazaspiro[5.5] undecane

Compound 33(3)

1-butyl-2,5-dioxo-3-cyclohexylmethyl-9-(4-propyloxyphenylmethyl)-1,4,9-triazaspiro[5.5]undecane

Compound 33(4)

1-(thiophen-2-ylmethyl)-2,5-dioxo-3-cyclohexylmethyl-9-(4-cyclopropylmethyloxyphenylmethyl)-1,4, 9-triazaspiro[5.5]undecane

Compound 33(5)

1-butyl-2, 5-dioxo-3-cyclohexylmethyl-9-(4-cyclopropylmethyloxyphenylmethyl)-1,4,9-triazaspiro[5.5]undecane

Compound 33(6)

1-butyl-2,5-dioxo-3-(2-methylpropyl)-9-(4-cyclopropylmethyloxyphenylmethyl)-1,4,9-triazaspiro[5.5]undecane

Compound 34

1 -butyl-2,5-dioxo-3-cyclohexylmethyl-9-(4-(dimethylamino)phenylmethyl)-1,4,9-triazaspiro[5.5]undecane

Compound 34(1)

1-butyl-2,5-dioxo-3-cyclohexylmethyl-9-(4-(diethylamino)phenylmethyl)-1,4,9-triazaspiro[5.5]undecane

Compound 35

(3S)-1-butyl-2,5-dioxo-3-(2-methylpropyl)-9-benzyloxycarbonyl-1,4,9-triazaspiro[5.5]undecane

Compound 36

(3S)-1-butyl-2,5-dioxo-3-(2-methylpropyl)-1,4,9-triazaspiro[5.5]undecane

Compound 37(1)

(3S)-1-butyl-2,5-dioxo-3-(2-methylpropyl)-9-(5-(3-methyl-4-chlorophenyl)-1-(4-methylphenylmethyl)pyrazol-3-ylmethyl)-1,4,9-triazaspiro[5.5]undecane

Compound 37(2)

(3S)-1-butyl-2,5-dioxo-3-(2-methylpropyl)-9-(4-dimethylaminophenylmethyl)-1,4,9-triazaspiro[5.5]undecane

Compound 37(3)

(3S)-1-butyl-2,5-dioxo-3-(2-methyipropyl)-9-(4-diethylaminophenylmethyl)-1,4,9-triazaspiro[5.5]undecane

Compound 37(4)

(3S)-1-butyl-2,5-dioxo-3-(2-methylpropyl)-9-(4-cyclohexyloxyphenylmethyl)-1,4,9-triazaspiro[5.5]undecane

Compound 37(5)

(3S)-1-bufyl-2,5-dioxo-3-(2-rnethylpropyf)-9-(4-(4-methylphenyloxy)phenylmethyl)-1,4,9-triazaspiro[5.5]undecane

Compound 37(6)

(3S)-1-butyl-2,5-dioxo-3-(2-methylpropyl)-9-(4-(4-methoxyphenyloxy)phenylmethyl)-1,4,9-triazaspiro[5.5]undecane

Compound 37(7)

(3S)-1-butyl-2,5-dioxo-3-(2-methylpropyl)-9-(4-butylphenylmethyl)-1,4,9-triazaspiro[5.5]undecane

Compound 37(8)

(3S)-1-butyl-2,5-dioxo-3-(2-methylpropyl)-9-(4-(2-methylpropyl)phenylmethyl)-1,4,9-triazaspiro[5.5]undecane

Compound 37(9)

(3S)-1-butyl-2,5-dioxo-3-(2-methylpropyl)-9-(4-(4-fluorophenyloxy)phenylmethyl)-1,4, 9-triazaspiro[5.5]undecane

Compound 37(10)

(3S)-1-butyl-2,5-dioxo-3-(2-methylpropyl)-9-(3-hydroxy-4-methoxyphenylmethyl)-1,4,9-triazaspiro[5.5]undecane

Compound 37(11)

(3S)-1-butyl-2,5-dioxo-3-(2-methylpropyl)-9-(2-fluorophenylmethyl)-1,4,9-triazaspiro[5.5]undecane

Compound 37(12)

(3S)-1-butyl-2,5-dioxo-3-(2-methylpropyl)-9-(3-fluorophenylmethyl)-1,4,9-triazaspiro[5.5]undecane

Compound 37(13)

(3S)-1-butyl-2,5-dioxo-3-(2-methylpropyl)-9-(4-fluorophenylmethyl)-1,4,9-triazaspiro[5.5]undecane

Compound 37(14)

(3S)-1-butyl-2,5-dioxo-3-(2-methylpropyl)-9-(2-chlorophenylmethyl)-1,4,9-triazaspiro[5.5]undecane

Compound 37(15)

(3S)-1-butyl-2,5-dioxo-3-(2-methylpropyl)-9-(4-chlorophenylmethyl)-1,4,9-triazaspiro[5.5]undecane

Compound 37(16)

(3S)-1-butyl-2,5-dioxo-3-(2-methylpropyl)-9-(3-chlorophenylmethyl)-1,4,9-triazaspiro[5.5]undecane

Compound 37(17)

(3S)-1-butyl-2,5-dioxo-3-(2-methylpropyl)-9-(3-methyl-4-methoxyphenylmethyl)-1,4,9-triazaspiro[5.5]undecane

Compound 37(18)

(3S)-1-butyl-2,5-dioxo-3-(2-methylpropyl)-9-(7-methoxy-1,3-benzodioxolan-5-ylmethyl)-1,4,9-triazaspiro [5.5]undecane

Compound 37(19)

(3S)-1-butyl-2,5-dioxo-3-(2-methylpropyl)-9-(4-phenylthiophenylmethyl)-1,4,9-tnazaspiro[5.5]undecane

Compound 37(20)

(3S)-1-butyl-2,5-dioxo-3-(2-methylpropyl)-9-(2-methylphenylmethyl)-1,4,9-triazaspiro[5.5]undecane

Compound 37(21)

(3S)-1-butyl-2,5-dioxo-3-(2-methylpropyl)-9-(3-methylphenylmethyl)-1,4,9-triazaspiro[5.5]undecane

Compound 37(22)

(3S)-1-butyf-2,5-dioxo-3-(2-methylpropyl)-9-(4-methylphenylmethyl)-1,4,9-triazaspiro[5.5]undecane

Compound 37(23)

(3S)-1-buty)-2,5-dioxo-3-(2-methylpropyl)-9-(4-(1-methylethyl)phenytmethyl)-1,4,9-triazaspiro[5.5]undecane

Compound 37(24)

(3S)-1-butyl-2,5-dioxo-3-(2-methylpropyl)-9-(3-fluoro-4-methoxyphenylmethyl)-1,4,9-triazaspiro[5.5]undecane

Compound 37(25)

(3S)-1-butyl-2,5-dioxo-3-(2-methylpropyl)-9-(4-(2-hydroxyethyloxy)phenylmethyl)-1,4,9-triazaspiro[5.5]undecane

Compound 37(26)

(3S)-1-butyl-2,5-dioxo-3-(2-methylpropyl)-9-(2-hydroxy-3-methylphenylmethyl)-1,4,9-triazaspiro[5.5]undecane

Compound 37(27)

(3S)-1-butyl-2,5-dioxo-3-(2-methylpropyl)-9-(4-trifluoromethyloxyphenylmethyl)-1,4,9-triazaspiro[5.5]undecane

Compound 37(28)

(3S)-1-butyl-2,5-dioxo-3-(2-methylpropyl)-9-(3-methyl-5-chloro-1-phenylpyrazol-4-ylmethyl)-1,4,9-triazaspiro[5.5]undecane

Compound 37(29)

(3S)-1-butyl-2,5-dioxo-3-(2-methylpropyl)-9-(6-phenylpyridin-3-ylmethyl)-1,4,9-triazaspiro[5.5]undecane

Compound 37(30)

(3S)-1-butyl-2,5-dioxo-3-(2-methylpropyl)-9-(4-(4-methylsulfony(aminophenyloxy)phenylmethyl)-1,4,9-triazaspiro[5.5]undecane

Compound 37(31)

(3S)-1-butyl-2,5-dioxo-3-(2-methylpropyl)-9-(3,5-dimethyl-1-(4-methylsulfonylaminophenyl)pyrazol-4-ylmethyl)-1,4,9-triazaspiro[5.5]undecane

Compound 37(32)

(3S)-1-butyl-2,5-dioxo-3-(2-methylpropyl)-9-(4-(5-methylpyridin-2-yloxy)phenylmethyl)-1,4,9-triazaspiro[5.5] undecane

Compound 37(33)

(3S)-1-butyl-2,5-dioxo-3-(2-methylpropyl)-9-(4-(6-methylpyridin-1-oxido-3-yloxy)phenylmethyl)-1,4,9-triazaspiro[5.5]undecane

Compound 37(34)

(3S)-1-butyl-2,5-dioxo-3-(2-methylpropyl)-9-(1-(2-methylpropyloxycarbanyl)indol-5-ylmethyl)-1,4,9-triazaspiro[5.5]undecane

Compound 37(35)

(3S)-1-butyl-2,5-dioxo-3-(2-methylpropyl)-9-(2-phenyl-5-methyloxazol-4-ylmethyl)-1,4,9-triazaspiro[5.5]undecane

Compound 37(36)

(3S)-1-butyl-2,5-dioxo-3-(2-methylpropyl)-9-(4-(tetrahydropyran-4-yloxy)phenylmethyl)-1,4,9-triazaspiro [5.5]undecane

Compound 37(37)

(3S)-1-butyl-2,5-dioxo-3-(2-methylpropyl)-9-(4-(6-methylpyridin-3-yloxy)phenylmethyl)-1,4,9-triazaspiro[5.5] undecane

Compound 37(38)

(3S)-1-butyl-2,5-dioxo-3-(2-methylpropyl)-9-(3,5-dimethyl-1-(4-fluorophenyl)pyrazol-4-ylmethyl)-1,4,9-triazaspiro[5.5]undecane

Compound 37(39)

(3S)-1-butyl-2,5-dioxo-3-(2-methylpropyl)-9-(3,5-dimethyl-1-(pyridin-2-yl)pyrazol-4-ylmethyl)-1,4,9-triazaspiro[5.5]undecane

Compound 37(40)

(3S)-1-butyl-2,5-dioxo-3-(2-methylpropyl)-9-(3,5-dimethyl-1-(4-hydroxyphenyl)pyrazol-4-ylmethyl)-1,4,9-triazaspiro[5.5)undecane

Compound 37(41)

(3S)-1-butyl-2,5-dioxo-3-(2-methylpropyl)-9-(4-(2-carboxyethyl)pheny[methyl)-1,4,9-triazaspiro[5.5]undecane

Compound 37(42)

(3S)-1-butyl-2,5-dioxo-3-(2-methylpropyl)-9-(3,5-dimethyl-1-(4-(dimethylaminosulfonyl)phenyl)pyrazol-4-ylmethyl)-1,4,9-triazaspiro[5.5]undecane

Compound 37(43)

(3S)-1-butyl-2,5-dioxo-3-(2-methylpropyl)-9-(4-(5-methylpyridin-1-oxido-2-yloxy)phenylmethyl)-1,4,9-triazaspiro[5.5]undecane

Compound 37(44)

(3S)-1-butyl-2,5-dioxo-3-(2-methylpropyl)-9-(4-(2-carboxy-1-ethenyl)phenylmethyl)-1,4,9-triazaspiro[5.5]undecane

Compound 37(45)

(3S)-1-butyl-2,5-dioxo-3-(2-methylpropyl)-9-(4-(4-(2-carboxy-1-ethenyl)phenyloxy)phenylmethyl)-1,4,9-triazaspiro(5.5]undecane

Compound 37(46)

(3S)-1-butyl-2,5-dioxo-3-(2-methylpropyl)-9-(4-(4-aminocarbonylphenyloxy)phenylmethyl)-1,4,9-triazaspiro[5.5]undecane

Compound 37(47)

(3S)-1-butyl-2,5-dioxo-3-(2-methylpropyl)-9-(4-(4-aminosulfonylphenyloxy)phenylmethyl)-1,4,9-triazaspiro[5.5]undecane

Compound 37(48)

(3S)-1-butyl-2,5-dioxo-3-(2-methylpropyl)-9-(3,5-dimethyl-1-benzylpyrazol-4-ylmethyl)-1,4,9-triazaspiro[5.5]undecane

Compound 37(49)

(3S)-1-butyl-2,5-dioxo-3-(2-methylpropyl)-9-(3,5-dimethyl-1-(2,4-difluorophenyl)pyrazol-4-ylmethyl)-1,4,9-triazaspiro[5.5]undecane

Compound 37(50)

(3S)-1-butyl-2,5-dioxo-3-(2-methylpropyl)-9-(4-(pyrrolidin-1-ylmethyl)phenylmethyl)-1,4,9-triazaspiro[5.5]undecane

Compound 37(51)

(3S)-1-butyl-2,5-dioxo-3-(2-methylpropyl)-9-(3,5-dimethyl-1-(4-(morpholin-4-ylsulfonyl)phenyl)pyrazol-4-ylmethyl)- 1,4,9-triazaspiro[5.5]undecane

Compound 37(52)

(3S)-1-butyl-2,5-dioxo-3-(2-methylpropyl)-9-(3,5-dimethyl-1-(4-(methylaminosulfonyl)phenyl)pyrazol-4-ylmethyl)-1,4,9-triazaspiro[5.5]undecane

Compound 37(53)

(3S)-1-butyl-2,5-dioxo-3-(2-methylpropyl)-9-(4-(4-cyanophenyloxy)phenylmethyl)-1,4,9-triazaspiro[5.5]undecane

Compound 37(54)

(3S)-1-butyl-2,5-dioxo-3-(2-methylpropyl)-9-(4-(dimethylaminomethyl)phenylmethyl)-1,4,9-triazaspiro[5.5]undecane

Compound 37(55)

(3S)-1-butyl-2,5-dioxo-3-(2-methylpropyl)-9-(3,5-dimethyl-1-(4-(2-dimethylaminoethylaminosulfonyl)phenyl)pyrazol-4-ylmethyl)-1,4,9-triazaspiro[5.5]undecane

Compound 37(56)

(3S)-1-butyl-2,5-dioxo-3-(2-methylpropyl)-9-(3-(4-hydroxyphenyl)phenylmethyl)-1,4,9-triazaspiro[5.5]undecane

Compound 37(57)

(3S)-1-butyl-2,5-dioxo-3-(2-methylpropyl)-9-(4-(3-methoxyphenyloxy)phenylmethyl)-1,4,9-triazaspiro[5.5]undecane

Compound 37(58)

(3S)-1-butyl-2,5-dioxo-3-(2-methylpropyl)-9-(3,5-dimethyl-1-(quinoxalin-2-yl)pyrazol-4-ylmethyl)-1,4,9-triazaspiro[5.5]undecane

Compound 37(59)

(3S)-1-butyl-2,5-dioxo-3-(2-methylpropyl)-9-(4-phenylcarbonylphenylmethyl)-1,4,9-triazaspiro[5.5]undecane

Compound 37(60)

(3S)-1-butyl-2,5-dioxo-3-(2-methylpropyl)-9-(3,5-dimethyl-1-(4-(N-(2-hydroxyethyl)-N-methylaminosulfonyl)phenyl)pyrazol-4-ylmethyl)-1,4,9-triazaspiro[5.5]undecane

Compound 37(61)

(3S)-1-butyl-2,5-dioxo-3-(2-methylpropyl)-9-(3,5-dimethyl-1-(2-phenylethyl)pyrazol-4-ylmethyl)-1,4,9-triazaspiro[5.5]undecane

Compound 37(62)

(3S)-1-butyl-2,5-dioxo-3-(2-methylpropyl)-9-(1,3,5-trimethylpyrazol-4-ylmethyl)-1,4,9-triazaspiro[5.5]undecane

Compound 37(63)

(3S)-1-butyl-2,5-dioxo-3-(2-methylpropyl)-9-(4-(morpholin-4-ylmethyl)phenylmethyl)-1,4,9-triazaspiro[5.5]undecane

Compound 37(64)

(3S)-1-butyl-2,5-dioxo-3-(2-methylpropyl)-9-(4-(4-methylpiperazin-1-ylmethyl)phenylmethyl)-1,4,9-triazaspiro[5.5]undecane

Compound 37(65)

(3S)-1-butyl-2,5-dioxo-3-(2-methylpropyl)-9-(4-phenylsulfonylphenylmethyl)-1,4,9-triazaspiro[5.5]undecane

Compound 37(66)

(3S)-1-butyl-2,5-dioxo-3-(2-methylpropyl)-9-(3,5-dimethyl-1-cyclohexylpyrazol-4-ylmethyl)-1,4,9-triazaspiro[5.5]undecane

Compound 37(67)

(3S)-1-butyl-2,5-dioxo-3-(2-methylpropyl)-9-(4-(3-carboxyphenytoxy)phenylmethyl)-1,4,9-triazaspiro[5.5]undecane

Compound 37(68)

(3S)-1-butyl-2,5-dioxo-3-(2-methylpropyl)-9-(4-(piperidin-1-ylmethyl)phenylmethyl)-1,4,9-triazaspiro[5.5]undecane

Compound 37(69)

(3S)-1-butyl-2,5-dioxo-3-(2-methylpropyl)-9-(3,5-dimethyl-1-(4-(pyrrolidin-1-ylsulfonyl)phenyl)pyrazol-4-ylmethyl)-1,4,9-triazaspiro[5.5]undecane

Compound 37(70)

(3S)-1-butyl-2,5-dioxo-3-(2-methy(propyl)-9-(2,3-dihydrobenzofuran-5-ylmethyl)-1,4,9-triazaspiro[5.5]undecane

Compound 37(71)

(3S)-1-butyl-2,5-dioxo-3-(2-methylpropyl)-9-(3,5-dimethyl-1-(4-(2-hydroxyethylaminosulfonyl)phenyl)pyrazol-4-ylmethyl)-1,4,9-triazaspiro[5.5]undecane

Compound 37(72)

(3S)-1-butyl-2,5-dioxo-3-(2-methylpropyl)-9-(4-(carboxymethyloxy)phenylmethyl)-1,4,9-triazaspiro[5.5]undecane

Compound 37(73)

(3S)-1-buty)-2,5-dioxo-3-(2-methy)propyl)-9-(4-(1-phenyl-1-hydroxymethyl)phenylmethyl)-1,4,9-triazaspiro [5.5]undecane

Compound 37(74)

(3S)-1-butyl-2,5-dioxo-3-(2-methylpropyl)-9-(4-(4-hydroxypiperidin-1-ylmethyl)phenylmethyl)-1,4,9-triazaspiro[5.5]undecane

Compound 37(75)

(3S)-1-butyl-2,5-dioxo-3-(2-methylpropyl)-9-(4-(3-carboxyphenylmethyloxy)phenylmethyl)-1,4,9-triazaspiro [5.5]undecane

Compound 37(76)

(3S)-1-butyl-2,5-dioxo-3-(2-methylpropyl)-9-(4-(bis(methylsulfonyl)amino)phenylmethyl)-1,4,9-triazaspiro [5.5]undecane

Compound 37(77)

(3S)-1-butyl-2,5-dioxo-3-(2-methylpropyl)-9-(4-(1,4-benzodioxan-6-yloxy)phenylmethyl)-1,4,9-triazaspiro [5.5]undecane

Compound 37(78)

(3S)-1-butyl-2,5-dioxo-3-(2-methylpropyl)-9-(3-(3-hydroxyphenyl)phenylmethyl)-1,4,9-triazaspiro[5.5]undecane

Compound 37(79)

(3S)-1-butyl-2,5-dioxo-3-(2-methylpropyl)-9-(4-(methylsulfonylamino)phenylmethyl)-1,4,9-triazaspiro[5.5]undecane

Compound 37(80)

(3S)-1-butyl-2,5-dioxo-3-(2-methylpropyl)-9-(6-(4-methoxyphenyloxy)pyridin-3-ylmethyl)-1,4, 9-triazaspiro [5.5]undecane

Compound 37(81)

(3S)-1-butyl-2,5-dioxo-3-(2-methylpropyl)-9-(4-(4-methylaminocarbonylphenytoxy)phenylmethyl)-1,4, 9-triazaspiro[5.5]undecane

Compound 37(82)

(3S)-1-butyl-2,5-dioxo-3-(2-methylpropyl)-9-(4-(4-chlorophenyloxy)phenylmethyl)-1,4,9-triazaspiro[5.5]undecane

Compound 37(83)

(3S)-1-butyl-2,5-dioxo-3-(2-methylpropyl)-9-(3-(4-carboxyphenyl)phenylmethyl)-1,4,9-triazaspiro[5.5]undecane

Compound 37(84)

(3S)-1-butyl-2,5-dioxo-3-(2-methylpropyl)-9-(4-(phenylaminocarbonyl)phenylmethyl)-1,4,9-triazaspiro[5.5] undecane

Compound 37(85)

(3S)-1-butyl-2,5-dioxo-3-(2-methylpropyl)-9-(4-(4-methylthiophenyloxy)phenylmethyl)-1,4,9-triazaspiro[5.5] undecane

Compound 37(86)

(3S)-1-butyl-2,5-dioxo-3-(2-methylpropyl)-9-(4-(4-(2-dimethylaminoethylaminocarbonyl)phenyloxy)phenylmethyl)-1,4,9-triazaspiro[5.5]undecane

Compound 37(87)

(3S)-1-butyl-2,5-dioxo-3-(2-methylpropyl)-9-(4-aminocarbonylphenylmethyl)-1,4,9-triazaspiro[5.5]undecane

Compound 37(88)

(3S)-1-butyl-2,5-dioxo-3-(2-methylpropyl)-9-(4-dimethylaminocarbonylphenylmethyl)-1,4,9-triazaspiro[5.5] undecane

Compound 38

(3S)-1-butyl-2,5-dioxo-3-cyclohexylmethyl-9-benzyloxycarbonyl-1,4,9-triazaspiro[5.5]undecane

Compound 39

(3S)-1-butyl-2,5-dioxo-3-cyclohexylmethyl-1,4,9-triazaspiro[5.5]undecane

Compound 40(1)

(3S)-1-butyl-2,5-dioxo-3-cyclohexylmethyl-9-{4-(4-methylphenyloxy)phenylmethyl)-1,4,9-triazaspiro[5.5]undecane

Compound 40(2)

(3S)-1-butyl-2,5-dioxo-3-cyclohexylmethyl-9-(4-(4-methoxyphenyloxy)phenylmethyl)-1,4,9-triazaspiro[5.5] undecane

Compound 40(3)

(3S)-1-butyl-2,5-dioxo-3-cyclohexylmethyl-9-(2-fluorophenylmethyl)-1,4,9-triazaspiro[5.5]undecane

Compound 40(4)

(3S)-1-butyl-2,5-dioxo-3-cylohexylmethyl-9-(3-fluorophenylmethyl)-1,4,9-triazaspiro[5.5]undecane

Compound 40(5)

(3S)-1-butyl-2,5-dioxo-3-cyclohexylmethyl-9-(4-fluorophenylmethyl)-1,4,9-triazaspiro[5.5]undecane

Compound 40(6)

(3S)-1-butyl-2,5-dioxo-3-cyclohexylmethyl-9-{3-chlorophenylmethyl)-1,4,9-triazaspiro[5.5]undecane

Compound 40(7)

(3S)-1-butyl-2,5-dioxo-3-cyclohexylmethyl-9-(4-cyclohexyloxyphenylmethyl)-1,4,9-triazaspiro[5.5]undecane

Compound 40(8)

(3S)-1-butyl-2,5-dioxo-3-cyclohexylmethyl-9-(4-methoxy-3-hydroxyphenylmethyl)-1,4,9-tnazaspiro[5.5]undecane

Compound 40(9)

(3S)-1-butyl-2,5-dioxo-3-cyclohexylmethyl-9-(2-chlorophenylmethyl)-1,4,9-triazaspiro[5.5]undecane

Compound 40(10)

(3S)-1-butyl-2,5-dioxo-3-cyclohexylmethyl-9-(2-methylphenylmethyl)-1,4,9-triazaspiro[5.5]undecane

Compound 40(11)

(3S)-1-butyl-2,5-dioxo-3-cyclohexylmethyl-9-(3-methylphenylmethyl)-1,4,9-triazaspiro[5.5]undecane

Compound 40(12)

(3S)-1-butyl-2,5-dioxo-3-cyclohexylmethyl-9-(4-methylphenylmethyl)-1,4,9-triazaspiro[5.5]undecane

Compound 40(13)

(3S)-1-butyl-2,5-dioxo-3-cyclohexylmethyl-9-(4-phenylthiophenylmethyl)-1,4,9-triazaspiro[5.5]undecane

Compound 40(14)

(3S)-1-butyl-2,5-dioxo-3-cyclohexylmethyl-9-(3-(2-methylpropyl)phenylmethyl)-1,4, 9-triazaspiro[5.5]undecane

Compound 40(15)

(3S)-1-butyl-2, 5-dioxo-3-cyclohexylmethyl-9-(3-butylphenylmethyl)-1,4,9-triazaspiro[5.5]undecane

Compound 40(16)

(3S)-1-butyl-2,5-dioxo-3-cyclohexylmethyl-9-(4-isopropylphenylmethyl)-1,4,9-triazaspiro[5.5]undecane

Compound 40(17)

(3S)-1-butyl-2,5-dioxo-3-cyclohexylmethy)-9-(4-methoxy-3-fluorophenylmethyl)-1,4,9-triazaspiro[5.5]undecane

Compound 40(18)

(3S)-1-butyl-2,5-dioxo-3-cyclohexylmethyl-9-(4-(2-hydroxyethoxy)phenylmethyl)-1,4,9-triazaspiro[5.5]undecane

Compound 40(19)

(3S)-1-butyl-2,5-dioxo-3-cyclohexylmethyl-9-(2-hydroxy-3-methylphenylmethyl)-1,4,9-triazaspiro[5.5]undecane

Compound 40(20)

(3S)-1-butyl-2,5-dioxo-3-cyclohexylmethyl-9-(4-chlorophenylmethyl)-1,4,9-triazaspiro[5.5]undecane

Compound 40(21)

(3S)-1-butyl-2,5-dioxo-3-cyctohexylmethyl-9-(7-methoxy-1,3-benzodioxolan-5-ylmethyl)-1,4,9-triazaspiro [5.5]undecane

Compound 40(22)

(3S)-1-butyl-2, 5-dioxo-3-cyclohexylmethyl-9-(3-methyl-4-methoxyphenylmethyl)-1,4,9-triazaspiro[5.5]undecane

Compound 40(23)

(3S)-1-butyl-2,5-dioxo-3-cyclohexylmethyl-9-(4-(4-fluorophenyloxy)phenylmethyl)-1,4,9-triazaspiro[5.5]undecane

Compound 40(24)

(3S)-1-butyl-2,5-dioxo-3-cyclohexylmethyl-9-(4-trifluoromethoxyphenylmethyl)-1,4,9-triazaspiro[5.5]undecane

Compound 40(25)

(3S)-1-butyl-2,5-dioxo-3-cyclohexylmethyl-9-(3-methyl-5-chloro-1-phenylpyrazol-4-ylmethyl)-1,4,9-triazaspiro[5.5]undecane

Compound 40(26)

(3S)-1-butyl-2,5-dioxo-3-cyclohexylmethyl-9-(2,3-dimethyl-5-oxo-1-phenylpyrazolin-4-ylmethyl)-1,4,9-triazaspiro[5.5]undecane

Compound 40(27)

(3S)-1-butyl-2,5-dioxo-3-cyclohexylmethyl-9-(1-(2-methylpropyloxycarbonyl)indol-5-ylmethyl)-1,4,9-triazaspiro[5.5]undecane

Compound 40(28)

(3S)-1-butyl-2,5-dioxo-3-cyclohexylmethyl-9-(5-methyl-2-phenyloxazol-4-ylmethyl)-1,4,9-triazaspiro[5.5]undecane

Compound 40(29)

(3S)-1-butyl-2,5-dioxo-3-cyclohexylmethyl-9-(3,5-dimethyl-1-(4-methylsulfonylaminophenyl)pyrazol-4-ylmethyl)-1,4,9-triazaspiro[5.5]undecane

Compound 40(30)

(3S)-1-butyl-2,5-dioxo-3-cyclohexylmethyl-9-(4-(4-methylsulfonylaminophenyloxy)phenylmethyl)-1,4,9-triazaspiro[5.5]undecane

Compound 40(31)

(3S)-1-butyl-2,5-dioxo-3-cyclohexylmethyl-9-(4-(6-methylpyridin-3-yloxy)phenylmethyl)-1,4,9-tnazaspiro [5.5]undecane

Compound 40(32)

(3S)-1-butyl-2,5-dioxo-3-cyclohexylmethyl-9-(4-(6-methylpyridin-1-oxido-3-yloxy)phenylmethyl)-1,4,9-triazaspiro[5.5]undecane

Compound 40(33)

(3S)-1-butyl-2,5-dioxo-3-cyclohexylmethyl-9-(4-(tetrahydropyran-4-yloxy)phenylmethyl)-1,4,9-tnazaspiro [5.5]undecane

Compound 40(34)

(3S)-1-butyl-2,5-dioxo-3-cyclohexylmethyl-9-(6-phenylpyridin-3-ylmethyl)-1,4,9-triazaspiro[5.5]undecane

Compound 40(35)

(3S)-1-butyl-2,5-dioxo-3-cyclohexylmethyl-9-(3,5-dimethyl-1-(4-fluorophenyl)pyrazol-4-ylmethyl)-1,4,9-triazaspiro[5.5]undecane

Compound 40(36)

(3S)-1-butyl-2,5-dioxo-3-cyclohexylmethyl-9-(3,5-dimethyl-1-(pyridin-2-yl)pyrazol-4-ylmethyl)-1,4,9-triazaspiro[5.5]undecane

Compound 40(37)

(3S)-1-butyl-2,5-dioxo-3-cyclohexylmethyl-9-(3,5-dimethyl-1-(4-hydroxyphenyl)pyrazol-4-ylmethyl)-1,4,9-triazaspiro[5.5]undecane

Compound 40(38)

(3S)-1-butyl-2,5-dioxo-3-cyclohexylmethyl-9-(4-(2-carboxyethyl)phenylmethyl)-1,4,9-triazaspiro[5.5]undecane

Compound 40(39)

(3S)-1-butyl-2,5-dioxo-3-cyclohexylmethyl-9-(4-{4-hydroxyphenyloxy)phenylmethyl)-1,4,9-triazaspiro[5.5] undecane

Compound 40(40)

(3S)-1-butyl-2,5-dioxo-3-cyclohexylmethyl-9-(3,5-dimethyl-1-(4-carboxyphenyl)pyrazol-4-ylmethyl)-1,4,9-triazaspiro[5.5]undecane

Compound 40(41)

(3S)-1-butyl-2,5-dioxo-3-cyclohexylmethyl-9-(3,5-dimethyl-1-(4-(dimethylaminosulfonyl)phenyl)pyrazol-4-ylmethyl)-1,4,9-triazaspiro[5.5]undecane

Compound 40(42)

(3S)-1-butyl-2,5-dioxo-3-cyclohexylmethyl-9-(4-(5-methylpyridin-1-oxido-2-yloxy)phenylmethyl)-1,4,9-triazaspiro[5.5]undecane

Compound 40(43)

(3S)-1-butyl-2,5-dioxo-3-cyclohexylmethyl-9-(4-(2-carboxy-1-ethynyl)phenylmethyl)-1,4,9-triazaspiro[5.5]undecane

Compound 40(44)

(3S)-1-butyl-2,5-dioxo-3-cyclohexylmethyl-9-(4-(4-((1E)-2-carboxy-1-ethynyl)phenyloxy)phenylmethyl)-1,4,9-triazaspiro[5.5]undecane

Compound 40(45)

(3S)-1-butyl-2,5-dioxo-3-cyclohexylmethyl-9-(4-(4-aminocarbonylphenyloxy)phenylmethyl)-1,4,9-triazaspiro[5.5]undecane

Compound 40(46)

(3S)-1-butyl-2,5-dioxo-3-cyclohexylmethyl-9-(4-(4-aminosulfonylphenyloxy)phenylmethyl)-1,4,9-triazaspiro[5.5]undecane

Compound 40(47)

(3S)-1-butyl-2,5-dioxo-3-cyclohexylmethyl-9-(3,5-dimethyl-1-benzylpyrazol-4-ylmethyl)-1,4,9-triazaspiro[5.5]undecane

Compound 40(48)

(3S)-1-butyl-2,5-dioxo-3-cyclohexylmethyl-9-(3,5-dimethyl-1-(2,4-difluorophenyl)pyrazol-4-ylmethyl)-1,4,9-triazaspiro[5.5]undecane

Compound 40(49)

(3S)-1-butyl-2,5-dioxo-3-cyclohexylmethyl-9-(4-(pyrrolidin-1-ylmethyl)phenylmethyl)-1,4,9-triazaspiro[5.5]undecane

Compound 40(50)

(3S)-1-butyl-2,5-dioxo-3-cyclohexylmethyl-9-(3,5-dimethyl-1-(4-(morpholin-4-ylsulfonyl)phenyl)pyrazol-4-ylmethyl)-1,4,9-triazaspiro[5.5]undecane

Compound 40(51)

(3S)-1-butyl-2,5-dioxo-3-cyclohexylmethyl-9-(4-(4-cyanophenyloxy)phenylmethyl)-1,4,9-triazaspiro[5.5]undecane

Compound 40(52)

(3S)-1-butyl-2,5-dioxo-3-cyclohexylmethyl-9-(3,5-dimethyl-1-(4-(N-(2-hydroxyethyl)-N-methylaminosulfonyl)phenyl)pyrazol-4-ylmethyl)-1,4,9-triazaspiro[5.5]undecane

Compound 40(53)

(3S)-1-butyl-2,5-dioxo-3-cyclohexylmethyl-9-(3,5-dimethyl-1-(2-phenylethyl)pyrazol-4-ylmethyl)-1,4,9-triazaspiro[5.5]undecane

Compound 40(54)

(3S)-1-butyl-2,5-dioxo-3-cyclohexylmethyl-9-(4-(dimethylaminomethyl)phenylmethyl)-1,4,9-triazaspiro[5.5]undecane

Compound 40(55)

(3S)-1-butyl-2,5-dioxo-3-cyclohexylmethyl-9-(3-(4-hydroxyphenyl)phenylmethyl)-1,4,9-triazaspiro[5.5]undecane

Compound 40(56)

(3S)-1-butyl-2,5-dioxo-3-cyclohexylmethyl-9-(3,5-dimethyl-1-(quinoxalin-2-yl)pyrazol-4-ylmethyl)-1,4,9-triazaspiro[5.5]undecane

Compound 40(57)

(3S)-1-butyl-2,5-dioxo-3-cyclohexylmethyl-9-(4-(phenylcarbonyl)phenylmethyl)-1,4,9-triazaspiro[5.5]undecane

Compound 40(58)

(3S)-1-butyl-2,5-dioxo-3-cyclohexylmethyl-9-(3,5-dimethyl-1-(4-methylaminosulfonylphenyl)pyrazol-4-ylmethyl)-1,4, 9-triazaspiro[5.5]undecane

Compound 40(59)

(3S)-1-butyl-2,5-dioxo-3-cyclohexylmethyl-9-(1,3,5-trimethylpyrazol-4-ylmethyl)-1,4,9-triazaspiro[5.5]undecane

Compound 40(60)

(3S)-1-butyl-2,5-dioxo-3-cyclohexylmethyl-9-(4-(morpholin-4-ylmethyl)phenylmethyl)-1,4,9-triazaspiro[5.5]undecane

Compound 40(61)

(3S)-1-butyl-2,5-dioxo-3-cyclohexylmethyl-9-(4-(3-methoxyphenyloxy)phenylmethyl)-1,4,9-triazaspiro[5.5]undecane

Compound 40(62)

(3S)-1-butyl-2,5-dioxo-3-cyclohexylmethyl-9-(4-(4-methylpiperazin-1-ylmethyl)phenylmethyl)-1,4,9-triazaspiro[5.5]undecane

Compound 40(63)

(3S)-1-butyl-2,5-dioxo-3-cyctohexylmethyl-9-(4-(pyridin-1-oxido-3-yloxy)phenylmethyl)-1,4,9-triazaspiro [5.5]undecane

Compound 40(64)

(3S)-1-butyl-2,5-dioxo-3-cyclohexylmethyl-9-(4-phenylsulfonylphenylmethyl)-1,4, 9-triazaspiro[5.5]undecane

Compound 40(65)

(3S)-1-butyl-2,5-dioxo-3-cyclohexylmethyl-9-(3,5-dimethyl-1-cyclohexylpyrazol-4-ylmethyl)-1,4,9-triazaspiro [5.5]undecane

Compound 40(66)

(3S)-1-butyl-2,5-dioxo-3-cyclohexylmethyl-9-(4-(3-carboxyphenyloxy)phenylmethyl)-1,4,9-triazaspiro[5.5] undecane

Compound 40(67)

(3S)-1-butyl-2,5-dioxo-3-cyclohexylmethyl-9-(4-(piperidin-1-ylmethyl)phenylmethyl)-1,4,9-tnazaspiro[5.5] undecane

Compound 40(68)

(3S)-1-butyl-2,5-dioxo-3-cyclohexylmethyl-9-(3,5-dimethyl-1-(4-(pyrrolidin-1-ylsulfonyl)phenyl)pyrazol-4-ylmethyl)-1,4,9-triazaspiro[5.5]undecane

Compound 40(69)

(3S)-1-butyl-2,5-dioxo-3-cyclohexylmethyl-9-(2,3-dihydrobenzofuran-5-ylmethyl)-1,4, 9-triazaspiro[5.5]undecane

Compound 40(70)

(3S)-1-butyl-2,5-dioxo-3-cyclohexylmethyl-9-(4-(4-carboxyphenyloxy)phenylmethyl)-1,4,9-triazaspiro[5.5] undecane

Compound 40(71)

(3S)-1-butyl-2,5-dioxo-3-cyclohexylmethyl-9-(3,5-dimethyl-1-(4-(2-hydroxyethytaminosutfonyl)phenyl)pyrazol-4-ylmethyl)-1,4,9-triazaspiro[5.5]undecane

Compound 40(72)

(3S)-1-butyl-2,5-dioxo-3-cyclohexylmethyl-9-(3,5-dimethyl-1-(4-(2-dimethylaminoethylaminosulfonyl)phenyl)pyrazol-4-ylmethyl)-1,4,9-triazaspiro[5.5]undecane

Compound 40(73)

(3S)-1-butyl-2,5-dioxo-3-cyclohexylmethyl-9-(4-(1-hydroxy-1-phenyimethyl)phenylmethyl)-1,4,9-triazaspiro [5.5]undecane

Compound 40(74)

(3S)-1-butyl-2,5-dioxo-3-cyctohexylmethyl-9-(4-(carboxymethyloxy)phenylmethyl)-1,4,9-triazaspiro[5.5]undecane

Compound 40(75)

(3S)-1-butyl-2,5-dioxo-3-cyclohexylmethyl-9-(4-(4-hydroxypiperidin-1-ylmethyl)phenylmethyl)-1,4,9-triazaspiro[5.5]undecane

Compound 40(76)

(3S)-1-butyl-2, 5-di oxo-3-cyclohexylmethyl-9-(4-(3-carboxyphenylmethyloxy)phenylmethyl)-1,4,9-triazaspiro[5.5]undecane

Compound 40(77)

(3S)-1-butyl-2,5-dioxo-3-cyclohexylmethyl-9-(4-(1,4-benzodioxan-6-yloxy)phenylmethyl)-1,4,9-triazaspiro [5.5]undecane

Compound 40(78)

(3S)-1-butyl-2,5-dioxo-3-cyclohexylmethyl-9-(3-(3-hydroxyphenyl)phenylmethyl)-1,4,9-triazaspiro[5.5]undecane

Compound 40(79)

(3S)-1-butyl-2,5-dioxo-3-cyclohexylmethyl-9-(4-(methylsulfonylamino)phenylmethyl)-1,4,9-thazaspiro[5.5] undecane

Compound 40(80)

(3S)-1-butyl-2,5-dioxo-3-cyclohexylmethyl-9-(6-(4-methoxyphenyl)pyridin-3-ylmethyl)-1,4,9-triazaspiro[5.5] undecane

Compound 40(81)

(3S)-1-butyl-2,5-dioxo-3-cyclohexylmethyl-9-(4-(4-methylaminocarbonylphenyloxy)phenylmethyl)-1,4,9-triazaspiro[5.5]undecane

Compound 40(82)

(3S)-1-butyl-2,5-dioxo-3-cyclohexylmethyl-9-(4-(4-chlorophenyloxy)phenylmethyl)-1,4,9-triazaspiro[5.5]undecane

Compound 40(83)

(3S)-1-butyl-2,5-dioxo-3-cyclohexylmethyl-9-(4-bis(methylsulfonyl)aminophenylmethyl)-1,4,9-triazaspiro [5.5]undecane

Compound 40(84)

(3S)-1-butyl-2,5-dioxo-3-cyclohexylmethyl-9-(3-(4-carboxyphenyl)phenylmethyl)-1,4,9-triazaspiro[5.5]undecane

Compound 40(85)

(3S)-1-butyl-2,5-dioxo-3-cyclohexylmethyl-9-(4-(phenylaminocarbonyl)phenylmethyl)-1,4,9-triazaspiro[5.5] undecane

Compound 40(86)

(3S)-1-butyl-2,5-dioxo-3-cyclohexylmethyl-9-(4-(4-methylthiophenyloxy)phenylmethyl)-1,4,9-triazaspiro[5.5]undecane

Compound 40(87)

(3S)-1-butyl-2,5-dioxo-3-cyclohexylmethyl-9-(4-(4-(2-dimethylaminoethylaminocarbonyl)phenyloxy)phenylmethyl)-1,4,9-triazaspiro[5.5]undecane

Compound 40(88)

(3S)-1-butyl-2,5-dioxo-3-cyclohexylmethyl-9-(4-aminocarbonylphenylmethyl)-1,4,9-triazaspiro[5.5]undecane

Compound 40(89)

(3S)-1-butyl-2,5-dioxo-3-cyclohexylmethyl-9-(4-(dimethylaminocarbonyl)phenylmethyl)-1,4,9-triazaspiro[5.5]undecane

Compound 40(90)

(3S)-1-butyl-2,5-dioxo-3-cyclohexylmethyl-9-(4-phenyloxyphenylmethyl)-1,4,9-triazaspiro[5.5]undecane

Compound 41(1)

1-butyl-2, 5-dioxo-3-(1-hydroxy-2-methylpropyl)-9-(1,4-benzodioxan-6-ylmethyl)-1,4,9-triazaspiro[5.5]undecane

Compound 41 (2)

(Z)-1-butyl-2,5-dioxo-3-(2-methylpropylidene)-9-(1,4-benzodioxan-6-ylmethyl)-1,4,9-triazaspiro[5.5]undecane

Compound 41 (3)

(3S)-1-butyl-2,5-dioxo-3-((1R)-1-hydroxyethyl)-9-(4-phenyloxyphenylmethyl)-1,4,9-triazaspiro[5.5]undecane

Compound 41(4)

(Z)-1-butyl-2,5-dioxo-3-ethylidene-9-(4-phenyloxyphenylmethyl)-1,4,9-triazaspiro[5.5]undecane

Compound 41 (5)

(Z)-1-butyl-2,5-dioxo-3-(2-methylpropylidene)-9-(4-phenyloxyphenylmethyl)-1,4,9-triazaspiro[5.5]undecane

Compound 42

(3R)-1-butyl-2,5-dioxo-3-((1R*)-1-hydroxy-2-methylpropyl)-9-benzyloxycarbonyl-1,4,9-triazaspiro[5.5]undecane

Compound 43

(3R*)-1-butyl-2,5-dioxo-3-((1R*)-1-hydroxy-2-methylpropyl)-1,4,9-triazaspiro[5.5]undecane

Compound 44(1)

(3R*)-1-butyl-2,5-dioxo-3-((1R*)-1-hydroxy-2-methylpropyl)-9-(4-phenyloxyphenylmethyl)-1,4,9-triazaspiro

[5.5]undecane

Compound 44(2)

(3R*)-1-butyl-2,5-dioxo-3-((1R*)-1-hydroxy-2-methylpropyl)-9-(3,5-dimethyl-1-phenylpyrazol-4-ylmethyl)-1,4,9-triazaspiro[5.5]undecane

Compound 44(3)

(3R*)-1-butyl-2,5-dioxo-3-((1R*)-1-hydroxy-2-methylpropyl)-9-(6-phenyloxypyddin-3-ylmethyl)-1,4,9-triazaspiro[5.5]undecane

Compound 44(4)

(3R*)-1-butyl-2,5-dioxo-3-((1R*)-1-hydroxy-2-methylpropyl)-9-(4-(4-methylphenyloxy)phenylmethyl)-1,4,9-triazaspiro[5.5]undecane

Compound 44(5)

(3R*)-1-butyl-2,5-dioxo-3-((1R*)-1-hydroxy-2-methylpropyl)-9-(4-cyclohexyloxyphenylmethyl)-1,4,9-triazaspiro[5.5]undecane

Compound 44(6)

(3R*)-1-butyl-2,5-dioxo-3-((1R*)-1-hydroxy-2-methylpropyl)-9-(4-(tetrahydropyran-4-yloxy)phenylmethyl)-1,4,9-triazaspiro[5.5]undecane

Compound 44(7)

(3R*)-1-butyl-2,5-dioxo-3-((1R*)-1-hydroxy-2-methylpropyl)-9-(4-(pyridin-3-yloxy)phenylmethyl)-1,4,9-triazaspiro[5.5]undecane

Compound 44(8)

(3R*)-1-butyl-2,5-dioxo-3-((1R*)-1-hydroxy-2-methylpropyl)-9-(4-isopropylphenylmethyl)-1,4,9-triazaspiro[5.5]undecane

Compound 44(9)

(3R*)-1-butyl-2,5-dioxo-3-((1R*)-1-hydroxy-2-methylpropyl)-9-(3,5-dimethyl-1-(4-methylphenyl)pyrazol-4-ylmethyl)-1,4,9-triazaspiro[5.5]undecane

Compound 44(10)

(3R*)-1-butyl-2,5-dioxo-3-((1R*)-1-hydroxy-2-methylpropyl)-9-(3-methyl-5-chloro-1-phenylpyrazol-4-ylmethyl)-1,4,9-triazaspiro[5.5]undecane

Compound 44(11)

(3R*)-1-butyl-2,5-dioxo-3-((1R*)-1-hydroxy-2-methylpropyl)-9-(4-(4-carboxyphenyloxylphenylmethyl)-1,4,9-triazaspiro[5.5]undecane

Compound 44(12)

(3R*)-1-butyl-2,5-dioxo-3-((1R*)-1-hydroxy-2-methylpropyl)-9-(3,5-dimethyl-1-(pyridin-2-yl)pyrazol-4-ylmethyl)-1,4,9-triazaspiro[5.5]undecane

Compound 44(13)

(3R*)-1-butyl-2,5-dioxo-3-((1R*)-1-hydroxy-2-methylpropyl)-9-(3,5-dimethyl-1-(4-carboxyphenyl)pyrazol-4-ylmethyl)-1,4,9-triazaspiro[5.5]undecane

Compound 45

(3R*)-1-butyl-2,5-dioxo-3-((1R*)-1-hydroxy-1-cyclohexylmethyl)-9-benzyloxycarbonyl-1,4,9-triazaspiro[5.5]undecane

Compound 46

(3R*)-1-butyl-2,5-dioxo-3-((1R*)-1-hydroxy-1-cyclohexylmethyl)-1,4,9-triazaspiro[5.5]undecane

Compound 47(1)

(3R*)-1-butyl-2,5-dioxo-3-((1R*)-1-hydroxy-1-cyclohexylmethyl)-9-(4-phenyloxyphenylmethyl)-1,4,9-triazaspiro[5.5]undecane

Compound 47(2)

(3R*)-1-butyl-2,5-dioxo-3-((1R*)-1-hydroxy-1-cyclohexylmethyl)-9-(3,5-dimethyl-1-phenylpyrazol-4-ylmethyl)-1,4,9-triazaspiro[5.5]undecane

Compound 47(3)

(3R*)-1-butyl-2,5-dioxo-3-((1R*)-1-hydroxy-1-cyclohexylmethyl)-9-(4-isopropylphenylmethyl)-1,4,9-triazaspiro[5.5]undecane

Compound 47(4)

(3R*)-1-butyl-2,5-dioxo-3-((1R*)-1-hydroxy-1-cyclohexy(methyl)-9-(4-(6-methylpyridin-3-yloxy)phenylmethyl)-1,4,9-triazaspiro[5.5]undecane

Compound 47(5)

(3R*)-1-butyl-2,5-dioxo-3-((1R*)-1-hydroxy-1-cydohexylmethyl)-9-(3,5-dimethy)-1-(4-fluorophenyl)pyrazol-4-ylmethyl)-1,4,9-triazaspiro[5.5]undecane

Compound 47(6)

(3R*)-1-butyl-2,5-dioxo-3-((1R*)-1-hydroxy-1-cyclohexylmethyl)-9-(4-(4-methoxyphenyloxy)phenylmethyl)-1,4,9-triazaspiro[5.5]undecane

Compound 47(7)

(3R*)-1-butyl-2,5-dioxo-3-((1R*)-1-hydroxy-1-cyclohexylmethyl)-9-(4-(4-fluorophenyloxy) phenylmethyl)-1,4,9-triazaspiro[5.5]undecane

Compound 47(8)

(3R*)-1-butyl-2,5-dioxo-3-((1R*)-1-hydroxy-1-cyclohexylmethyl)-9-(4-(4-methylsulfonylaminophenyloxy) phenylmethyl)-1,4,9-triazaspiro[5.5]undecane

Compound 48

(3R*)-1-(2-butynyl)-2,5-dioxo-3-((1R*)-1-hydroxy-1-cyclohexylmethyl)-9-allyloxycarbonyl-1,4,9-triazaspiro[5.5]undecane

Compound 49

(3R*)-1-(2-butynyl)-2,5-dioxo-3-((1R*)-1-hydroxy-1-cyclohexylmethyl)-1,4,9-triazaspiro[5.5]undecane

Compound 50(1)

(3R*)-1-(2-butynyl)-2,5-dioxo-3-((1R*)-1-hydroxy-1-cyclohexylmethyl)-9-(3,5-dimethyl-1-phenylpyrazol-4-ylmethyl)-1,4,9-triazaspiro[5.5]undecane

Compound 50(2)

(3R*)-1-(2-butynyl)-2,5-dioxo-3-((1R*)-1-hydroxy-1-cyclohexylmethyl)-9-(3,5-dimethyl-1-(4-methylphenyl)pyrazol-4-ylmethyl)-1,4,9-triazaspiro[5.5]undecane

Compound 50(3)

(3R*)-1-(2-butynyl)-2,5-dioxo-3-((1R*)-1-hydroxy-1-cyclohexylmethyl)-9-(4-isopropylphenylmethyl)-1,4,9-triazaspiro[5.5]undecane

Compound 50(4)

(3R*)-1-(2-butynyl)-2,5-dioxo-3-((1R*)-1-hydroxy-1-cyclohexylmethyl)-9-(4-phenyloxyphenylmethyl)-1,4,9-triazaspiro[5.5]undecane

Compound 50(5)

(3R*)-1-(2-butynyl)-2,5-dioxo-3-((1R*)-1-hydroxy-1-cyclohexylmethyl)-9-(4-(4-methylphenyloxy)phenylmethyl)-1,4,9-triazaspiro[5.5]undecane

Compound 50(6)

(3R*)-1-(2-butynyl)-2,5-dioxo-3-((1R*)-1-hydroxy-1-cyclohexylmethyl)-9-(1,4-benzodioxan-6-ylmethyl)-1,4,9-triazaspiro[5.5]undecane

Compound 51

(3R*)-1-(2-butynyl)-2,5-dioxo-3-((1R*)-1-hydroxy-2-methylpropyl)-1,4,9-triazaspiro[5.5]undecane

Compound 52(1)

(3R*)-1-(2-butynyl)-2,5-dioxo-3-((1R*)-1-hydroxy-2-methylpropyl)-9-(3,5-dimethyl-1-(4-methylphenyl)pyrazol-4-ylmethyl)-1,4,9-triazaspiro[5.5]undecane

Compound 52(2)

(3R*)-1-(2-butynyl)-2,5-dioxo-3-((1R*)-1-hydroxy-2-methylpropyl)-9-(4-(4-methylphenyloxy)phenylmethyl)-1,4,9-triazaspiro[5.5]undecane

Compound 52(3)

(3R*)-1-(2-butynyl)-2,5-dioxo-3-((1R*)-1-hydroxy-2-methylpropyl)-9-(1,4-benzodioxan-6-ylmethyl)-1,4,9-triazaspiro[5.5]undecane

Compound 52(4)

(3R*)-1-(2-butynyl)-2,5-dioxo-3-((1R*)-1-hydroxy-2-methylpropyl)-9-(4-isopropylphenylmethyl)-1,4,9-triazaspiro[5.5]undecane

Compound 52(5)

(3R*)-1-(2-butynyl)-2,5-dioxo-3-((1R*)-1-hydroxy-2-methylpropyl)-9-(4-phenyloxyphenylmethyl)-1,4,9-tciazaspiro[5.5]undecane

Compound 53

(3R*)-1-butyl-2,5-dioxo-3-((1S*)-1-hydroxy-1-cyclohexylmethyl)-9-benzyl-1,4,9-thazaspiro[5.5]undecane

Compound 54

(3R*)-1-butyl-2,5-dioxo-3-((1S*)-1-hydroxy-1-cyclohexylmethyl)-1,4,9-triazaspiro[5.5]undecane

Compound 55(1)

(3R*)-1-butyl-2,5-dioxo-3-((1S*)-1-hydroxy-1-cyclohexylmethyl)-9-(4-phenyloxyphenylmethyl)-1,4,9-triazaspiro[5.5]undecane

Compound 55(2)

(3R*)-1-butyl-2,5-dioxo-3-((1S*)-1-hydroxy-1-cyclohexylmethyl)-9-(1,4-benzodioxan-6-ylmethyl)-1,4,9-triazaspiro[5.5]undecane

Compound 55(3)

(3R*)-1-butyl-2,5-dioxo-3-((1S*)-1-hydroxy-1-cyclohexylmethyl)-9-(3,5-dimethyl-1-phenylpyrazol-4-ylmethyl)-1,4,9-triazaspiro[5.5]undecane

Compound 56

(3S)-1-butyl-2,5-dioxo-3-((1S)-1-hydroxy-2-methylpropyl)-1,4,9-triazaspiro[5.5]undecane

Compound 57(1)

(3S)-1-butyl-2,5-dioxo-3-((1S)-1-hydroxy-2-methylpropyl)-9-(3,5-dimethyl-1-phenylpyrazol-4-ylmethyl)-1,4,9-triazaspiro[5.5]undecane

Compound 57(2)

(3S)-1-butyl-2,5-dioxo-3-((1S)-1-hydroxy-2-methylpropyl)-9-(4-phenyloxyphenylmethyl)-1,4,9-triazaspiro [5.5]undecane

Compound 57(3)

(3S)-1-butyl-2,5-dioxo-3-((1S)-1-hydroxy-2-methylpropyt)-9-(1,4-benzodioxan-6-ylmethyl)-1,4,9-triazaspiro [5.5]undecane

Compound 57(4)

(3S)-1-butyl-2,5-dioxo-3-((1R)-1-hydroxy-2-methylpropyl)-9-(4-(4-methylsulfonylaminophenytoxy)phenylmethyl)-1,4,9-triazaspiro[5.5]undecane

Compound 58

(3R)-1-butyl-2,5-dioxo-3-((1R)-1-hydroxy-2-methylpropyl)-1,4,9-triazaspiro[5.5]undecane

Compound 59(1)

(3R)-1-butyl-2,5-dioxo-3-((1R)-1-hydroxy-2-methylpropyl)-9-(3,5-dimethyl-1-phenylpyrazol-4-ylmethyl)-1,4,9-triazaspiro[5.5]undecane

Compound 59(2)

(3R)-1-butyl-2,5-dioxo-3-((1R)-1-hydroxy-2-methylpropyl)-9-(4-phenyloxyphenylmethyl)-1,4,9-triazaspiro [5.5]undecane

Compound 59(3)

(3R)-1-butyl-2,5-dioxo-3-((1R)-1-hydroxy-2-methylpropyl)-9-(1,4-benzodioxan-6-ylmethyl)-1,4,9-triazaspiro [5.5]undecane

Compound 59(4)

(3R)-1-butyl-2,5-dioxo-3-((1R)-1-hydroxy-2-methylpropyl)-9-(4-(4-methylsulfonylaminophenyloxy)phenylmethyl)-1,4,9-triazaspiro[5.5]undecane

Compound 60

(3R)-1-butyl-2,5-dioxo-3-((1S)-1-hydroxy-2-methylpropyl)-1,4,9-triazaspiro[5.5]undecane

Compound 61(1)

(3R)-1-butyl-2,5-dioxo-3-((1S)-1-hydroxy-2-methylpropyl)-9-(3,5-dimethyl-1-phenylpyrazol-4-ylmethyl)-1,4,9-triazaspiro[5.5]undecane

Compound 61(2)

(3R)-1-butyl-2,5-dioxo-3-((1S)-1-hydroxy-2-methylpropyl)-9-(1,4-benzodioxan-6-ylmethyl)-1,4,9-triazaspiro [5.5]undecane

Compound 61 (3)

(3R)-1-butyl-2,5-dioxo-3-((1S)-1-hydroxy-2-methytpropyl)-9-(4-(4-methylsulfonylaminophenyloxy)phenylmethyl)-1,4,9-triazaspiro[5.5]undecane

Compound 62

(3S)-1-butyl-2,5-dioxo-3-((1R)-1-hydroxy-2-methylpropyl)-1,4,9-triazaspiro[5.5]undecane

Compound 63(1)

(3S)-1-butyl-2,5-dioxo-3-((1R)-1-hydroxy-2-methylpropyl)-9-(3,5-dimethyl-1-phenylpyrazol-4-ylmethyl)-1,4,9-triazaspiro[5.5]undecane

Compound 63(2)

(3S)-1-butyl-2,5-dioxo-3-((1R)-1-hydroxy-2-methylpropyl)-9-(1,4-benzodioxan-6-ylmethyl)-1,4,9-triazaspiro [5.5]undecane

Compound 63(3)

(3S)-1-butyl-2,5-dioxo-3-((1R)-1-hydroxy-2-methylpropyl)-9-(4-(4-methylsulfonylaminophenyloxy)phenylmethyl)-1,4,9-triazaspiro[5.5]undecane

Compound 64

(3S)-2,5-dioxo-3-(3-benzyloxycarbonylaminopropyl)-9-(2-phenylethyl)-1,4,9-triazaspiro[5.5]undecane

Compound 65

(3S)-1-methyl-2,5-dioxo-3-(3-benzyloxycarbonylaminopropyl)-9-(2-phenylethyl)-1,4,9-triazaspiro[5.5]undecane

Compound 66

(3S)-1-butyl-2,5-dioxo-3-cyclohexylmethyl-9-(4-phenyloxyphenylmethyl)-9-oxido-1,4,9-triazaspiro[5.5]undecane

Compound 67

(3R)-1-butyl-2,5-dioxo-3-((1R)-1-hydroxy-2-methylpropyl)-9-benzyl-1,4,9-triazaspiro[5.5]undecane

Compound 68

(3R)-1-butyl-2,5-dioxo-3-((1R)-1-hydroxy-2-methylpropyl)-9-(6-phenyloxypyridin-3-ylmethyl)-1,4, 9-triazaspiro[5.5]undecane

Compound 68(1)

(3R)-1-butyl-2,5-dioxo-3-((1R)-1-hydroxy-2-methylpropyl)-9-(4-(4-methoxyphenyloxy)phenylmethyl)-1,4,9-triazaspiro[5.5]undecane

Compound 68(2)

(3R)-1-butyl-2,5-dioxo-3-((1R)-1-hydroxy-2-methylpropyl)-9-(4-(3-methoxyphenyloxy)pheny(methyl)-1,4,9-triazaspiro[5.5]undecane

Compound 68(3)

(3R)-1-butyl-2,5-dioxo-3-((1R)-1-hydroxy-2-methylpropyl)-9-(4-(4-fluorophenyloxy)phenylmethyl)-1,4,9-triazaspiro[5.5]undecane

Compound 68(4)

(3R)-1-butyl-2,5-dioxo-3-((1R)-1-hydroxy-2-methylpropyl)-9-(4-(4-chlorophenyloxy)phenylmethyl)-1,4,9-triazaspiro[5.5]undecane

Compound 68(5)

(3R)-1 -butyl-2.5-dioxo-3-((1R)-1-hydroxy-2-methylpropyl)-9-(4-(phenylcarbonyl)phenylmethyl)-1,4,9-triazaspiro[5.5]undecane

Compound 68(6)

(3R)-1-butyl-2,5-dioxo-3-((1R)-1-hydroxy-2-methylpropyl)-9-(4-(1-phenyl-1-hydroxymethyl)phenylmethyl)-1,4,9-triazaspiro[5.5]undecane

Compound 68(7)

(3R)-1-butyl-2,5-dioxo-3-((1R)-1-hydroxy-2-methylpropyl)-9-(4-(4-(morpholin-4-ylcarbonyl)phenyloxy)phenylmethyl)-1,4,9-triazaspiro[5.5]undecane

Compound 68(8)

(3R)-1-butyl-2,5-dioxo-3-((1R)-1-hydroxy-2-methylpropyl)-9-(4-(6-methylpyridin-3-yloxy)phenylmethyl)-1,4,9-triazaspiro[5.5]undecane

Compound 68(9)

(3R)-1-butyl-2,5-dioxo-3-((1R)-1-hydroxy-2-methylpropyl)-9-(4-(pyridin-1-oxido-3-yloxy)phenylmethyl)-1,4,9-triazaspiro[5.5]undecane

Compound 68(10)

(3R)-1-butyl-2,5-dioxo-3-((1R)-1-hydroxy-2-methylpropyl)-9-(4-(4-hydroxypiperidin-1-ylmethyl)phenylmethyl)-1,4,9-triazaspiro[5.5]undecane

Compound 68(11)

(3R)-1-butyl-2,5-dioxo-3-((1R)-1-hydroxy-2-methylpropyl)-9-(3,5-dimethyl-1-cyclohexylpyrazol-4-ylmethyl)-1,4,9-triazaspiro[5.5]undecane

Compound 68(12)

(3R)-1-butyl-2,5-dioxo-3-((1 R)-1-hydroxy-2-methylpropyl)-9-(1,3,5-trimethylpyrazol-4-ylmethyl)-1,4,9-triazaspiro[5.5]undecane

Compound 68(13)

(3R)-1-butyl-2,5-dioxo-3-((1R)-1-hydroxy-2-methylpropyl)-9-(4-(4-aminosulfonylphenyloxy)phenylmethyl)-1,4,9-triazaspiro(5.5]undecane

Compound 68(14)

(3R)-1-butyl-2,5-dioxo-3-((1R)-1-hydroxy-2-methylpropyl)-9-(4-(4-methylthiophenyloxy)phenylmethyl)-1,4,9-triazaspiro[5.5]undecane

Compound 68(15)

(3R)-1-butyl-2,5-dioxo-3-((1R)-1-hydroxy-2-methylpropyl)-9-(4-(4-methylsulfonylphenyloxy)phenylmethyl)-1,4,9-triazaspiro[5.5]undecane

Compound 68(16)

(3R)-1-butyl-2,5-dioxo-3-((1R)-1-hydroxy-2-methylpropyl)-9-(4-(4-cyanophenyloxy)phenylmethyl)-1,4,9-triazaspiro[5.5]undecane

Compound 68(17)

(3R)-1-butyl-2,5-dioxo-3-((1R)-1-hydroxy-2-methylpropyl)-9-(4-(phenylthio)phenylmethyl)-1,4,9-triazaspiro [5.5]undecane

Compound 68(18)

(3R)-1-butyl-2,5-dioxo-3-((1R)-1-hydroxy-2-methylpropyl)-9-(3,5-dimethyl-1-(4-hydroxyphenyl)pyrazol-4-ylmethyl)-1,4,9-triazaspiro[5.5]undecane

Compound 68(19)

(3R)-1-butyt-2,5-dioxo-3-((1R)-1-hydroxy-2-methylpropyt)-9-(3,5-dimethyl-1-(4-methylsulfonylaminophenyl)pyrazol-4-ylmethyl)-1,4,9-triazaspiro[5.5]undecane

Compound 68(20)

(3R)-1-butyl-2,5-dioxo-3-((1R)-1-hydroxy-2-methylpropyl)-9-(3,5-dimethyl-1-(4-(2-(N,N-dimethylamino)ethylaminosulfonyl)phenyl)pyrazol-4-ylmethyl)-1,4,9-triazaspiro[5.5]undecane

Compound 68(21)

(3R)-1-butyl-2,5-dioxo-3-((1R)-1-hydroxy-2-methylpropyl)-9-(3,5-dimethyl-1-(4-(pyrrolidin-1-ylsulfonyl)phenyl)pyrazol-4-ylmethyl)-1,4,9-triazaspiro[5.5]undecane

Compound 68(22)

(3R)-1-butyl-2,5-dioxo-3-((1R)-1-hydroxy-2-methylpropyl)-9-(4-(6-methylpyridin-1-oxido-3-yloxy)phenylmethyl)-1,4,9-triazaspiro[5.5]undecane

Compound 68(23)

(3R)-1-butyl-2,5-dioxo-3-((1R)-1-hydroxy-2-methylpropyl)-9-(4-(4-hydroxyphenyloxy)phenylmethyl)-1,4,9-triazaspiro[5.5]undecane

Compound 68(24)

(3R)-1-butyl-2,5-dioxo-3-((1R)-1-hydroxy-2-methylpropyl)-9-(6-(4-methoxyphenyloxy)pyridin-3-ylmethyl)-1,4,9-triazaspiro[5.5]undecane

Compound 68(25)

(3R)-1-butyl-2,5-dioxo-3-((1R)-1-hydroxy-2-methylpropyl)-9-(3,5-dimethyl-1-(4-(methylaminosulfonyl)phenyl)pyrazol-4-ylmethyl)-1,4,9-triazaspiro[5.5]undecane

Compound 68(26)

(3R)-1-butyl-2,5-dioxo-3-((1R)-1-hydroxy-2-methylpropyl)-9-(3,5-dimethyl-1-(4-(N-methy)-N-(2-hydroxyethyl)aminosulfony))phenyl)pyrazol-4-ylmethy()-1,4,9-triazaspiro[5.5]undecane

Compound 68(27)

(3R)-1-butyl-2,5-dioxo-3-((1R)-1-hydroxy-2-methylpropyl)-9-(3,5-dimethyl-1-(4-(2-hydroxyethylaminosulfonyl)phenyl)pyrazol-4-ylmethyl)-1,4,9-triazaspiro[5.5]undecane

Compound 68(28)

(3R)-1-butyl-2,5-dioxo-3-((1R)-1-hydroxy-2-methylpropyl)-9-(3,5-dimethyl-1-(4-(N,N-dimethylaminocarbonyl)phenyl)pyrazol-4-ylmethyl)-1,4,9-triazaspiro[5.5]undecane

Compound 68(29)

(3R)-1-butyl-2,5-dioxo-3-((1R)-1-hydroxy-2-methylpropyl)-9-(3,5-dimethyl-1-(4-(2-(morpholin-4-yl)ethylaminosulfonyl)phenyl)pyrazol-4-ylmethyl)-1,4, 9-triazaspiro[5.5]undecane

Compound 68(30)

(3R)-1-butyl-2,5-dioxo-3-((1R)-1-hydroxy-2-methylpropyl)-9-(3,5-dimethyl-1-(4-(pyrrolidin-1-ylcarbonyl)phenyl)pyrazol-4-ylmethyl)-1,4,9-triazaspiro[5.5]undecane

Compound 68(31)

(3R)-1-butyl-2,5-dioxo-3-((1R)-1-hydroxy-2-methylpropyl)-9-(4-(4-methylsulfinylphenyloxy)phenylmethyl)-1,4,9-triazaspiro[5.5]undecane

Compound 68(32)

(3R)-1-butyl-2,5-dioxo-3-((1R)-1-hydroxy-2-methylpropyl)-9-(3,5-dimethyl-1-(4-(N,N-dimethylaminosulfonyl)phenyl)pyrazol-4-ylmethyl)-1,4,9-triazaspiro[5.5]undecane

Compound 68(33)

(3R)-1-butyl-2,5-dioxo-3-((1R)-1-hydroxy-2-methylpropyl)-9-(3,5-dimethyl-1-(4-(morpholin-4-ylsulfonyl)phenyl)pyrazol-4-ylmethyl)-1,4,9-triazaspiro[5.5]undecane

Compound 68(34)

(3R)-1-butyl-2,5-dioxo-3-((1R)-1-hydroxy-2-methylpropyl)-9-(4-(4-aminocarbonylphenyloxy)phenylmethyl)-1,4,9-triazaspiro[5.5]undecane

Compound 68(35)

(3R)-1-butyl-2,5-dioxo-3-((1R)-1-hydroxy-2-methylpropyl)-9-(4-(4-methylaminocarbonylphenyloxy)phenylmethyl)-1,4,9-triazaspiro[5.5]undecane

Compound 68(36)

(3R)-1-butyl-2,5-dioxo-3-((1R)-1-hydroxy-2-methylpropyl)-9-(3,5-dimethyl-1-(4-methylphenyl)pyrazol-4-ylmethyl)-1,4,9-triazaspiro[5.5]undecane

Compound 68(37)

(3R)-1-butyl-2,5-dioxo-3-((1R)-1-hydroxy-2-methylpropyl)-9-(3,5-dimethyl-1-(4-(N,N-diethylaminosutfonyl)phenyl)pyrazol-4-ylmethyl)-1,4,9-triazaspiro[5.5]undecane

Compound 68(38)

(3R)-1-butyl-2,5-dioxo-3-((1R)-1-hydroxy-2-methylpropyl)-9-(3,5-dimethyl-1-(4-(4-methylpiperazin-1-ylsulfonyl)phenyl)pyrazol-4-ylmethyl)-1,4,9-triazaspiro[5.5]undecane

Compound 68(39)

(3R)-1-butyl-2,5-dioxo-3-((1R)-1-hydroxy-2-methylpropyl)-9-(5-chloro-3-methyl-1-phenylpyrazol-4-ylmethyl)-1,4,9-triazaspiro[5.5]undecane

Compound 68(40)

(3R)-1-butyl-2,5-dioxo-3-((1R)-1-hydroxy-2-methylpropyl)-9-(3,5-dimethyl-1-(4-(morpholin-4-ylcarbonyl)phenyl)pyrazol-4-ylmethyl)-1,4,9-triazaspiro[5.5]undecane

Compound 68(41)

(3R)-1-butyl-2,5-dioxo-3-((1R)-1-hydroxy-2-methylpropyl)-9-(3,5-dimethyl-1-(4-(2-hydroxyethylaminocarbonyl)phenyl)pyrazol-4-ylmethyl)-1,4,9-triazaspiro[5.5]undecane

Compound 68(42)

(3R)-1-butyl-2,5-dioxo-3-((1R)-1-hydroxy-2-methylpropyl)-9-(4-(4-(2-(N,N-dimethylamino)ethylaminocarbonyl)phenyloxy)phenylmethyl)-1,4,9-triazaspiro[5.5]undecane

Compound 68(43)

(3R)-1-butyl-2,5-dioxo-3-((1R)-1-hydroxy-2-methylpropyl)-9-(4-(pyridin-3-yloxy)phenylmethyl)-1,4,9-triazaspiro[5.5]undecane

Compound 68(44)

(3R)-1-butyl-2,5-dioxo-3-((1R)-1-hydroxy-2-methylpropyl)-9-(4-(4-carboxyphenyloxy)phenylmethyl)-1,4,9-triazaspiro[5.5]undecane

Compound 68(45)

(3R)-1-butyl-2,5-diaxo-3-((1R)-1-hydroxy-2-methylpropyl)-9-(4-(4-methylphenyloxy)phenylmethyl)-1,4,9-triazaspiro[5.5]undecane

Compound 68(46)

(3R)-1-butyl-2,5-dioxo-3-((1R)-1-hydroxy-2-methylpropyl)-9-(3,5-dimethyl-1-(2,4-difluorophenyl)pyrazol-4-ylmethyl)-1,4,9-triazaspiro[5.5]undecane

Compound 68(47)

(3R)-1-butyl-2,5-dioxo-3-((1R)-1-hydroxy-2-methylpropyl)-9-(3,5-dimethyl-1-(pyridin-2-yl)pyrazol-4-ylmethyl)-1,4,9-triazaspiro[5.5]undecane

Compound 68(48)

(3R)-1-butyl-2,5-dioxo-3-((1R)-1-hydroxy-2-methylpropyl)-9-(3,5-dimethyl-1-(4-methylaminocarbonylphenyl) pyrazol-4-ylmethyl)-1,4,9-triazaspiro[5.5]undecane

Compound 68(49)

(3R)-1-butyl-2,5-dioxo-3-((1R)-1-hydroxy-2-methylpropyl)-9-(4-cyclohexyloxyphenylmethyl)-1,4,9-triazaspiro[5.5]undecane

Compound 68(50)

(3R)-1-butyl-2,5-dioxo-3-((1R)-1-hydroxy-2-methylpropyl)-9-(4-(3,4,5,6-tetrahydropyran-4-yloxy)phenylmethyl)-1,4,9-triazaspiro[5.5]undecane

Compound 68(51)

(3R)-1-butyl-2,5-dioxo-3-((1R)-1-hydroxy-2-methylpropyl)-9-(4-(4-methoxyphenylmethylaminocarbonyl)phenylmethyl)-1,4,9-triazaspiro[5.5]undecane

Compound 68(52)

(3R)-1-butyl-2,5-dioxo-3-((1R)-1-hydroxy-2-methylpropyl)-9-(4-(cyclohexylaminocarbonyl)phenylmethyl)-1,4,9-triazaspiro[5.5]undecane

Compound 68(53)

(3R)-1-butyl-2,5-dioxo-3-((1R)-1-hydroxy-2-methylpropyl)-9-(4-(4-(pyrrolidin-1-ylcarbonyl)phenyloxy)phenylmethyl)-1,4,9-triazaspiro[5.5]undecane

Compound 68(54)

(3R)-1-butyl-2,5-dioxo-3-((1R)-1-hydroxy-2-methylpropyl)-9-(3,5-dimethyl-1-(4-fluorophenyl)pyrazol-4-ylmethyl)-1,4,9-triazaspiro[5.5]undecane

Compound 68(55)

(3R)-1-butyl-2,5-dioxo-3-((1R)-1-hydroxy-2-methy)propyl)-9-(3,5-dimethy)-1-(4-(2-phenylethyl)phenyl)pyrazol-4-ylmethyl)-1,4,9-triazaspiro[5.5]undecane

Compound 68(56)

(3R)-1-butyl-2,5-dioxo-3-((1R)-1-hydroxy-2-methylpropyl)-9-(3,5-dimethyl-1-(1-benzyloxycarbonylpiperidin-4-yl) pyrazol-4-ylmethyl)-1,4,9-triazaspiro[5.5]undecane

Compound 68(57)

(3R)-1-butyl-2,5-dioxo-3-((1R)-1-hydroxy-2-methylpropyl)-9-(4-(4-(2-hydroxyethylaminocarbonyl)phenyloxy) phenylmethyl)-1,4,9-triazaspiro[5.5]undecane

Compound 68(58)

(3R)-1-butyl-2,5-dioxo-3-((1R)-1-hydroxy-2-methylpropyl)-9-(3,5-dimethyl-1-(1-methylsulfonylpiperidin-4-yl) pyrazol-4-ylmethyl)-1,4,9-triazaspiro[5.5]undecane

Compound 68(59)

(3R)-1-butyl-2, 5-dioxo-3-((1R)-1-hydroxy-2-methylpropyl)-9-(4-(4-hydroxymethylphenyloxy)phenylmethyl)-1,4,9-triazaspiro[5.5]undecane

Compound 69

(3S)-1-butyl-2,5-dioxo-3-((1S)-1-hydroxy-2-methylpropyl)-9-(6-phenyloxypyridin-3-ylmethyl)-1,4,9-triazaspiro[5.5]undecane

Compound 70

(3S)-1-butyl-2,5-dioxo-3-(2-methylpropyl)-9-(3,5-dimethyl-1-(4-(N,N-dimethylaminocarbonyl)phenyl)pyrazol-4-ylmethyl)-1,4,9-triazaspiro[5.5]undecane

Compound 70(1)

(3S)-1-butyl-2,5-dioxo-3-(2-methylpropyl)-9-(3,5-dimethyl-1-(4-(pyrrolidin-1-ylcarbonyl)phenyl)pyrazol-4-ylmethyl)-1,4,9-triazaspiro[5.5]undecane

Compound 70(2)

(3S)-1-butyl-2,5-dioxo-3-(2-methylpropyl)-9-(3,5-dimethyl-1-(4-(morpholin-4-ylcarbonyl)phenyl)pyrazol-4-ylmethyl)-1,4,9-triazaspiro[5.5]undecane

Compound 70(3)

(3S)-1-butyl-2,5-dioxo-3-(2-methylpropyl)-9-(4-methylsulfonylphenylmethyl)-1,4,9-triazaspiro[5.5]undecane

Compound 70(4)

(3S)-1-butyl-2,5-dioxo-3-(2-methylpropyl)-9-(4-(4-methylsulfonylphenyloxy)phenylmethyl)-1,4,9-triazaspiro [5.5]undecane

Compound 70(5)

(3S)-1-butyl-2,5-dioxo-3-(2-methylpropyl)-9-(3,5-dimethyl-1-(4-(2-(morpholin-4-yl)ethylaminosulfonyl)phenyl)pyrazol-4-ylmethyl)-1,4,9-triazaspiro[5.5]undecane

Compound 70(6)

(3S)-1-butyl-2,5-dioxo-3-(2-methylpropyl)-9-(3,5-dimethyl-1-(4-(4-methylpiperazin-1-ylsulfonyl)phenyl)pyrazol-4-ylmethyl)-1,4,9-triazaspiro[5.5]undecane

Compound 70(7)

(3S)-1-butyl-2,5-dioxo-3-(2-methylpropyl)-9-(4-(4-methylsulfinylphenyloxy)phenylmethyl)-1,4,9-triazaspiro [5.5]undecane

Compound 70(8)

(3S)-1-butyl-2,5-dioxo-3-(2-methylpropyl)-9-(4-(pyridin-1-oxido-3-yloxy)phenylmethyl)-1,4,9-triazaspiro[5.5] undecane

Compound 70(9)

(3S)-1-butyl-2,5-dioxo-3-(2-methylpropyl)-9-(3,5-dimethyl-1-{4-(2-hydroxyethylaminocarbonyl)phenyl)pyrazol-4-ylmethyl)-1,4,9-triazaspiro[5.5]undecane

Compound 70(10)

(3S)-1-butyl-2,5-dioxo-3-(2-methylpropyl)-9-(4-(4-(morpholin-4-ylcarbonyl)phenyloxy)phenylmethyl)-1,4,9-triazaspiro[5.5]undecane

Compound 70(11)

(3S)-1-butyl-2,5-dioxo-3-(2-methylpropyl)-9-(3,5-dimethyl-1-(4-(N,N-diethylaminosulfonyl)phenyl)pyrazol-4-ylmethyl)-1,4,9-triazaspiro[5.5]undecane

Compound 70(12)

(3S)-1-butyl-2,5-dioxo-3-(2-methylpropyl)-9-(4-(4-(2-hydroxyethylaminocarbonyl)phenyloxy)phenylmethyl)-1,4,9-triazaspiro[5.5]undecane

Compound 70(13)

(3S)-1-butyl-2,5-dioxo-3-(2-methylpropyl)-9-(4-(4-(pyrrolidin-1-ylcarbonyl)phenyloxy)phenylmethyl)-1,4,9-triazaspiro[5.5]undecane

Compound 70(14)

(3S)-1-butyl-2,5-dioxo-3-(2-methylpropyl)-9-(3,5-dimethyl-1-(4-(cyclohexylaminosulfonyl)phenyl)pyrazol-4-ylmethyl)-1,4,9-triazaspiro[5.5]undecane

Compound 70(15)

(3S)-1-butyl-2,5-dioxo-3-(2-methylpropyl)-9-(3,5-dimethyl-1-(4-(3-methoxypropylaminosulfonyl)phenyl)pyrazol-4-ylmethyl)-1,4,9-triazaspiro[5.5]undecane

Compound 70(16)

(3S)-1-butyl-2,5-dioxo-3-(2-methylpropyl)-9-(4-methylsulfinylphenylmethyl)-1,4,9-triazaspiro[5.5]undecane

Compound 70(17)

(3S)-1-butyl-2,5-dioxo-3-(2-methylpropyl)-9-(3,5-dimethyl-1-propylpyrazol-4-ylmethyl)-1,4,9-triazaspiro[5.5] undecane

Compound 70(18)

(3S)-1-butyl-2,5-dioxo-3-(2-methylpropyl)-9-(3,5-dimethyl-1-ethylpyrazol-4-ylmethyl)-1,4,9-triazaspiro[5.5] undecane

Compound 70(19)

(3S)-1-butyl-2,5-dioxo-3-(2-methylpropyl)-9-(3,5-dimethyl-1-cyclopentylpyrazol-4-ylmethyl)-1,4,9-triazaspiro [5.5]undecane

Compound 70(20)

(3S)-1-butyl-2,5-dioxo-3-(2-methylpropyl)-9-(3,5-dimethyl-1-(1,1-dimethylethyl)pyrazol-4-ylmethyl)-1,4,9-triazaspiro[5.5]undecane

Compound 70(21)

(3S)-1-butyl-2,5-dioxo-3-(2-methylpropyl)-9-(3,5-dimethyl-1-(1-benzyloxycarbonylpiperidin-4-yl)pyrazol-4-ylmethyl)-1,4,9-triazaspiro[5.5]undecane

Compound 70(22)

(3S)-1-butyl-2,5-dioxo-3-(2-methylpropyl)-9-(4-((4-methoxyphenyl)methylaminocarbonyl)phenylmethyl)-1,4,9-triazaspiro[5.5]undecane

Compound 70(23)

(3S)-1-butyl-2,5-dioxo-3-(2-methylpropyl)-9-(4-(3-methoxypropylaminocarbonyl)phenyimethyl)-1,4,9-triazaspiro[5.5]undecane

Compound 70(24)

(3S)-1-butyl-2,5-dioxo-3-(2-methylpropyl)-9-(3,5-dimethyl-1-(4-methoxycarbonylphenyl)pyrazol-4-ylmethyl)-1,4,9-triazaspiro[5.5]undecane

Compound 70(25)

(3S)-1-butyl-2,5-dioxo-3-(2-methylpropyl)-9-(3,5-dimethyl-1-(4-methoxyphenyl)pyrazol-4-ylmethyl)-1,4,9-triazaspiro[5.5]undecane

Compound 70(26)

(3S)-1-butyl-2,5-dioxo-3-(2-methylpropyl}-9-(3,5-dimethyl-1-(4-(3-(morpholin-4-yl)propylaminosulfonyl)phenyl)pyrazol-4-ylmethyl)-1,4,9-triazaspiro[5.5]undecane

Compound 70(27)

(3S)-1-butyl-2,5-dioxo-3-(2-methylpropyl)-9-(4-(pyrrolidin-1-ylcarbonyl)phenylmethyl)-1,4,9-triazaspiro[5.5] undecane

Compound 70(28)

(3S)-1-butyl-2,5-dioxo-3-(2-methylpropyl)-9-(4-(piperidin-1-ylcarbonyl)phenylmethyl)-1,4,9-triazaspiro[5.5]undecane

Compound 70(29)

(3S)-1-butyl-2,5-dioxo-3-(2-methylpropyl)-9-(4-(morpholin-4-ylcarbonyl)phenylmethyl)-1,4,9-triazaspiro[5.5] undecane

Compound 70(30)

(3S)-1-butyl-2,5-dioxo-3-(2-methy(propyl)-9-(4-(N-methyl-N-(2-(pyridin-2-yl)ethyl)aminocarbonyl)phenylmethyl)-1,4,9-triazaspiro[5.5]undecane

Compound 70(31)

(3S)-1-butyl-2,5-dioxo-3-(2-methylpropyl)-9-(4-(cyclohexylaminocarbonyl)phenylmethyl)-1,4,9-triazaspiro [5.5]undecane

Compound 70(32)

(3S)-1-butyl-2,5-dioxo-3-(2-methylpropyl)-9-(4-(N,N-dimethylaminosulfonyl)phenylmethyl)-1,4,9-triazaspiro [5.5]undecane

Compound 70(33)

(3S)-1-butyl-2,5-dioxo-3-(2-methylpropyl)-9-(4-(4-methoxycarbonylphenyloxy)phenylmethyl)-1,4,9-triazaspiro[5.5]undecane

Compound 70(34)

(3S)-1-butyl-2,5-dioxo-3-(2-methylpropyl)-9-(3,5-dimethyl-1-(1-methylpiperidin-4-yl)pyrazol-4-ylmethyl)-1,4,9-triazaspiro(5.5]undecane

Compound 70(35)

(3S)-1-butyl-2,5-dioxo-3-(2-methylpropyl)-9-(3,5-dimethyl- 1-(1-methylsulfonylpiperidin-4-yl)pyrazol-4-ylmethyl)-1,4,9-triazaspiro[5.5]undecane

Compound 70(36)

(3S)-1-butyl-2,5-dioxo-3-(2-methylpropyl)-9-(3,5-dimethyl-1-(4-(3-(N, N-dimethylamino)propylaminosulfonyl) phenyl)pyrazol-4-ylmethyl)-1,4,9-triazaspiro[5.5]undecane

Compound 70(37)

(3S)-1-butyl-2,5-dioxo-3-(2-methylpropyl)-9-(4-(4-(N, N-dimethylamino)phenyloxy)phenylmethyl)-1,4,9-triazaspiro[5.5]undecane

Compound 70(38)

(3S)-1-butyl-2,5-dioxo-3-(2-methylpropyl)-9-(3,5-dimethyl-1-(4-(N-methyl-N-(2-(N',N'-dimethylamino)ethyl) aminosulfonylphenyl)pyrazol-4-ylmethyl)-1,4,9-triazaspiro[5.5]undecane

Compound 70(39)

(3S)-1-butyl-2,5-dioxo-3-(2-methylpropyl)-9-(4-(4-((N,N-dimethylamino)methyl)phenyloxy)phenylmethyl)-1,4,9-triazaspiro[5.5]undecane

Compound 70(40)

(3S)-1-butyl-2,5-dioxo-3-(2-methylpropyl)-9-(4-(4-carboxyphenyloxy)phenylmethyl)-1,4,9-triazaspiro[5.5]undecane

Compound 70(41)

(3S)-1-butyl-2,5-dioxo-3-(2-methylpropyl-9-(3,5-dimethyl-1-(4-methylaminocarbonylphenyl)pyrazol-4-ylmethyl)-1,4,9-triazaspiro[5.5]undecane

Compound 70(42)

(3S)-1-butyl-2,5-dioxo-3-(2-methylpropyl)-9-(4-((methoxycarbonyl)methylaminocarbonyl)phenylmethyl)-1,4,9-triazaspiro[5.5]undecane

Compound 70(43)

(3S)-1-butyl-2,5-dioxo-3-(2-methylpropyl)-9-(3-(3,5-dimethyl-1-phenylpyrazol-4-yl)-2E-propenyl)-1,4,9-triazaspiro[5.5]undecane

Compound 71

(3S)-1-butyl-2,5-dioxo-3-(2-methylpropyl)-9-(4-(carboxymethylaminocarbonyl)phenylmethyl)-1,4,9-triazaspiro[5.5]undecane

Compound 72

(3S)-1-butyl-2,5-dioxo-3-(2-methylpropyl)-9-{3-(3,5-dimethyl-1-phenylpyrazol-4-yl)propyl)-1,4,9-triazaspiro[5.5]undecane

Compound 73

(3S)-1-butyl-2,5-dioxo-3-cyclohexylmethyl-9-(3,5-dimethyl-1-(4-(N, N-dimethylaminocarbonyl)phenyl)pyrazol-4-ylmethyl)-1,4,9-triazaspiro[5.5]undecane

Compound 73(1)

(3S)-1-butyl-2,5-dioxo-3-cyclohexylmethyl-9-(3,5-dimethyl-1-(4-(pyrrolidin-1-ylcarbonyl)phenyl)pyrazol-4-ylmethyl)-1,4,9-triazaspiro[5.5]undecane

Compound 73(2)

(3S)-1-butyl-2,5-dioxo-3-cyclohexylmethyl-9-(3,5-dimethyl-1-(4-(morphoiin-4-ylcarbonyl)phenyl)pyrazol-4-ylmethyl)-1,4,9-triazaspiro[5.5]undecane

Compound 73(3)

(35)-1-butyl-2,5-dioxo-3-cyclohexylmethyl-9-(3,5-dimethyl-1-(4-(2-(N,N-dimethylamino)ethylaminocarbonyl) phenyl)pyrazol-4-ylmethyl)-1,4,9-triazaspiro[5.5]undecane

Compound 73(4)

(3S)-1-butyl-2,5-dioxo-3-cyclohexylmethyl-9-(4-(4-(morpholin-4-ylcarbonyl)phenyloxy)phenylmethyl)-1,4,9-triazaspiro[5.5]undecane

Compound 73(5)

(3S)-1-butyl-2,5-dioxo-3-cyclohexylmethyl-9-(4-methylsulfonylphenylmethyl)-1,4,9-triazaspiro[5.5]undecane

Compound 73(6)

(3S)-1-butyl-2,5-dioxo-3-cyclohexylmethyl-9-(4-(4-methylsulfonylphenyloxy)phenylmethyl)-1,4,9-triazaspiro [5.5]undecane

Compound 73(7)

(3S)-1-butyl-2,5-dioxo-3-cyclohexylmethyl-9-(3,5-dimethyl-1-(4-(2-(morpholin-4-yl)ethylaminosulfonyl)phenyl)pyrazol-4-ylmethyl)-1,4,9-triazaspiro[5.5]undecane

Compound 73(8)

(3S)-1-butyl-2,5-dioxo-3-cyclohexylmethyl-9-(3,5-dimethyl-1-(4-(4-methylpiperazin-1-ylsulfonyl)phenyl)pyrazol-4-ylmethyl)-1,4,9-triazaspiro[5.5]undecane

Compound 73(9)

(3S)-1-butyl-2,5-dioxo-3-cyclohexylmethyl-9-(4-(4-methylsulfinylphenyloxy)phenylmethyl)-1,4,9-triazaspiro [5.5]undecane

Compound 73(10)

(35)-1-butyl-2,5-dioxo-3-cyclohexylmethyl-9-(3,5-dimethyl-1-(4-(2-hydroxyethylaminocarbonyl)phenyl)pyrazol-4-ylmethyl)-1,4,9-triazaspiro[5.5]undecane

Compound 73(11)

(3S)-1-butyl-2,5-dioxo-3-cyclohexylmethyl-9-(4-(4-(2-hydroxyethylaminocarbonyl)phenyloxy)phenylmethyl)-1,4,9-triazaspiro[5.5]undecane

Compound 73(12)

(3S)-1-butyl-2,5-dioxo-3-cyclohexylmethyl-9-(4-(4-(pyrrolidin-1-ylcarbonyl)phenyloxy)phenylmethyl)-1,4,9-triazaspiro[5.5]undecane

Compound 73(13)

(3S)-1-butyl-2,5-dioxo-3-cyclohexylmethyl-9-(3,5-dimethyl-1-(4-(cyclohexylaminosulfonyl)phenyl)pyrazol-4-ylmethyl)-1,4,9-triazaspiro[5.5]undecane

Compound 73(14)

(3S)-1-butyl-2,5-dioxo-3-cyclohexylmethyl-9-(3,5-dimethyl-1-(4-(3-methoxypropylaminosulfonyl)phenyl) pyrazol-4-ylmethyl)-1,4,9-triazaspiro[5.5]undecane

Compound 73(15)

(3S)-1-butyl-2,5-dioxo-3-cyclohexylmethyl-9-(4-methylsuffinylphenylmethyl)-1,4,9-triazaspiro[5.5]undecane

Compound 73(16)

(3S)-1-butyl-2,5-dioxo-3-cyclohexylmethyl-9-(3,5-dimethyl-1-propylpyrazol-4-ylmethyl)-1,4,9-triazaspiro[5.5] undecane

Compound 73(17)

(3S)-1-butyl-2,5-dioxo-3-cyclohexylmethyl-9-(3,5-dimethyl-1-ethylpyrazol-4-ylmethyl)-1,4,9-triazaspiro[5.5] undecane

Compound 73(18)

(3S)-1-butyl-2,5-dioxo-3-cyclohexylmethyl-9-(3,5-dimethyl-1-cyclopentylpyrazol-4-ylmethyl)-1,4,9-triazaspiro[5.5]undecane

Compound 73(19)

(3S)-1-butyl-2,5-dioxo-3-cyclohexylmethyl-9-(3,5-dimethyl-1-(4-(3-(morpholin-4-yl)propylaminosulfonyl)phenyl)pyrazol-4-ylmethyl)-1,4,9-triazaspiro[5.5]undecane

Compound 73(20)

(3S)-1-butyl-2,5-dioxo-3-cyclohexylmethyl-9-(4-(N, N-dimethylaminosulfony)phenylmethyl)-1,4,9-triazaspiro [5.5]undecane

Compound 73(21)

(3S)-1-butyl-2,5-dioxo-3-cyclohexylmethyl-9-(4-(pyrrolidin-1-ylcarbonyl)phenylmethyl)-1,4,9-triazaspiro[5.5] undecane

Compound 73(22)

(3S)-1-butyl-2,5-dioxo-3-cyclohexylmethyl-9-(4-(4-(N, N-dimethylamino)phenyloxy)phenylmethyl)-1,4,9-triazaspiro[5.5]undecane

Compound 73(23)

(3S)-1-butyl-2,5-dioxo-3-cyclohexylmethyl-9-(4-(cyclohexylaminocarbonyl)phenylmethyl)-1,4,9-triazaspiro [5.5]undecane

Compound 73(24)

(3S)-1-butyl-2,5-dioxo-3-cyclohexylmethyl-9-(3,5-dimethyl-1-(4-methoxycarbonylphenyl)pyrazol-4-ylmethyl)-1,4,9-triazaspiro[5.5]undecane

Compound 73(25)

(3S)-1-butyl-2,5-dioxo-3-cyclohexylmethyl-9-(4-(3-methoxypropylaminocarbonyl)phenylmethyl)-1,4,9-triazaspiro[5.5]undecane

Compound 73(26)

(3S)-1-butyl-2,5-dioxo-3-cyclohexylmethyl-9-(4-(N-methyl-N-(2-(pyridin-2-yl)ethyl)aminocarbonyl)phenylmethyl)-1,4,9-triazaspiro[5.5]undecane

Compound 73(27)

(3S)-1-butyl-2,5-dioxo-3-cyclohexylmethyl-9-(4-((4-methoxyphenyl)methylaminocarbonyl)phenylmethyl)-1,4,9-triazaspiro[5.5]undecane

Compound 73(28)

(3S)-1-butyl-2,5-dioxo-3-cyclohexylmethyl-9-(4-(4-methoxycarbonylphenyloxy)phenylmethyl)-1,4,9-triazaspiro[5.5]undecane

Compound 73(29)

(3S)-1-butyl-2,5-dioxo-3-cyclohexylmethyl-9-(3,5-dimethyl-1-(4-methoxyphenyl)pyrazol-4-ylmethyl)-1,4,9-triazaspiro[5.5]undecane

Compound 73(30)

(3S)-1-butyl-2,5-dioxo-3-cyclohexylmethyl-9-(3,5-dimethyl-1-(1-methylpiperidin-4-yl)pyrazol-4-ylmethyl)-1,4,9-triazaspiro[5.5]undecane

Compound 73(31)

(3S)-1-butyl-2,5-dioxo-3-cyclohexylmethyl-9-(3,5-dimethyl-1-(1-methylsulfonylpiperidin-4-yl)pyrazol-4-ylmethyl)-1,4,9-triazaspiro[5.5]undecane

Compound 73(32)

(3S)-1-butyl-2,5-dioxo-3-cyclohexylmethyl-9-(3,5-dimethyl-1-(4-(3-(N, N-dimethylamino)propylaminosulfonyl)phenyl)pyrazol-4-ylmethyl)-1,4,9-triazaspiro[5.5]undecane

Compound 73(33)

(3S)-1-butyl-2,5-dioxo-3-cyclohexylmethyl-9-(3,5-dimethyl-1-(4-(N-methyl-N-(2-(N', N'-dimethylamino)ethyl) aminosulfonylphenyl)pyrazol-4-ylmethyl)-1,4,9-triazaspiro[5.5]undecane

Compound 73(34)

(3S)-1-butyl-2,5-dioxo-3-cyclohexylmethyl-9-(4-(piperidin-1-ylcarbonyl)phenylmethyl)-1,4,9-triazaspiro[5.5] undecane

Compound 73(35)

(3S)-1-butyl-2,5-dioxo-3-cyclohexy[methyl-9-(4-(morpholin-4-ylcarbonyl)phenylmethyl)-1,4,9-tnazaspiro [5.5]undecane

Compound 73(36)

(3S)-1-butyl-2,5-dioxo-3-cyclohexylmethyl-9-(4-(4-((N,N-dimethylamino)methyl)phenyloxy)phenylmethyl)-1,4,9-triazaspiro[5.5]undecane

Compound 73(37)

(3S)-1-butyl-2,5-dioxo-3-cyclohexylmethyl-9-(3,5-dimethyl-1-(4-methylaminocarbonylphenyl)pyrazol-4-ylmethyl)-1,4,9-triazaspiro[5.5]undecane

Compound 73(38)

(3S)-1-butyl-2,5-dioxo-3-cyclohexylmethyl-9-(3,5-dimethyl-1-(1,1-dimethylethyl)pyrazol-4-ylmethyl)-1,4,9-triazaspiro[5.5]undecane

Compound 73(39)

(3S)-1-butyl-2,5-dioxo-3-cyclohexylmethyl-9-(3,5-dimethyl-1-(1-benzyloxycarbonylpiperidin-4-yl)pyrazol-4-ylmethyl)-1,4,9-triazaspiro[5.5]undecane

Compound 73(40)

(3S)-1-butyl-2,5-dioxo-3-cyclohexylmethyl-9-(4-(4-hydroxymethylphenyloxy)phenylmethyl)-1,4,9-triazaspiro [5.5]undecane

Compound 73(41)

(3S)-1-butyl-2,5-dioxo-3-cyclohexylmethyl-9-(4-((methoxycarbonyl)methylaminocarbonyl)phenylmethyl)-1,4,9-triazaspiro[5.5]undecane

Compound 74

(3S)-1-butyl-2,5-dioxo-3-cyclohexylmethyl-9-(4-(carboxymethylaminocarbonyl)phenylmethyl)-1,4,9-triazaspiro[5.5]undecane

Compound 75

(3R)-1-butyl-2,5-dioxo-3-((1R)-1-hydroxy-1-cyclohexylmethyl)-9-(4-phenyloxyphenylmethyl)-1,4,9-triazaspiro[5.5]undecane

Compound 75(1)

(3R)-1-butyl-2,5-dioxo-3-((1R)-1-hydroxy-1-cyclohexylmethyl)-9-(6-phenyloxypyridin-3-ylmethyl)-1,4,9-triazaspiro[5.5]undecane

Compound 75(2)

(3R)-1-butyl-2,5-dioxo-3-((1R)-1-hydroxy-1-cyclohexylmethyl)-9-(4-(4-fluorophenyloxy)phenylmethyl)-1,4,9-triazaspiro[5.5]undecane

Compound 75(3)

(3R)-1-butyl-2,5-dioxo-3-((1R)-1-hydroxy-1-cyclohexylmethyl)-9-(4-(4-chlorophenyloxy)phenylmethyl)-1,4,9-triazaspiro[5.5]undecane

Compound 75(4)

(3R)-1-butyl-2,5-dioxo-3-((1R)-1-hydroxy-1-cyclohexylmethyl)-9-(4-(4-cyanophenyloxy)phenylmethyl)-1,4,9-triazaspiro[5.5]undecane

Compound 75(5)

(3R)-1-butyl-2,5-dioxo-3-((1R)-1-hydroxy-1-cyclohexylmethyl)-9-(4-(4-methylsulfonylaminophenyloxy)phenylmethyl)-1,4,9-triazaspiro[5.5]undecane

Compound 75(6)

(3R)-1-butyl-2,5-dioxo-3-((1R)-1-hydroxy-1-cyclohexylmethyl)-9-(4-(6-methylpyridin-3-yloxy)phenylmethyl)-1,4,9-triazaspiro[5.5]undecane

Compound 75(7)

(3R)-1-butyl-2,5-dioxo-3-((1R)-1-hydroxy-1-cyclohexylmethyl)-9-(4-(1-methylethyl)phenylmethyl)-1,4,9-triazaspiro[5.5]undecane

Compound 75(8)

(3R)-1-butyl-2,5-dioxo-3-((1R)-1-hydroxy-1-cyclohexylmethyl)-9-(4-(4-methylsulfinylphenyloxy)phenylmethyl)-1,4,9-triazaspiro[5.5]undecane

Compound 75(9)

(3R)-1-butyl-2,5-dioxo-3-((1R)-1-hydroxy-1-cyclohexylmethyl)-9-(4-(3，4，5,6-tetrahydropyran-4-yloxy)phenylmethyl)-1,4,9-triazaspiro[5.5]undecane

Compound 75(10)

(3R)-1-butyl-2,5-dioxo-3-((1R)-1-hydroxy-1-cyclohexylmethyl)-9-(4-phenylcarbonylphenylmethyl)-1,4,9-triazaspiro[5.5]undecane

Compound 75(11)

(3R)-1-butyl-2,5-dioxo-3-((1R)-1-hydroxy-1-cyclohexylmethyl)-9-(4-(1-phenyl-1-hydroxymethyl)phenylmethyl)-1,4,9-triazaspiro[5.5]undecane

Compound 75(12)

(3R)-1-butyl-2,5-dioxo-3-((1R)-1-hydroxy-1-cyclohexylmethyl)-9-(3,5-dimethyl-1-(4-(morpholin-4-ylsulfonyl) phenyl)pyrazol-4-ylmethyl)-1,4,9-triazaspiro[5.5]undecane

Compound 75(13)

(3R)-1-butyl-2,5-dioxo-3-((1R)-1-hydroxy-1-cyclohexylmethyl)-9-(3,5-dimethyl-1-(4-methylaminosulfanylphenyl)pyrazol-4-ylmethyl)-1,4,9-triazaspira[5.5]undecane

Compound 75(14)

(3R)-1-butyl-2,5-dioxo-3-((1R)-1-hydroxy-1-cyclohexylmethyl)-9(3,5-dimethyl-1-(4-(N-methyl-N-(2-hydroxyethyl)aminosulfonyl)phenyl)pyrazol-4-ylmethyl)-1,4,9-triazaspiro[5.5]undecane

Compound 75(15)

(3R)-1-butyl-2,5-dioxo-3-((1R)-1-hydroxy-1-cyclohexylmethyl)-9-(3,5-dimethyl-1-(pyridin-2-yl)pyrazol-4-ylmethyl)-1,4,9-triazaspiro[5.5]undecane

Compound 75(16)

(3R)-1-butyl-2,5-dioxo-3-((1R)-1-hydroxy-1-cyclohexylmethyl)-9-(3,5-dimethyl-1-cyclohexylpyrazol-4-ylmethyl)-1,4,9-triazaspiro[5.5]undecane

Compound 75(17)

(3R)-1-butyl-2,5-dioxo-3-((1R)-1-hydroxy-1-cyclohexylmethyl)-9-(1,3,5-trimethylpyrazol-4-ylmethyl)-1,4,9-triazaspiro[5.5]undecane

Compound 75(18)

(3R)-1-butyl-2,5-dioxo-3-((1R)-1-hydroxy-1-cyclohexylmethyl)-9-(3,5-dimethyl-1-(4-(N,N-dimethylaminocarbonyl)phenyl)pyrazol-4-ylmethyl)-1,4,9-triazaspiro[5.5]undecane

Compound 75(19)

(3R)-1-butyl-2,5-dioxo-3-((1R)-1-hydroxy-1-cyclohexylmethyl)-9-(4-(N,N-bismethylsulfonyl)phenylmethyl)-1,4,9-triazaspiro[5.5]undecane

Compound 75(20)

(3R)-1-butyl-2,5-dioxo-3-((1R)-1-hydroxy-1-cyclohexylmethyl)-9-(3,5-dimethyl-1-(4-methylsulfonylaminophenyl)pyrazol-4-ylmethyl)-1,4,9-triazaspiro[5.5]undecane

Compound 75(21)

(3R)-1-butyl-2,5-dioxo-3-((1R)-1-hydroxy-1-cyclohexylmethyl)-9-(3,5-dimethyl-1-(4-(N,N-dimethylsulfonyl) phenyl)pyrazol-4-ylmethyl)-1,4,9-triazaspiro[5.5]undecane

Compound 75(22)

(3R)-1-butyl-2,5-dioxo-3-((1R)-1-hydroxy-1-cyclohexylmethyl)-9-(3, 5-dimethyl-1-(4-(pyrrolidin-1-ylcarbonyl) phenyl)pyrazol-4-ylmethyl)-1,4,9-triazaspiro[5.5]undecane

Compound 75(23)

(3R)-1-butyl-2,5-dioxo-3-((1R)-1-hydroxy-1-cyclohexylmethyl)-9-(3,5-dimethyl-1-(4-(morpholin-4-ylcarbonyl) phenyl)pyrazol-4-ylmethyl)-1,4,9-triazaspiro[5.5]undecane

Compound 75(24)

(3R)-1-butyl-2,5-dioxo-3-((1R)-1-hydroxy-1-cyclohexylmethyl)-9-(4-aminocarbonylphenylmethyl)-1,4,9-triazaspiro[5.5]undecane

Compound 75(25)

(3R)-1-butyl-2,5-dioxo-3-((1R)-1-hydroxy-1-cyclohexylmethyl)-9-(4-(4-aminocarbonylphenyloxy)phenylmethyl)-1,4,9-triazaspiro[5.5]undecane

Compound 75(26)

(3R)-1-butyl-2, 5-dioxo-3-((1R)-1-hydroxy-1-cyclohexylmethyl)-9-(4-(4-aminosulfonylphenyloxy)phenylmethyl)-1,4,9-triazaspiro[5.5]undecane

Compound 75(27)

(3R)-1-butyl-2, 5-dioxo-3-((1R)-1-hydroxy-1-cyclohexylmethyl)-9-(4-(B-methylpyridin-1-oxido-3-yloxy)phenylmethyl)-1,4,9-triazaspiro[5.5]undecane

Compound 75(28)

(3R)-1-butyl-2,5-dioxo-3-((1R)-1-hydroxy-1-cyclohexylmethyl)-9-(4-(4-hydroxyphenyloxy)phenylmethyl)-1,4,9-triazaspiro[5.5]undecane

Compound 75(29)

(3R)-1-butyl-2,5-dioxo-3-((1R)-1-hydroxy-1-cyclohexylmethyl)-9-(3,5-dimethyl-1-(4-hydroxyphenyl)pyrazol-4-ylmethyl)-1,4,9-triazaspiro[5.5]undecane

Compound 75(30)

(3R)-1-butyl-2,5-dioxo-3-((1R)-1-hydroxy-1-cyclohexylmethyl)-9-(3,5-dimethyl-1-(4-(2-hydroxyethylaminosulfonyl)phenyl)pyrazol-4-ylmethyl)-1,4,9-triazaspiro[5.5]undecane

Compound 75(31)

(3R)-1-butyl-2,5-dioxo-3-((1R)-1-hydroxy-1-cyclohexylmethyl)-9-(3,5-dimethyl-1-(4-(pyrrolidin-1-ylsulfonyl) phenyl)pyrazol-4-ylmethyl)-1,4,9-triazaspiro[5.5]undecane

Compound 75(32)

(3R)-1-butyl-2,5-dioxo-3-((1R)-1-hydroxy-1-cyclohexylmethyl)-9-(5-chloro-3-methyl-1-phenylpyrazol-4-ylmethyl)-1,4,9-triazaspiro[5.5]undecane

Compound 75(33)

(3R)-1-butyl-2,5-dioxo-3-((1R)-1-hydroxy-1-cyclohexytmethyl)-9-(4-(4-methoxyphenyloxy)phenylmethyl)-1,4,9-triazaspiro[5.5]undecane

Compound 75(34)

(3R)-1-butyl-2,5-dioxo-3-((1R)-1-hydroxy-1-cyclohexylmethyl)-9-(4-(3-methoxyphenyloxy)phenylmethyl)-1,4,9-triazaspiro[5.5]undecane

Compound 75(35)

(3R)-1-butyl-2,5-dioxo-3-((1R)-1-hydroxy-1-cyclohexylmethyl)-9-(4-(N,N-dimethylaminocarbonyt)phenylmethyl)-1,4,9-triazaspiro[5.5]undecane

Compound 75(36)

(3R)-1-butyl-2,5-dioxo-3-((1R)-1-hydroxy-1-cyclohexylmethyl)-9-(3,5-dimethyl-1-phenylpyrazol-4-ylmethyl)-1,4,9-triazaspiro[5.5]undecane

Compound 75(37)

(3R)-1-butyl-2,5-dioxo-3-((1R)-1-hydroxy-1-cyclohexylmethyl)-9-(3,5-dimethyl-1-(4-methylphenyl)pyrazol-4-ylmethyl)-1,4,9-triazaspiro[5.5]undecane

Compound 75(38)

(3R)-1-butyl-2,5-dioxo-3-((1R)-1-hydroxy-1-cyclohexylmethyl)-9-(3,5-dimethyl-1-(4-fluorophenyl)pyrazol-4-ylmethyl)-1,4,9-triazaspiro[5.5]undecane

Compound 75(39)

(3R)-1-butyl-2,5-dioxo-3-((1R)-1-hydroxy-1-cyclohexylmethyl)-9-(6-(4-methoxyphenyloxy)pyridin-3-ylmethyl)-1,4,9-triazaspiro[5.5]undecane

Compound 75(40)

(3R)-1-butl)-2,5-dioxo-3-((1R)-1-hydroxy-1-cyclohexylmethyl)-9-(4-(4-methylsulfonylphenyloxy)phenylmethyl)-1,4,9-triazaspiro[5.5]undecane

Compound 75(41)

(3R)-1-butyl-2,5-dioxo-3-((1R)-1-hydroxy-1-cyclohexylmethyl)-9-(4-(4-(2-(N,N-dimethyiamino)ethylaminocarbonyl)phenyloxy)phenylmethyl)-1,4,9-triazaspiro[5.5]undecane

Compound 75(42)

(3R)-1-butyl-2,5-dioxo-3-((1R)-1-hydroxy-1-cyclohexylmethyl)-9-(3,5-dimethyl-1-(4-(2-hydroxyethylaminocarbonyl)phenyl)pyrazol-4-ylmethyl)-1,4,9-triazaspiro[5.5]undecane

Compound 75(43)

(3R)-1-butyl-2,5-dioxo-3-((1R)-1-hydroxy-1-cyclohexylmethyl)-9-(3,5-dimethyl-1-(4-(2-(N,N-dimethylamino) ethylaminocarbonyl)phenyl)pyrazol-4-ylmethyl)-1,4,9-triazaspiro[5.5]undecane

Compound 75(44)

(3R)-1-butyl-2,5-dioxo-3-((1R)-1-hydroxy-1-cyclohexylmethyl)-9-(3,5-dimethyl-1-(4-(2-(morpholin-4-yl)ethylaminosulfonyl)phenyl)pyrazol-4-ylmethyl)-1,4,9-triazaspiro[5.5]undecane

Compound 75(45)

(3R)-1-butyl-2,5-dioxo-3-((1R)-1-hydroxy-1-cyclohexylmethyl)-9-(4-(4-(morpholin-4-ylcarbonyl)phenyloxy)phenylmethyl)-1,4,9-triazaspiro[5.5]undecane

Compound 75(46)

(3R)-1-butyl-2,5-dioxo-3-((1R)-1-hydroxy-1-cyclohexylmethyl)-9-(1,4-benzodioxan-6-ylmethyl)-1,4,9-triazaspiro[5.5]undecane

Compound 75(47)

(3R)-1-butyl-2,5-dioxo-3-((1R)-1-hydroxy-1-cyclohexylmethyl)-9-(3,5-dimethyl-1-(4-(N, N-diethylaminosulfonyl)phenyl)pyrazol-4-ylmethyl)-1,4,9-triazaspiro[5.5]undecane

Compound 75(48)

(3R)-1-butyl-2,5-dioxo-3-((1R)-1-hydroxy-1-cyclohexylmethyl)-9-(4-(pyridin-1-oxido-3-yloxy)phenylmethyl)-1,4,9-triazaspiro[5.5]undecane

Compound 75(49)

(3R)-1-butyl-2,5-dioxo-3-((1R)-1-hydroxy-1-cyclohexylmethyl)-9-(3,5-dimethyl-1-(4-(4-methylpiperazin-1-ylsulfonyl)phenyl)pyrazol-4-ylmethyl)-1,4,9-triazaspiro[5.5]undecane

Compound 75(50)

(3R)-1-butyl-2,5-dioxo-3-((1R)-1-hydroxy-1-cyclohexylmethyl)-9-(4-(4-methylaminocarbonylphenyloxy)phenylmethyl)-1,4,9-triazaspiro[5.5]undecane

Compound 75(51)

(3R)-1-butyl-2,5-dioxo-3-((1R)-1-hydroxy-1-cyclohexylmethyl)-9-(3,5-dimethyl-1-(2,4-difluorophenyl)pyrazol-4-ylmethyl)-1,4,9-triazaspiro[5.5]undecane

Compound 75(52)

(3R)-1-butyl-2,5-dioxo-3-((1R)-1-hydroxy-1-cyclohexylmethyl)-9-(3,5-dimethyl-1-(4-(2-(N,N-dimethylamino)ethylaminosulfonyl)phenyl)pyrazol-4-yimethyl)-1,4,9-triazaspiro[5.5]undecane

Compound 75(53)

(3R)-1-butyl-2,5-dioxo-3-((1R)-1-hydroxy-1-cyctohexylmethyl)-9-(3,5-dimethyl-1-(4-methylaminocarbonylphenyl)pyrazol-4-ylmethyl)-1,4,9-triazaspiro[5.5]undecane

Compound 75(54)

(3R)-1-butyl-2,5-dioxo-3-((1R)-1-hydroxy-1-cyclohexylmethyl)-9-(4-(4-carboxyphenyloxy)phenylmethyl)-1,4,9-triazaspiro[5.5]undecane

Compound 75(55)

(3R)-1-butyl-2,5-dioxa-3-((1R)-1-hydroxy-1-cyclohexylmethyl)-9-(4-((4-methoxyphenyl)methylaminocarbonyl)phenylmethyl)-1,4,9-triazaspiro[5.5]undecane

Compound 75(56)

(3R)-1-butyl-2,5-dioxo-3-((1R)-1-hydroxy-1-cyclohexylmethyl)-9-(4-(3-methoxypropylaminocarbonyl)phenylmethyl)-1,4,9-triazaspiro[5.5]undecane

Compound 75(57)

(3R)-1-butyl-2,5-dioxo-3-((1R)-1-hydroxy-1-cyclohexylmethyl)-9-(4-(N-methyl-N-(2-(pyridin-2-yl)ethyl)aminocarbonyl)phenylmethyl)-1,4, 9-triazaspiro[5.5]undecane

Compound 75(58)

(3R)-1-butyl-2,5-dioxo-3-((1R)-1-hydroxy-1-cyclohexylmethyl)-9-(4-(4-(pyrrolidin-1-ylcarbonyl)phenyloxy) phenylmethyl)-1,4,9-triazaspiro[5.5)undecane

Compound 75(59)

(3R)-1-butyl-2,5-dioxo-3-((1R)-1-hydroxy-1-cyclohexylmethyl)-9-(3,5-dimethyl-1-(4-chlorophenyl)pyrazol-4-ylmethyl)-1,4,9-triazaspiro[5.5]undecane

Compound 75(60)

(3R)-1-butyl-2,5-dioxo-3-((1R)-1-hydroxy-1-cyclohexylmethyl)-9-(3,5-dimethyl-1-(4-trifluoromethylphenyl) pyrazol-4-ylmethyl)-1,4,9-triazaspiro[5.5]undecane

Compound 75 (61)

(3R)-1-butyl-2,5-dioxo-3-((1R)-1-hydroxy-1-cyclohexylmethyl)-9-(3,5-dimethyl-1-(4-methoxyphenyl)pyrazol-4-ylmethyl)-1,4,9-triazaspiro[5.5]undecane

Compound 75(62)

(3R)-1-butyl-2,5-dioxo-3-((1R)-1-hydroxy-1-cyclohexylmethyl)-9-(3,5-dimethyl-1-ethylpyrazol-4-ylmethyl)-1,4,9-triazaspiro[5.5]undecane

Compound 75(63)

(3R)-1-butyl-2,5-dioxo-3-((1R)-1-hydroxy-1-cyclohexylmethyl)-9-(3,5-dimethyl-1-propylpyrazol-4-ylmethyl)-1,4,9-triazaspiro[5.5]undecane

Compound 75(64)

(3R)-1-butyl-2,5-dioxo-3-((1R)-1-hydroxy-1-cyclohexylmethyl)-9-(3,5-dimethyl-1-(1,1-dimethylethyl)pyrazol-4-ylmethyl)-1,4,9-triazaspiro[5.5]undecane

Compound 75(65)

(3R)-1-butyl-2,5-dioxo-3-((1R)-1-hydroxy-1-cyclohexylmethyl)-9-(3,5-dimethyl-1-cyclopentylpyrazol-4-ylmethyl)-1,4,9-triazaspiro[5.5]undecane

Compound 75(66)

(3R)-1-butyl-2,5-dioxo-3-((1R)-1-hydroxy-1-cyclohexylmethyl)-9-(3,5-dimethyl-1-(2-phenylethyl)pyrazol-4-ylmethyl)-1,4,9-triazaspiro[5.5]undecane

Compound 75(67)

(3R)-1-butyl-2,5-dioxo-3-((1R)-1-hydroxy-1-cyclohexylmethyl)-9-(3,5-dimethyl-1-(1-benzyloxycarbonylpiperidin-4-yl)pyrazol-4-ylmethyl)-1,4,9-triazaspiro[5.5)undecane

Compound 75(68)

(3R)-1-butyl-2, 5-dioxo-3-((1R)-1-hydroxy-1-cyclohexylmethyl)-9-(4-(cyclohexylaminocarbonyl)phenylmethyl)-1,4,9-triazaspiro[5.5)undecane

Compound 75(69)

(3R)-1-butyl-2,5-dioxo-3-((1R)-1-hydroxy-1-cyclohexylmethyl)-9-(3,5-dimethyl-1-(1-methylsulfonylpiperidin-4-y()pyrazol-4-ylmethyl)-1,4,9-triazaspiro[5.5]undecane

Compound 75(70)

(3R)-1-butyl-2,5-dioxo-3-((1R)-1-hydroxy-1-cyclohexylmethyl)-9-(4-(4-(2-hydroxyethylaminocarbonyl)phenyloxy)phenylmethyl)-1,4,9-triazaspiro[5.5]undecane

Compound 75(71)

(3R)-1-butyl-2,5-dioxo-3-((1R)-1-hydroxy-1-cyclohexylmethyl)-9-(4-(4-hydroxymethylphenyloxy)phenylmethyl)-1,4,9-triazaspiro[5.5]undecane

Compound 76

(3S)-1-butyl-2,5-dioxo-3-((1S)-1-hydroxy-1-cyclohexylmethyl)-9-(4-(4-methylsulfonylaminophenyloxy)phenylmethyl)-1,4,9-triazaspiro[5.5]undecane

Compound 77

(3R)-1-butyl-2,5-dioxo-3-((1R)-1-hydroxy-1-(3,4,5,6-tetrahydropyran-4-yl)methyl)-9-(3,5-dimethyl-1-phenylpyrazol-4-ylmethyl)-1,4,9-triazaspiro[5.5]undecane

Compound 77(1)

(3R)-1-butyl-2,5-dioxo-3-((1R)-1-hydroxy-1-(3,4,5,6-tetrahydropyran-4-yl)methyl)-9-(4-(4-methylaminocarbonylphenyloxy)phenylmethyl)-1,4,9-triazaspiro[5.5]undecane

Compound 77(2)

(3R)-1-butyl-2,5-dioxo-3-((1R)-1-hydroxy-1-(3,4,5,6-tetrahydropyran-4-yl)methyl)-9-(4-(4-(4-methoxyphenylmethylaminocarbonyl)phenylmethyl)-1,4,9-triazaspiro[5.5]undecane

Compound 77(3)

(3R)-1-butyl-2,5-dioxo-3-((1R)-1-hydroxy-1-(3,4,5,6-tetrahydropyran-4-yl)methyl)-9-(3,5-dimethyl-1-(4-(N,N-dimethylaminocarbonyl)phenylpyrazol-4-ylmethyl)-1,4,9-triazaspiro[5.5]undecane

Compound 77(4)

(3R)-1-butyl-2,5-dioxo-3-((1R)-1-hydroxy-1-(3,4,5,6-tetrahydropyran-4-yl)methyl)-9-(4-(4-carboxyphenyloxy)phenylmethyl)-1,4,9-triazaspiro[5.5]undecane

Compound 77(5)

(3R)-1-butyl-2,5-dioxo-3-((1R)-1-hydroxy-1-(3,4,5,6-tetrahydropyran-4-yl)methyl)-9-(4-(4-methylsulfonylaminophenyloxy)phenylmethyl)-1,4,9-triazaspiro[5.5]undecane

Compound 78

(3R)-1-butyl-2,5-dioxo-3-((1R)-1-hydroxy-1-cyclopentylmethyl)-9-(3,5-dimethyl-1-phenylpyrazol-4-ylmethyl)-1,4,9-triazaspiro[5.5]undecane

Compound 78(1)

(3R)-1-butyl-2,5-dioxo-3-((1R)-1-hydroxy-1-cyclopentylmethyl)-9-(4-(4-(4-methoxyphenylmethylaminocarbonyl)phenylmethyl)-1,4,9-triazaspiro[5.5]undecane

Compound 78(2)

(3R)-1-butyl-2,5-dioxo-3-((1R)-1-hydroxy-1-cyclopentylmethyl)-9-(4-(4-methylaminocarbonylphenyloxy) phenytmethyl)-1,4, 9-triazaspiro[5.5]undecane

Compound 78(3)

(3R)-1-butyl-2,5-dioxo-3-((1R)-1-hydroxy-1-cyclopentylmethyl)-9-(4-(4-carboxyphenyloxy)phenylmethyl)-1,4,9-triazaspiro[5.5]undecane

Compound 79

(3R)-1-propyl-2,5-dioxo-3-((1R)-1-hydroxy-2-methylpropyl)-9-(4-(4-methylaminocarbonylphenyloxy)phenylmethyl)-1,4,9-triazaspiro[5.5]undecane

Compound 79(1)

(3R)-1-propyl-2,5-dioxo-3-((1R)-1-hydroxy-2-methylpropyl)-9-(3,5-dimethyl-1-cyclohexylpyrazol-4-ylmethyl)-1,4,9-triazaspiro[5.5jundecane

Compound 79(2)

(3R)-1-propyl-2,5-dioxo-3-((1R)-1-hydroxy-2-methylpropyl)-9-(4-(4-methoxyphenylmethylaminocarbonyl) phenylmethyl)-1,4,9-triazaspiro[5.5]undecane

Compound 80

(3R)-1-propyl-2,5-dioxo-3-((1R)-1-hydroxy-1-cyclohexylmethyl)-9-(4-(4-methylaminocarbonylphenyloxy) phenylmethyl)-1,4,9-triazaspiro[5.5]undecane

Compound 80(1)

(3R)-1-propyl-2,5-dioxo-3-((1R)-1-hydroxy-1-cyclohexylmethyl)-9-(3,5-dimethyl-1-cyclohexylpyrazol-4-ylmethyl)-1,4,9-triazaspiro[5.5]undecane

Compound 80(2)

(3R)-1-propyl-2,5-dioxo-3-((1R)-1-hydroxy-1-cyclohexylmethyl)-9-(4-(4-methoxyphenylmethylaminocarbonyl)phenylmethyl)-1,4,9-triazaspiro[5.5]undecane

Compound 80(3)

(3R)-1-propyl-2,5-dioxo-3-((1R)-1-hydroxy-1-cyclohexylmethyl)-9-(3,5-dimethyl-1-(4-(N, N-dimethylaminocarbonyl)phenyl)pyrazol-4-ylmethyl)-1,4,9-triazaspiro[5.5]undecane

Compound 80(4)

(3R)-1-propyl-2,5-dioxo-3-((1R)-1-hydroxy-1-cyclohexylmethyl)-9-(4-(4-carboxyphenyloxy)phenylmethyl)-1,4,9-triazaspiro[5.5]undecane

Compound 80(5)

(3R)-1-propyl-2,5-dioxo-3-((1R)-1-hydroxy-1-cyclohexylmethyl)-9-(4-(4-methoxycarbonylphenyloxy)phenylmethyl)-1,4,9-triazaspiro[5.5]undecane

Compound 81

(3R)-1-propyl-2,5-dioxo-3-(1-cyctohexylmethylidene)-9-(4-(4-carboxyphenyloxy)phenylmethyl)-1,4,9-triazaspiro[5.5]undecane

Compound 82

(3S)-1-propyl-2,5-dioxo-3-(2-methylpropyl)-9-(3,5-dimethyl-1-cyclohexylpyrazol-4-ylmethyl)-1,4,9-triazaspiro[5.5]undecane

Compound 82(1)

(3S)-1-propyl-2,5-dioxo-3-(2-methylpropyl)-9-(4-(4-methylsulfonylaminophenyloxy)phenylmethyl)-1,4,9-triazaspiro[5.5]undecane

Compound 82(2)

(3S)-1-propyl-2,5-dioxo-3-(2-methylpropyl)-9-(3,5-dimethyl-1-(4-(2-(N,N-dimethylamino)ethylaminosulfonyl)phenyl)pyrazol-4-ylmethyl)-1,4,9-triazaspiro[5.5]undecane

Compound 82(3)

(3S)-1-propyl-2,5-dioxo-3-cyclohexylmethyl-9-(3,5-dimethyl-1-cyclohexylpyrazol-4-ylmethyl)-1,4,9-triazaspiro[5.5]undecane

Compound 82(4)

(3S)-1-propyl-2,5-dioxo-3-cyclohexylmethyl-9-(4-(4-methylsulfonylaminophenyloxy)phenylmethyl)-1,4,9-triazaspiro[5.5]undecane

Compound 82(5)

1-butyl-2,5-dioxo-9-(4-(4-methylsulfonylaminophenyloxy)phenylmethyl)-1,4,9-triazaspiro[5.5]undecane

Compound 82(6)

1-butyl-2,5-dioxo-9-(3,5-dimethyl-1-cyclohexylpyrazol-4-ylmethyl)-1,4,9-triazaspiro[5.5]undecane

Compound 83

(3R)-1-(2-butynyl)-2,5-dioxo-3-((1R)-1-hydroxy-1-cyclohexylmethyl)-9-(3,5-dimethyl-1-phenylpyrazol-4-ylmethyl)-1,4,9-triazaspiro[5.5]undecane

Compound 83(1)

(3S)-1-(2-butynyl)-2,5-dioxo-3-((1S)-1-hydroxy-1-cyclohexylmethyl)-9-(3,5-dimethyl-1-phenylpyrazol-4-ylmethyl)-1,4,9-triazaspiro[5.5]undecane

Compound 84

(3S)-1-butyl-2,5-dioxo-3-cyc(ohexylmethyl-9-(2-(4-phenyloxyphenyl)ethyl)-1,4,9-triazaspiro[5.5]undecane

Compound 84(1)

(3S)-1-butyl-2,5-dioxo-3-(2-methylpropyl)-9-(2-(4-phenyloxyphenyl)ethyl)-1,4,9-triazaspiro[5.5]undecane

Compound 84(2)

(3S)-1-butyl-2,5-dioxo-3-(2-methylpropyl)-9-(2-(4-methoxyphenyl)ethyl)-1,4,9-triazaspiro[5.5]undecane

Compound 85

(3S)-1-butyl-2,5-dioxo-3-cyclohexylmethyl-9-(4-ethoxycarbonylphenyl)-1,4,9-triazaspiro[5.5]undecane

Compound 86

(3S)-1-butyl-2,5-dioxo-3-cyclohexylmethyl-4-methyl-9-(4-phenyloxyphenylmethyl)-1,4,9-triazaspiro[5.5]undecane

Compound 87

(3R)-1-butyl-2,5-dioxo-3-((1R)-1-hydroxy-1-cyclohexylmethyl)-9-(4-(2-methylpropanoylamino)phenylmethyl)-1,4,9-triazaspiro[5.5]undecane

Compound 87(1)

(3R)-1-butyl-2,5-dioxo-3-((1R)-1-hydroxy-1-cyclohexylmethyl)-9-(4-(2-methoxyacetylamino)phenylmethyl)-1,4,9-triazaspiro[5.5]undecane

Compound 87(2)

(3R)-1-butyl-2,5-dioxo-3-((1R)-1-hydroxy-1-cyclohexylmethyl)-9-(4-(2-phenylacetylamino)phenylmethyl)-1,4,9-triazaspiro[5.5]undecane

Compound 87(3)

(3R)-1-butyl-2,5-dioxo-3-((1R)-1-hydroxy-1-cyclohexylmethyl)-9-(4-(2-(4-fluorophenyl)acetylamino)phenylmethyl)-1,4,9-triazaspiro[5.5]undecane

Compound 87(4)

(3R)-1-butyl-2,5-dioxo-3-((1R)-1-hydroxy-1-cyclohexylmethyl)-9-(4-(4-methoxycarbonylphenylaminocarbonyl)phenylmethyl)-1,4,9-triazaspiro[5.5]undecane

Compound 87(5)

(3R)-1-butyl-2,5-dioxo-3-((1R)-1-hydroxy-1-cyclohexylmethyl)-9-(4-(4-methoxyphenylmethyloxycarbonyl) phenylmethyl)-1,4,9-triazaspiro[5.5]undecane

Compound 87(6)

(3R)-1-butyl-2,5-dioxo-3-((1R)-1-hydroxy-1-cyclohexylmethyl)-9-(2-(4-methylaminocarbonylphenyloxy)pyridin-5-ylmethyl)-1,4,9-triazaspiro[5.5]undecane

Compound 88

(3S)-1-butyl-2,5-dioxo-3-((1S)-1-hydroxy-1-cyclohexylmethyl)-9-(4-(4-carboxyphenyloxy)phenylmethyl)-1,4,9-triazaspiro[5.5]undecane

Compound 89

(3S)-1-butyl-2,5-dioxo-3-(pyridin-3-ylmethyl)-9-(4-(4-methylaminocarbonylphenyloxy)phenylmethyl)-1,4,9-triazaspiro[5.5]undecane

Compound 89(1)

(3S)-1-butyl-2,5-dioxo-3-phenylmethyl-9-(4-(4-methylaminocarbonylphenyloxy)phenylmethyl)-1,4,9-triazaspiro[5.5)undecane

Compound 89(2)

(3S)-1-butyl-2,5-dioxo-3-(pyridin-2-ylmethyl)-9-(4-(4-methylaminocarbonylphenyloxy)phenylmethyl)-1,4,9-triazaspiro[5.5]undecane

Compound 89(3)

(3S)-1-butyl-2,5-dioxo-3-hydroxymethyl-9-(4-(4-methylaminocarbonylphenyloxy)phenylmethyl)-1,4,9-triazaspiro[5.5]undecane

Compound 89(4)

(3S)-1-butyl-2,5-dioxo-3-(pyridin-1-oxido-2-ylmethyl)-9-(4-(4-methylaminocarbonylphenyloxy)phenylmethyl)-1,4,9-triazaspiro[5.5]undecane

Compound 89(5)

(3S)-1-butyl-2,5-dioxo-3-(pyridin-1-oxido-3-ylmethyl)-9-(4-(4-methylaminocarbonylphenyloxy)phenylmethyl)-1,4,9-triazaspiro[5.5]undecane

Compound 90

(3R)-1-(4-methoxyphenylmethyl)-2,5-dioxo-3-((1R)-1-hydroxy-1-cyclohexylmethyl)-9-(4-(4-methylaminocarbonylphenyloxy)phenylmethyl)-1,4,9-triazaspiro[5.5]undecane

Compound 90(1)

(3R)-1-phenyimethyl-2,5-dioxo-3-((1R)-1-hydroxy-1-cyclohexylmethyl)-9-(4-(4-methylaminocarbonylphenyloxy)phenylmethyl)-1,4,9-triazaspiro[5.5]undecane

Compound 90(2)

(3R)-1-(2-methoxyethyl)-2,5-dioxo-3-((1R)-1-hydroxy-1-cyclohexylmethyl)-9-(4-(4-methylaminocarbonylphenyloxy)phenylmethyl)-1,4,9-triazaspiro[5.5]undecane

Compound 90(3)

(3R)-1-(pyridin-2-ylmethyl)-2,5-dioxo-3-((1R)-1-hydroxy-1-cyclohexylmethyl)-9-(4-(4-methylaminocarbonylphenyloxy)phenylmethyl)-1,4,9-triazaspiro[5.5]undecane

Compound 90(4)

(3R)-1-(pyridin-3-ylmethyl)-2,5-dioxo-3-((1R)-1-hydroxy-1-cyclohexylmethyl)-9-(4-(4-methylaminocarbonytphenyloxy)phenylmethyl)-1,4,9-triazaspiro[5.5]undecane

Compound 91

(3R)-1-butyl-2,5-dioxo-3-cyclohexylmethyl-9-(4-(4-carboxyphenyloxy)phenylmethyl)-1,4,9-triazaspiro[5.5]undecane

Compound 92

(3S)-1-butyl-2,5-dioxo-3-(pyridin-1-oxido-2-ylmethyl)-9-(4-(4-methylaminocarbonylphenyloxy)phenylmethyl)-1,4,9-triazaspiro[5.5]undecane·9-oxide

Compound 93

1-butyl-2,5-dioxo-3-(morpholin-4-ylmethyl)9-(4-(4-methylaminocarbonylphenyloxy)phenylmethyl)-1,4,9-triazaspiro[5.5]undecane

Compound 94

(3R)-1-butyl-2, 5-dioxo-3-((1R)-1-hydroxy-1-cyclohexylmethyl)-9-(4-(4-(N-hydroxycarbamoyl)phenyloxy)phenylmethyl)-1,4,9-triazaspiro[5.5]undecane

Compound 95

(3R)-1-butyl-2,5-dioxo-3-((1R)-1-hydroxy-1-cyclohexylmethyl)-9-(4-(4-methylaminocarbonylphenyloxy)phenylcarbonyl)-1,4,9-triazaspiro[5.5]undecane

Compound 96

(3R)-1-butyl-2,5-dioxo-3-((1R)-1-hydroxy-1-cyclohexylmethyl)-9-(4-(4-methylaminocarbonylphenyloxy)phenyl)-1,4,9-triazaspiro[5.5]undecane; however, the present invention is not limited thereto.
Moreover, particularly preferred compounds are Compound 3(1) and Compound 75(54), quaternary ammonium salts thereof, and N-oxides thereof, and non-toxic salts thereof.
Unless otherwise specified, all isomers are included in the present invention. For example, alkyl, alkoxy and alkylene groups include straight or branched ones. In addition, isomers on double bond, ring, fused ring (E-, Z-, cis-, trans-isomer), isomers generated from asymmetric carbon atoms (R-, S-, α-, β-isomer, enantiomer, diastereomer), optically active isomers (D-, L-, *d*-, *l*-isomer), polar compounds generated by chromatographic separation (more polar compound, less polar compound), equilibrium compounds, mixtures thereof, at voluntary ratios and racemic mixtures are also included in the present invention.
Non-toxic salts of the present invention include all pharmaceutically acceptable salts, for example, general salts, acid addition salts, and hydrates.
The compounds of the present invention of the formula (I) may be converted into the corresponding salts by conventional means. Non-toxic and watersoluble salts are preferred. Suitable salts, for example, include:

salts of alkali metals (potassium or sodium etc.), salts of alkaline earth metals (calcium or magnesium etc.), ammonium salts, and salts of pharmaceutically acceptable organic amines (tetramethylammonium, triethylamine, methylamine, dimethylamine, cyclopentylamine, benzylamine, phenethylamine, piperidine, monoethanolamine, diethanolamine, tris(hydroxymethyl)amine, lysine, arginine or N-methyl-D-glucamine etc.).

The compounds of the present invention of the formula (I) may be converted into the corresponding acid addition salts by conventional means. Watersoluble salts are preferred. Suitable salts for example, include: salts of inorganic acids e.g. hydrochloride, hydrobromide, sulfate, phosphate or nitrate; salts of organic acids e.g. acetate, trifluoroacetate, lactate, tartrate, oxalate, fumarate, maleate, citrate, benzoate, methanesulfonate, ethanesulfonate, benzenesulfonate, toluenesulfonate, isethionate, glucuronate or gluconate.
The compounds of the present invention of the formula (I) and salts thereof may be converted into the corresponding hydrates by conventional means.
Quaternary ammonium salts of the compounds of the formula (I) are the compounds where nitrogen atoms of the compounds of the formula (I) is quartemalized by $R^0$.

$R^0$ is C1-8 alkyl or C1-8 alkyl substituted by phenyl.

N-oxides of the compounds of the formula (I) are the compounds where nitrogen atoms of the compounds of the formula (I) are oxidized.

The concomitant medicament according to the present invention may be in the form of a concomitant administration or, in other words, a pharmaceutical composition containing a triazaspiro[5.5]undecane derivative, a quaternary ammonium salt thereof, or an N-oxide thereof, or a non-toxic salt thereof as an active ingredient and a pharmaceutical composition containing a CYP 3A4 inhibitor and/or a P-gp inhibitor are prepared separately and then administered. The concomitant administration includes simultaneous administration and administration with time interval. In the administration with time interval, the compound of the formula (I) may be firstly administered and then a pharmaceutical composition containing the CYP 3A4 inhibitor and/or the P-gp inhibitor as active ingredients may be administered. It is also possible that a pharmaceutical composition containing the CYP 3A4 inhibitor and/or the P-gp inhibitor as active ingredients is firstly administered and then the compound of the formula (I) is administered. Each of the administering methods may be the same or different.

Furthermore, the concomitant medicament according to the present invention may be in the form of a single pharmaceutical composition in which a pharmaceutical composition containing a triazaspiro[5.5]undecane derivative, a quaternary ammonium salt thereof, or an N-oxide thereof, or a non-toxic salt thereof and a pharmaceutical composition containing a CYP 3A4 inhibitor and/or a P-gp inhibitor are simultaneously contained. The administration route of the pharmaceutical composition is preferably to be an oral administration.

In the present invention, the CYP 3A4 inhibitor includes not only a drug which inhibits CYP 3A4 but also a drug which acts as a substrate for CYP 3A4 and is competitively metabolized by CYP 3A4. Examples include HIV protease inhibitors (e.g., indinavir, saquinavir, ritonavir, nelfinavir, amprenavir, lopinavir, *etc.*), non-nucleoside reverse transcriptase inhibitors (e.g., delavirdine, *etc.*), azole antifungal agents (e.g., ketoconazole, itraconazole, miconazole, *etc.),* macrolide antibiotics (e.g., erythromycin, clarithromycin, *etc.*), immunosuppressants (e.g., cyclosporine, tacrolimus, *etc.),* calcium channel blockers (e.g., diltiazem, verapamil, nifedipine, *etc.),* antihistaminics (e.g., cimetidine, astemizole, terfenadine, lovatidine, *etc.*), antiarrhythmics (e.g., amiodarone, lidocaine, quinidine, theophylline, disopyramide, propafenone, bepridil, *etc.*), antitumor agents (e.g., etoposide, flutamide, ifosfamide, toremifene, tamoxifen, *etc.*), benzodiazepine drugs (e.g., alprazolam, midazolam, triazolam, *etc.*), antidepressants (e.g., imipramine, buprenorphine, amitriptyline, clomipramine, *etc.*), sex hormones (e.g., testosterone, estradiol, progesterone, *etc.*), adrenocortical hormones (e.g., budesonide, cortisone, *etc.*), antihyperlipemic agents (e.g., simvastatin, lovastatin, atorvastatin, *etc.*), ergotamine preparations (e.g., dihydroergotamine, ergotamine, *etc.*), anesthetic analgesics (e.g., fentanyl, ethylmorphine, *etc.*), remedies for asthma (e.g., seratrodast, dextrin, *etc.*), remedies for peptic ulcer (e.g., omeprazole, lansoprazole, *etc.*), activation regulators for enteric movement (e.g., cisapride, *etc.*), agents for treating gout (e.g., colchicine, *etc.),* cardiotonics (e.g., digitoxin, *etc.*), anticoagulants (e.g., warfarin, *etc.*), analgesics (e.g., acetaminophen, *etc.*), antitussives and expectorants (e.g., codeine, *etc.*), agents for schizophrenia (e.g., pimozide, *etc.*), tranquilizers (e.g., diazepam, *etc.*), agents for Hansen's disease (e.g., diaphenylsulfone, *etc.*), agents for treating erectile failure (e.g., sildenafil, *etc.*) and other CYP 3A4 inhibitors (e.g., grapefruit juice, *etc.),* although not limited thereto. Names in the parentheses are generic names of the examples of the active ingredients and the CYP 3A4 inhibitors of the present invention also include non-toxic salts thereof.

In the present invention, the P-gp inhibitor includes not only a drug which inhibits P-gp but also a drug which acts as a substrate for P-gp and is competitively excreted by P-gp. Examples include HIV protease inhibitors (e.g., indinavir, saquinavir, ritonavir, nelfinavir, amprenavir, lopinavir, *etc.*), immunosuppressants (e.g., cyclosporine, tacrolimus, PSC 833, *etc.*), antitumor agents (e.g., tamoxifen, doxorubicin, daunorubicin, vinblastine, vincristine, actinomycin, paclitaxel, teniposide, etoposide, docetaxel, mitomycin, *etc.*), steroids (e.g., aldosterone, hydrocortisone, cortisol, corticosterone, dexamethasone, *etc.*), calcium channel blockers (e.g., diltiazem, verapamil, nifedipine, nicardipine, nitrendipine, felodipine, *etc.*), azole antifungal agents (e.g., ketoconazole, itraconazole, miconazole, *etc.*), new quinolone antibacterials (e.g., fleroxacin, enoxacin, levofloxacin, norfloxacin, *etc.*), sex hormones (e.g., testosterone, estradiol, progesterone, *etc.*), benzodiazepine drugs (e.g., midazolam, *etc.*), antihyperlipemic agents (e.g., lovastatin, atorvastatin, *etc.*), antihistaminics (e.g., terfenadine, *etc.*), cardiotonics (e.g., digoxin, *etc.*), antiarrhythmic agents (e.g., quinidine, amiodarone, lidocaine, propranolol, bepridil, *etc.*), antihypertensive agents (e.g., losartan, reserpine, *etc.*), coronary vasodilators (e.g., dipyridamole, *etc.*), tranquilizers (e.g., chlorpromazine, *etc.*), antidepressants (e.g., amitriptyline, *etc.*), antiemetic agents (e.g., ondansetron, domperidone), antidiarrheal agents (e.g., loperamide, *etc.*), narcotic analgesics (e.g., morphine, *etc.*), remedies for gout (e.g., colchicine, *etc.*), macrolide antibiotic substances (e.g., erythromycin, clarithromycin, *etc.*), other P-gp inhibitors (e.g., d-$\alpha$-tocopheryl polyethylene glycol 1000 succinate (hereinafter, may be referred to as TPGS), polyethylene glycol, poloxamer, polyethylene oxide, polyoxyethylene castor oil derivative, chrysin, (+)-taxifolin, naringenin, diosmin, quercetin, bergamottin, LY 335979, GG 918, reversin 121 and 205, rhodamine 123, *etc.*), although not limited thereto. Names in the parentheses are generic names of the examples of the active ingredients and the P-gp inhibitors of the present invention also include non-toxic salts thereof.

The CYP 3A4 inhibitors and/or the P-gp inhibitors used in the present invention include not only the compounds

which have been known already but also CYP 3A4 inhibitors and/or P-gp inhibitors which will be found in future. The CYP 3A4 inhibitors and/or the P-gp inhibitors used in the present invention also include those in the form of a non-toxic salt and in the form of a prodrug.

Among the CYP 3A4 inhibitors and/or the P-gp inhibitors, particularly preferred ones are HIV protease inhibitors (e.g., indinavir, saquinavir, ritonavir, nelfinavir, amprenavir, lopinavir, *etc.),* non-nucleoside reverse transcriptase inhibitors (e.g., delavirdine, *etc.*), azole antifungal agents (e.g., ketoconazole, itraconazole, miconazole, *etc.*) and non-toxic salts thereof.

Moreover, the most preferred ones are HIV protease inhibitors (e.g., indinavir, saquinavir, ritonavir, nelfinavir, amprenavir, lopinavir, *etc.*) and non-nucleoside reverse transcriptase inhibitors (e.g., delavirdine, *etc.)* having a therapeutic effect to AIDS caused by HIV infection.

The CYP 3A4 inhibitors and/or the P-gp inhibitors used in the present invention may be the same compounds or different ones.

Toxicity of the compound of the present invention is very low and is judged to be sufficiently safe for use as a medicament.

INDUSTRIAL APPLICABILITY

Application to pharmaceuticals:

The medicament according to the present invention is able to be utilized as a preventive and/or treating agent for various kinds of inflammatory diseases, asthma, atopic dermatitis, urticaria, allergic diseases (allergic bronchopulmonary aspergillosis, allergic eosinophilic gastroenteritis), nephritis, nephropathy, hepatitis, arthritis, chronic articular rheumatism, psoriasis, rhinitis, conjunctivitis, ischemia-reperfusion injury, multiple sclerosis, ulcerative colitis, acute respiratory distress syndrome, shock accompanied by bacterial infection, diabetes mellitus, autoimmune disease, transplant rejection, immunosuppression, metastasis of cancer, acquired immune deficiency syndrome and human immune deficiency virus infection.

Pharmaceutical preparation when the present invention is utilized as a concomitant medicament where pharmaceutical agents are administered separately

When a triazaspiro[5.5]undecane derivative, a quaternary ammonium salt thereof, or an N-oxide thereof, or a non-toxic salt thereof and a cytochrome P 450 isozyme 3A4 inhibitor and/or a P-glycoprotein inhibitor are used as separate pharmaceutical agents, they are usually administered systemically or locally and orally or parenterally. In that case, the form of each of the pharmaceutical agents may be same or different.

A dose may vary depending on age, body weight, symptom, therapeutic effect, administering method, treating time, and the like. Usually the dose in terms of the triazaspiro[5.5]undecane derivative which is an active ingredient is within a range of 1 mg to 1,000 mg for each administration for an adult once daily or several times a day orally or it is within a range of 0.1 mg to 100 mg for each administration for an adult once daily or several times a day parenterally (preferably by intravenous administration) or by means of a continuous administration into vein during 1 to 24 hours a day.

It goes without saying that, as described above, the dose varies depending on various conditions and, therefore, less doses than the above may be sufficient in some cases while, in other cases, more doses than the above may be necessary.

In the administration of the present compound, it is used as oral solid preparation and oral liquid preparation for oral administration and as injection, topical preparation, suppository, and the like for parenteral administration.

The oral solid preparation for oral administration includes tablets, pills, capsules, powders, granules, and the like. Capsules include hard capsules and soft capsules.

In the oral solid preparation as such, at least one active substance is used either *per* se or by mixing with excipients (e.g., lactose, mannitol, glucose, microcrystalline cellulose, starch, *etc.),* binders (e.g., hydroxypropyl cellulose, polyvinylpyrrolidone, magnesium metasilicate aluminate, *etc.*), disintegrating agents (e.g., calcium cellulose glycolate, *etc.),* lubricants (e.g., magnesium stearate, *etc.),* stabilizers, solubilizers (e.g., glutamic acid, aspartic acid, *etc.*), and the like, followed by making into a pharmaceutical preparation by the usual method. If necessary, the preparation may be coated with a coating agent (e.g., sugar, gelatin, hydroxypropyl cellulose, hydroxypropyl methylcellulose phthalate, *etc.*) or may be coated with two or more layers. Capsules of an absorbable substance such as gelatin is included as well.

The oral liquid preparation for oral administration includes pharmaceutically acceptable aqueous agents, suspensions, emulsions, syrups, elixirs, and the like. In such a liquid preparation, at least one active substance is dissolved, suspended or emulsified in a commonly used diluent (e.g., pure water, ethanol, a mixture thereof, *etc.*). The liquid

preparation may further contain moisturizers, suspending agents, emulsifiers, sweeteners, flavoring agents, aromatizing agents, preservers, buffers, and the like.

The injection preparation for parenteral administration includes solution, suspension, emulsion and solid injection preparation which is dissolved or suspended in a solvent upon use. The injection preparation is used by dissolving, suspending or emulsifying at least one active substance in a solvent. The solvent which may be used include distilled water for injection, physiological saline, vegetable oil, propylene glycol, polyethylene glycol, alcohol such as ethanol and a combination thereof. The injection preparation may further contain stabilizers, solubilizers (glutamic acid, aspartic acid, Polysolvate 80 (registered trademark), *etc.*), suspending agents, emulsifiers, analgesic agents, buffers, preservers, and the like. They are manufactured or prepared by sterilizing in the final step or by means of an aseptic operation. It is also possible that an aseptic solid agent such as a freeze-dried product is manufactured and, before use, it is dissolved in a sterilized or aseptic distilled water for injection or in other solvents and then used.

Other preparations for parenteral administration include topical liquid preparations, ointments, liniments, inhalants, sprays, powders, pessaries for intravaginal administration, and the like which contain at least one active substance and are formulated according to the common method.

In addition to the commonly used diluent, the spray agent may further contain stabilizers such as sodium hydrogen sulfite and buffers giving isotonic property such as sodium chloride, sodium citrate or citric acid. Method for the manufacture of the sprays is described in detail, for example, in U.S. Patents 2,868,691 and 3,095,355.

Preparation when the present invention is utilized as a single pharmaceutical composition

With regard to a single pharmaceutical composition of the present invention, other pharmaceutically acceptable carrier may be mixed, so long as it contains a triazaspiro[5.5]undecane derivative, a quaternary ammonium salt thereof, or an N-oxide thereof, or a non-toxic salt thereof and a cytochrome P 450 isozyme 3A4 inhibitor and/or a P-glycoprotein inhibitor as an active ingredient.

When a triazaspiro[5.5]undecane derivative, a quaternary ammonium salt thereof, or an N-oxide thereof, or a non-toxic salt thereof and a cytochrome P 450 isozyme 3A4 inhibitor and/or a P-glycoprotein inhibitor are used as a single pharmaceutical composition, it is preferred that the form is an oral preparation. Therefore, in order to use the pharmaceutical composition used in the present invention for treatment and/or prevention of the above-described diseases, it is usually administered systemically and orally.

A dose may vary depending on age, body weight, symptom, therapeutic effect, administering method, treating time, and the like. Usually the dose in terms of the triazaspiro[5.5]undecane derivative which is an active ingredient is within a range of 1 mg to 1,000 mg for each oral administration for an adult once daily or several times a day.

It goes without saying that, as described above, the dose varies depending on various conditions and, therefore, less doses than the above may be sufficient in some cases while, in other cases, more doses than the above may be necessary.

In the administration of the present compound, it is used as an internal solid preparation and an internal liquid preparation for oral administration.

The intemal solid preparation for oral administration includes tablets, pills, capsules, powders, granules, and the like. Capsules include hard capsules and soft capsules.

In the internal solid preparation, at least one active substance is used either *per se* or by mixing with excipients (e.g., lactose, mannitol, glucose, microcrystalline cellulose, starch, *etc.*), binders (e.g., hydroxypropyl cellulose, polyvinylpyrrolidone, magnesium metasilicate aluminate, *etc.*), disintegrating agents (e.g., calcium cellulose glycolate, *etc.*), lubricants (e.g., magnesium stearate, *etc.*), stabilizers, solubilizers (e.g., glutamic acid, aspartic acid, *etc.*), and the like, followed by making into pharmaceutical preparation by the usual method. If necessary, the preparation may be coated with a coating agent (e.g., sugar, gelatin, hydroxypropyl cellulose, hydroxypropyl methylcellulose phthalate, *etc.*) or may be coated with two or more layers. Capsules of an absorbable substance such as gelatin are also included.

The internal liquid preparation for oral administration includes pharmaceutically acceptable aqueous agents, suspensions, emulsions, syrups, elixirs, and the like. In the liquid preparation, at least one active substance is dissolved, suspended or emulsified in a commonly used diluent (e.g., pure water, ethanol, a mixture thereof, *etc.*). The liquid preparation may further contain moisturizers, suspending agents, emulsifiers, sweeteners, flavoring agents, aromatizing agents, preservers, buffers, and the like.

In the present invention, the mixing ratio of the compound of the formula (I) to the CYP 3A4 inhibitor and/or the P-gp inhibitor in the present invention is within such a range that "the compound of the formula (I)" : "the CYP 3A4 inhibitor and/or the P-gp inhibitor" is from 1 : 1000 to 1000 : 1. Preferably, they are mixed within such a ratio that "the compound of the formula (I)" : "the CYP 3A4 inhibitor and/or the P-gp inhibitor" is from 1 : 100 to 100 : 1 or, most preferably, they are mixed within such a ratio that "the compound of the formula (I)" : "the CYP 3A4 inhibitor and/or the P-gp inhibitor" is from 1 : 20 to 20 : 1.

The present invention is described in detail based on Examples; however, the present invention is not limited

thereto.

BEST MODE FOR CARRYING OUT THE INVENTION

The fact that, as a result of a use of the CYP 3A4 inhibitor and/or the P-gp inhibitor in combination with the tnazaspiro[5.5]undecane derivative represent by formula (I), rise of plasma concentration of the triazaspiro[5.5]undecane derivative of the formula (I) has been demonstrated by the following experiments.

Example 1

Estimation of metabolic CYP molecular species using human liver microsome and CYP inhibitor:

Metabolic reaction of a test compound in pooled human liver microsome (Zenotec) in the presence of a specific inhibitor for each CYP molecular species was investigated and the CYP molecular species participating in the metabolism was estimated from the effect of each inhibitor.

The following inhibitors were used as the specific inhibitors for CYP molecular species:

| | |
|---|---|
| α-Naphthoftavone (CYP 1A2 inhibitor) | 1 μmol/L (final concentration) |
| Sulfaphenazole (CYP 2C9 inhibitor) | 1 μmol/L (final concentration) |
| S-Mephenytoin (CYP 2C19 inhibitor) | 1 μmol/L (final concentration) |
| Quinidine (CYP 2D6 inhibitor) | 1 μmol/L (final concentration) |
| Ketoconazole (CYP 3A4 inhibitor) | 1 μmol/L (final concentration) |

A mixed solution containing 100 mmol/L of a phosphate buffer (pH 7.4), 0.05 mmol/L disodium ethylenediaminetetraacetate, 5 mmol /L magnesium chloride, 1 μmol/L of a test compound, 0.2 mg/L of human liver microsome (8 mg/L only for the measurement of a metabolism inhibiting rate by CYP 2C19) and each inhibitor (each of the numerals being the final concentration) was pre-incubated at 37°C, and then 2 mmol/L NADPH was added thereto to start the reaction. Immediately thereafter and after 60 minutes, 100 μL. of the sample was collected. The collected sample was immediately added to 2 mL of diethyl ether containing 400 μL of distilled water and 0.1 μg/L of an internal standard substance, followed by stirring, and the reaction was stopped. After a centrifugal treatment (2,000 rpm for 3 minutes), diethyl ether which was an upper layer was collected and dried *in vacuo.* The residue was dissolved in 150 μL of 0.01% TFA-80% acetonitrile and 40 μL thereof was subjected to a measurement. In this test, no quantitative determination was conducted but the residual rate to the concentration of the compound immediately after starting the reaction was determined from the area ratio in liquid chromatography/tandem mass analysis (LC/MS/MS) chromatogram.

<Results>

In the presence of specific inhibitors against various CYP, a metabolism reaction by human liver microsome was investigated and, as a result, metabolism of the test compound was significantly inhibited by ketoconazole which was a specific inhibitor for CYP 3A4 (Table 1).

Table 1

| Compound No. | Residual rate (%) | |
|---|---|---|
| | Ketoconazole (-) | Ketoconazole (+) |
| 3(1) | 32.1 | 89.1 |
| 40(70) | 28.2 | 104.0 |
| 75(53) | 19.4 | 114.4 |
| 75(54) | 34.4 | 95.3 |

However, in the inhibitors for molecular species other than CYP 3A4, metabolism of the test compound was hardly inhibited. The similar results were also confirmed for other compounds of the formula (I). Accordingly, it was suggested that the triazaspiro[5.5]undecane derivative of the formula (I) was mainly metabolized by CYP 3A4.

Example 2

Plasma concentration of the compound by intravenous administration in rat in the presence of CYP 3A4 inhibitor:

<Method>

Ketoconazole (50 mg/kg), an inhibitor against CYP 3A4, was intraperitoneally administered to male SD rats. One hour thereafter, the test compound (3 mg/kg) was intravenously administered. After 2, 5, 15, 30, 60, 120 and 240 minutes from the administration of the test compound, blood was collected from the rat jugular vein and centrifuged at 3,000 rpm for 10 minutes to obtain plasma. To 150 μL of the resulting plasma were added 5 ng of 2-[4-[4-(2-methoxyphenyl)-1-piperazinyl]butylamino]-5-ethoxycarbonyl-4,6-dimethylpyrimidine trihydrochloride as an internal standard, proteins were removed therefrom using ethanol and a plasma concentration of the test compound was measured by high-performance liquid chromatography. From the resulting plasma concentration of the test compound, the area under the curve of plasma concentration vs. time (AUC, μg·min/mL) was calculated.

<Results>

In tested Compound 3(1), the AUCs in the presence and absence of ketoconazole were 261.2 and 60.9 (μg·min/mL), respectively and, in the presence of ketoconazole, the AUC increased about 4 times as high as the case in the absence thereof. In addition, in tested Compound 75(54), the AUC also increased about 3 times as high as it. From the above, it is suggested that, when the CYP 3A4 inhibitor is used in combination with the triazaspiro[5.5] undecane derivative of the formula (I), plasma concentration of the triazaspiro[5.5]undecane derivative of the formula (I) increases.

Example 3

Measurement of membrane permeability of the P-gp expression cells:

<Methods>

Human MDR1 expressed cells (LLC-GA-5-COL 150) and parent cells thereof (LLC-PK 1) were incubated until arriving confluent in a 12-well transwell plate to prepare a single-layer membrane. Compound 75(54) was adjusted to 10 μM using a buffer for the measurement (M 199, 26.2 mM $NaHCO_3$, 10% FBS, 0.03% L-glutamine; pH 7.4). In measuring the membrane permeability from lumen to blood vessel, the drug solution was loaded to the lumen while, in measuring the membrane permeability from blood vessel to lumen, it was loaded to the blood vessel and incubation was carried out under conditions at 37°C, 5% $CO_2$ and relative humidity of 90%. After a certain period, the sample solution was collected and, in order to keep each volume, a buffer for the measurement was supplemented to an extent of the collected volume. Similarly, membrane permeability of Compound 75(54) was observed as well in the presence of cyclosporine A (abbreviated as CsA; final concentration: 10 μM) which is an inhibitor for P-gp. The measurement was carried out 3 times. All samples were evaluated by means of LS/MS/MS.

<Results>

The membrane permeability of Compound 75(54) from the lumen side was smaller in LLC-GA-5-COL 150 cells than in LLC-PK 1 cells and the membrane permeability from the blood vessel side was large. By addition of CsA which is a P-gp inhibitor, the membrane permeability from the lumen side increased in the LLC-GA-5-COL 150 cells and the membrane permeability from the blood vessel side decreased. However, in the LLC-PK 1 cells, there was no change in the membrane permeability even when CsA was added (Table 2).

Table 2

| | | Permeability (μL/$cm^2$/hr) | |
|---|---|---|---|
| | | From lumen to blood vessel | From blood vessel to lumen |
| PK 1 | CsA (-) | 35.3 | 18.8 |
| | CsA (+) | 41.9 | 20.8 |

Table 2 (continued)

| | | Permeability ($\mu L/cm^2/hr$) | |
|---|---|---|---|
| | | From lumen to blood vessel | From blood vessel to lumen |
| COL 150 | CsA (-) | 2.06 | 34.3 |
| | CsA (+) | 25.6 | 18.6 |

From the results, it was suggested that Compound 75(54) is a substrate for P-gp. In the cells where P-gp was expressed, discharge of the compound by P-gp to the lumen side decreased by CsA and transfer to the blood vessel was promoted. That is likely due to be the fact that function of taking-out of the compound to the lumen side was inhibited by the inhibition of P-gp existing in the lumen side of the cells. Accordingly, it was suggested that the plasma concentration of the triazaspiro[5.5]undecane derivative of the formula (I) rises when the P-gp inhibitor is used in combination with the triazaspiro[5.5]undecane derivative of the formula (I).

Example 4

Measurement of permeability of Caco-2 in the presence of P-gp inhibitor:

<Methods>

Caco-2 cells were incubated in a 12-well transwell place on a collagen-coated porous polycarbonate membrane until arriving confluent to prepare a single-layer membrane of Caco-2 cells. The single-layer membrane was preincubated for 30 minutes in a buffer for the measurement (a Hanks buffer containing 10 mM HEPES and 15 mM glucose; pH 7.0 ± 0.2) to which d-α-tocopheryl polyethylene glycol 1000 succinate, a P-gp inhibitor, was added. The drug concentration was adjusted to 10 µM by a buffer for the measurement. In measuring the membrane permeability from lumen to blood vessel, the drug solution was loaded to the lumen while, in measuring the membrane permeability from blood vessel to lumen, it was loaded to the blood vessel and incubation was carried out under conditions at 37°C, 5% $CO_2$ and relative humidity of 90%. After 1 and 2 hours from the administration of the drug, 200 µL of the buffer for the measurement containing the drug solution were recovered and substituted with a new buffer for the measurement. The measurement was carried out 2 times. Membrane permeability in a cell-free state was measured and used as a blank. As a control, permeability of Lucifer Yellow was measured. All samples were evaluated by LC/MS. A permeation coefficient ($P_{app}$) was calculated according to the following equation.

$$P_{app} = (dc / dt) \cdot V_r / (A \cdot C_0)$$

In the equation, dc/dt is a permeation rate after lag time; $V_r$ is a volume of the solution at the side of a donor; A is a surface area; and $C_0$ is the initial concentration of the solution at the side of the donor.

<Results>

As a result of addition of TPGS which is a P-gp inhibitor, a permeation coefficient of Compound 75(54) from the lumen side increased while that from the blood vessel side decreased (Table 3).

Table 3

| | Permeation Coefficient (cm/s) | |
|---|---|---|
| | From lumen to blood vessel | From blood vessel to lumen |
| 0.025% TPGS (-) | 3.42 | 32.4 |
| 0.025% TPGS (+) | 10.5 | 13 |

Thus, by TPGS, excretion of the compound outside the cells decreased and transfer to blood vessel was accelerated. It is likely that function of taking-out of the compound to the outside of the cells was inhibited by inhibition of P-gp existing at the side of lumen of the cells. Accordingly, it was suggested that plasma concentration of the triazaspiro[5.5]undecane derivative of the formula (I) rises when the P-gp inhibitor is used in combination with the triazaspiro[5.5]undecane derivative of the formula (I).

Example 5

Plasma concentration of the compound upon oral administration to rats in the presence of CYP 3A4 inhibitor:

<Methods>

Ketoconazole (50 mg/kg), a CYP 3A4 inhibitor, was intraperitoneally administered to male SD rats. One hour thereafter, 30 mg/kg of the test compound was orally administered. After 15, 30, 60, 120, 240 and 360 minutes from administration of the test compound, blood was collected from the rat jugular vein and centrifuged at 3,000 rpm for 10 minutes to give plasma. To 150 μL of the resulting plasma were added 5 ng of 2-[4-[4-(2-methoxyphenyl)-1-piperazinyl] butyl-amino]-5-ethoxycarbonyl-4,6-dimethylpyrimidine trihydrochloride, proteins were removed therefrom using ethanol and plasma concentration of the test compound was measured by high-performance liquid chromatography. From the concentration of the test compound in the resulting plasma, the area under curve of plasma concentration vs. time (AUC, μg•min/mL) was calculated.

<Results>

In tested Compound 75(54), the AUC increased in the presence of ketoconazole 10 times as high as the case in the absence thereof. From the above, it was suggested that oral absorption of the triazaspiro[5.5]undecane derivative of the formula (I) rises when the CYP 3A4 inhibitor is used in combination with the triazaspiro[5.5]undecane derivative.

Example 6

Plasma concentration of the compound upon oral administration to rats in the presence of an HIV protease inhibitor:

<Methods>

Amprenavir, nelfinavir, ritonavir or saquinavir (10 mg/kg) which is an HIV protease inhibitor and 10 mg/kg of the test compound were orally administered to male SD rats at the same time. After 15, 30, 60, 120, 240 and 360 minutes from the administration of the test compound, blood was collected from the rat jugular vein and centrifuged at 3,000 rpm for 10 minutes to give plasma. To 200 μL of the resulting plasma were added 50 ng of (3S)-1-butyl-2,5-dioxo-3-(2-methylpropyl)-9-)4-phenyloxyphenylmethyl)-1,4,9-triazaspiro[5.5]undecane as an internal standard. To this were added 300 μL of distilled water and 2,000 μL of diethyl ether, followed by well stirring, and the diethyl ether layer recovered therefrom was dried *in vacuo.* The dried substance was dissolved in 150 μL of a 50% acetonitrile solution containing 0.005% trifluoroacetic acid and subjected to a liquid chromatography/tandem mass analysis (LC/MS/MS) method to measure a plasma concentration of the test compound. From the concentration of the test compound in the resulting plasma, the area under curve of plasma concentration vs. time (AUC, μg·min/mL) was calculated.

<Results>

In tested Compound 75(54), the AUC increased 3.7 times, 3.4 times, 7.7 times and 1.7 times as high as amprenavir, nelfinavir, ritonavir and saquinavir, respectively in the presence of such an HIV protease inhibitor as compared with the case in the absence thereof. All of those HIV protease inhibitors have an CYP 3A4 inhibiting action and, from the above, it was suggested that oral absorption of the triazaspiro[5.5]undecane derivative of the formula (I) rises when the HIV protease inhibitor having a CYP inhibiting action is used in combination with the triazaspiro[5.5]undecane derivative of the formula (I).

## Claims

**1.** A pharmaceutical composition which comprises, as an active ingredient, a combination of a triazaspiro[5.5]undecane derivative of the formula (I)

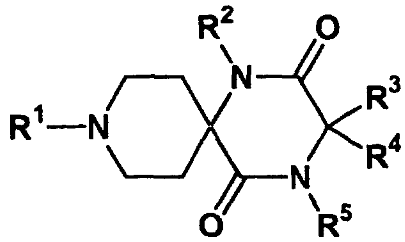

(I)

wherein $R^1$ is

(1) hydrogen,
(2) C1-18 alkyl,
(3) C2-18 alkenyl,
(4) C2-18 alkynyl,
(5) $-COR^6$,
(6) $-CONR^7R^8$,
(7) $-COOR^9$,
(8) $-SO_2R^{10}$,
(9) $-COCOOR^{11}$,
(10) $-CONR^{12}COR^{13}$,
(11) Cyc1, or
(12) C1-18 alkyl, C2-18 alkenyl or C2-18 alkynyl substituted by 1-5 of optionally selected from (a) halogen, (b) $-CONR^7R^8$, (c) $-COOR^9$, (d) $-OR^{14}$, (e) $-SR^{15}$, (f) $-NR^{16}R^{17}$, (g) $-NR^{18}COR^{19}$, (h) $-SO_2NR^{20}R^{21}$, (i) $-OCOR^{22}$, (j) $-NR^{23}SO_2R^{24}$, (k) $-NR^{25}COOR^{26}$, (l) $-NR^{27}CONR^{28}R^{29}$, (m) Cyc1, (n) keto or (o) $-N(SO_2R^{24})_2$,

$R^6$-$R^9$, $R^{11}$-$R^{21}$, $R^{23}$, $R^{25}$ and $R^{27}$-$R^{29}$ are each independently,

(1) hydrogen,
(2) C1-8 alkyl,
(3) C2-8 alkenyl,
(4) C2-8 alkynyl,
(5) Cyc1, or
(6) C1-8 alkyl, C2-8 alkenyl or C2-8 alkynyl substituted by 1-5 of optionally selected from (a) Cyc1, (b) halogen, (c) $-OR^{30}$, (d) $-SR^{31}$, (e) $-NR^{32}R^{33}$, (f) $-COOR^{34}$, (g) $-CONR^{35}R^{36}$, (h) $-NR^{37}COR^{38}$, (i) $-NR^{39}SO_2R^{40}$ or (j) $-N(SO_2R^{40})_2$, or

$R^7$ and $R^8$, $R^{20}$ and $R^{21}$, $R^{28}$ and $R^{29}$, taken together, are 1) C2-6 alkylene, 2) -(C2-6 alkylene)-O-(C2-6 alkylene)-, 3) -(C2-6 alkylene)-S-(C2-6 alkylene)-, or 4) -(C2-6 alkylene)-$NR^{195}$-(C2-6 alkylene)-, wherein $R^{195}$ is hydrogen, C1-8 alkyl, phenyl or C1-8 alkyl substituted by phenyl,

$R^{10}$, $R^{22}$, $R^{24}$ and $R^{26}$ are each independently,

(1) C1-8 alkyl,
(2) C2-8 alkenyl,
(3) C2-8 alkynyl,
(4) Cyc1, or
(5) C1-8 alkyl, C2-8 alkenyl or C2-8 alkynyl substituted by 1-5 of optionally selected from (a) Cyc1, (b) halogen, (c) $-OR^{30}$, (d) $-SR^{31}$, (e) $-NR^{32}R^{33}$, (f) $-COOR^{34}$, (g) $-CONR^{35}R^{36}$, (h) $-NR^{37}COR^{38}$, (i) $-NR^{39}SO_2R^{40}$ or (j) $-N(SO_2R^{40})_2$,

$R^{30}$-$R^{37}$ and $R^{39}$ are each independently, hydrogen, C1-8 alkyl, Cyc1 or C1-8 alkyl substituted by Cyc1, or

$R^{35}$ and $R^{36}$, taken together, are 1) C2-6 alkylene, 2) -(C2-6 alkylene)-O-(C2-6 alkylene)-, 3) -(C2-6 alkylene)-S-(C2-6 alkylene)- or 4) -(C2-6 alkylene)-$NR^{196}$-(C2-6 alkylene)-, wherein $R^{196}$ is hydrogen, C1-8 alkyl, phenyl or C1-8 alkyl substituted by phenyl,

$R^{38}$ and $R^{40}$ are each independently C1-8 alkyl, Cyc1 or C1-8 alkyl substituted by Cyc1,

Cyc1 is C3-15 mono-, bi- or tri-(fused or spiro)carbocyclic ring or 3-15 membered mono-, bi- or tri-(fused or spiro)cyclic hetero ring containing 1-4 nitrogen atoms, 1-3 oxygen atoms and/or 1-3 sulfur atoms, wherein Cyc1 may be optionally substituted by 1-5 of $R^{51}$,

$R^{51}$ is

(1) C1-8 alkyl,
(2) C2-8 alkenyl,
(3) C2-8 alkynyl,
(4) halogen,
(5) nitro,
(6) trifluoromethyl,
(7) trifluoromethoxy,
(8) nitrile,
(9) keto,
(10) Cyc2
(11) -$OR^{52}$,
(12) -$SR^{53}$,
(13) -$NR^{54}R^{55}$,
(14) -$COOR^{56}$,
(15) -$CONR^{57}R^{58}$,
(16) -$NR^{59}COR^{60}$,
(17) -$SO_2NR^{61}R^{62}$,
(18) -$OCOR^{63}$,
(19) -$NR^{64}SO_2R^{65}$,
(20) -$NR^{66}COOR^{67}$,
(21) -$NR^{68}CONR^{69}R^{70}$,
(22) -$B(OR^{71})_2$,
(23) -$SO_2R^{72}$,
(24) -$N(SO_2R^{72})_2$,
(25) -$S(O)R^{72}$, or
(26) C1-8 alkyl, C2-8 alkenyl or C2-8 alkynyl substituted by 1-5 of optionally selected from (a) halogen, (b) Cyc2, (c) -$OR^{52}$, (d) -$SR^{53}$, (e) -$NR^{54}R^{55}$, (f) -$COOR^{56}$, (g) -$CONR^{57}R^{58}$, (h) -$NR^{59}COR^{60}$, (i) -$SO_2NR^{61}R^{62}$, (j) -$OCOR^{63}$, (k) -$NR^{64}SO_2R^{65}$, (l) -$NR^{66}COOR^{67}$, (m) -$NR^{68}CONR^{69}R^{70}$, (n) -$B(OR^{71})_2$, (o) -$SO_2R^{72}$, (p) -$N(SO_2R^{72})_2$, (q) - $S(O)R^{72}$, or (r) keto,

$R^{52}$-$R^{52}$, $R^{64}$, $R^{66}$ and $R^{68}$-$R^{71}$ are each independently 1) hydrogen, 2) C1-8 alkyl, 3) C2-8 alkenyl, 4) C2-8 alkynyl, 5) Cyc2, 6) -$OR^{73}$, or 7) C1-8 alkyl, C2-8 alkenyl or C2-8 alkynyl substituted by Cyc2, -$OR^{73}$, -$COOR^{74}$, -$NR^{75}R^{76}$, or

$R^{57}$ and $R^{58}$, $R^{61}$ and $R^{62}$, $R^{69}$ and $R^{70}$, taken together, are 1) C2-6 alkylene, 2) -(C2-6 alkylene)-O-(C2-6 alkylene)-, 3) -(C2-6 alkylene)-S-(C2-6 alkylene)-, or 4) -(C2-6 alkylene)-$NR^{197}$-(C2-6 alkylene)-, wherein $R^{197}$ is hydrogen, C1-8 alkyl, phenyl or C1-8 alkyl substituted by phenyl,

$R^{63}$, $R^{65}$, $R^{67}$ and $R^{72}$ are each independently 1) C1-8 alkyl, 2) C2-8 alkenyl, 3) C2-8 alkynyl, 4) Cyc2 or 5) C1-8 alkyl, C2-8 alkenyl or C2-8 alkynyl substituted by Cyc2, -$OR^{73}$, -$COOR^{74}$ or -$NR^{75}R^{76}$,

$R^{73}$-$R^{76}$ are independently hydrogen, C1-8 alkyl, Cyc2 or C1-8 alkyl substituted by Cyc2,

Cyc2 has the same meaning as Cyc1, wherein Cyc2 may be optionally substituted by 1-5 of $R^{77}$,

$R^{77}$ is

1) C1-8 alkyl,
2) halogen,
3) nitro,
4) trifluoromethyl,
5) triftuoromethoxy,
6) nitrile,
7) -$OR^{78}$,
8) -$NR^{79}R^{80}$,
9) -$COOR^{81}$,
10) -$SR^{82}$,
11) -$CONR^{83}R^{84}$,
12) C2-8 alkenyl,
13) C2-8 alkynyl,
14) keto,

15) Cyc6,
16) $-NR^{161}COR^{162}$,
17) $-SO_2NR^{163}R^{164}$,
18) $-OCOR^{165}$,
19) $-NR^{166}SO_2R^{167}$,
20) $-NR^{168}COOR^{169}$,
21) $-NR^{170}COR^{171}R^{172}$,
22) $-SO_2R^{173}$,
23) $-N(SO_2R^{167})_2$,
24) $-S(O)R^{173}$, or
25) C1-8 alkyl, C2-8 alkenyl or C2-8 alkynyl substituted by 1-5 of optionally selected from (a) halogen, (b) $-OR^{78}$, (c) $-NR^{79}R^{80}$, (d) $-COOR^{81}$, (e) $-SR^{82}$, (f) $-CONR^{83}R^{84}$, (g) keto, (h) Cyc6, (i) $-NR^{161}COR^{162}$, (j) $-SO_2NR^{163}R^{164}$, (k) $-OCOR^{165}$, (l) $-NR^{166}SO_2R^{167}$, (m) $-NR^{168}COOR^{169}$, (n) $-NR^{170}COR^{171}R^{172}$, (o) $-SO_2R^{173}$, (p) $-N(O_2R^{167})_2$, or (q) $-S(O)R^{173}$,

$R^{78}$-$R^{84}$, $R^{161}$-$R^{164}$, $R^{166}$, $R^{168}$ and $R^{170}$-$R^{172}$ are each independently (a) hydrogen, (b) C1-8 alkyl, (c) C2-8 alkenyl, (d) C2-8 alkynyl, (e) Cyc6, (f) $-OR^{174}$, or (g) C1-8 alkyl, C2-8 alkenyl or C2-8 alkynyl substituted by Cyc6, $-OR^{174}$, $-COOR^{175}$,$-NR^{176}R^{177}$ or $-CONR^{178}R^{179}$, or

$R^{83}$ and $R^{84}$, $R^{163}$ and $R^{164}$, $R^{171}$ and $R^{172}$, taken together, are 1) C2-6 alkylene, 2) -(C2-6 alkylene)-O-(C2-6 alkylene)-, 3) -(C2-6 alkylene)-S-(C2-6 alkylene)-or 4) -(C2-6 alkylene)-$NR^{198}$-(C2-6 alkylene)-, wherein $R^{198}$ is hydrogen, C1-8 alkyl, phenyl or C1-8 alkyl substituted by phenyl,

$R^{165}$, $R^{167}$, $R^{169}$ and $R^{173}$ are each independently (a) C1-8 alkyl, (b) C2-8 alkenyl, (c) C2-8 alkynyl, (d) Cyc6 or (e) C1-8 alkyl, C2-8 alkenyl, C2-8 alkynyl substituted by Cyc6, $-OR^{174}$, $-COOR^{175}$, $-NR^{176}R^{177}$ or $-CONR^{178}R^{179}$,

$R^{174}$-$p^{177}$ are each independently 1) hydrogen, 2) C1-8 alkyl, 3) Cyc6 or 4) C1-8 alkyl substituted by Cyc6, or

$R^{178}$ and $R^{179}$, taken together, are 1) C2-6 alkylene, 2) -(C2-6 alkylene)-O-(C2-6 alkylene)-, 3) -(C2-6 alkylene)-S-(C2-6 alkylene)- or 4) -(C2-6 alkylene)-$NR^{199}$-(C2-6 alkylene)-, wherein $R^{199}$ is hydrogen, C1-8 alkyl, phenyl or C1-8 alkyl substituted by phenyl,

Cyc6 is C3-8 mono-carbocyclic ring or 3-8 membered mono-cyclic hetero ring containing 1-4 nitrogen atoms, 1-2 oxygen atoms and/or 1-2 sulfur atoms, so long as Cyc6 may be optionally substituted by 1-5 of $R^{180}$,

$R^{180}$ is,

(1) C1-8 alkyl,
(2) halogen,
(3) nitro,
(4) trifluoromethyl,
(5) trifluoromethoxy,
(6) nitrile,
(7) $-OR^{181}$,
(8) $-NR^{182}R^{183}$,
(9) $-COOR^{184}$,
(10) $-SR^{185}$, or
(11) $-CONR^{186}R^{187}$,

$R^{181}$-$R^{187}$ are each independently 1) hydrogen, 2) C1-8 alkyl, 3) phenyl or 4) C1-8 alkyl substituted by phenyl,

$R^{182}$ and $R^{183}$, $R^{186}$ and $R^{187}$, taken together, are 1) C2-6 alkylene, 2) -(C2-6 alkylene)-O-(C2-6 alkylene)-, 3) -(C2-6 alkylene)-S-(C2-6 alkylene)- or 4)-(C2-6 alkylene)-$NR^{200}$-(C2-6 alkylene)-, wherein $R^{200}$ is hydrogen, C1-8 alkyl, phenyl or C1-8 alkyl substituted by phenyl;

$R^2$ is

(1) hydrogen,
(2) C1-8 alkyl,
(3) C2-8 alkenyl,
(4) C2-8 alkynyl,
(5) $-OR^{90}$,
(6) Cyc3, or
(7) C1-8 alkyl, C2-8 alkenyl or C2-8 alkynyl substituted by 1-5 of optionally selected from (a) halogen, (b) $-OR^{90}$, (c) $-SR^{91}$, (d) $-NR^{92}R^{93}$, (e) $-COOR^{94}$, (f) $-CONR^{95}R^{96}$, (g) $-NR^{97}COR^{98}$, (h) $-SO_2NR^{99}R^{100}$, (i) $-OCOR^{101}$, (j) $-NR^{102}SO_2R^{103}$, (k) $-NR^{104}COOR^{105}$, (l) $-NR^{106}CONR^{107}R^{108}$, (m) Cyc3, (n) keto or (o) -N

$(SO_2R^{103})_2$,

$R^{90}$-$R^{100}$, $R^{102}$, $R^{104}$ and $R^{106}$-$R^{108}$ are each independently 1) hydrogen, 2) C1-8 alkyl, 3) C2-8 alkenyl, 4) C2-8 alkynyl, 5) Cyc3 or 6) C1-8 alkyl, C2-8 alkenyl or C2-8 alkynyl substituted by Cyc3, or

$R^{95}$ and $R^{96}$, $R^{99}$ and $R^{100}$, $R^{107}$ and $R^{108}$, taken together, are 1) C2-6 alkylene, 2) -(C2-6 alkylene)-O-(C2-6 alkylene)-, 3) -(C2-6 alkylene)-S-(C2-6 alkylene)-or 4) -(C2-6 alkylene)-NR $^{201}$ -(C2-6 alkylene)-,

$R^{201}$ is hydrogen, C1-8 alkyl, phenyl or C1-8 alkyl substituted by phenyl,

$R^{101}$, $R^{103}$ and $R^{105}$ are each independently 1) C1-8 alkyl, 2) C2-8 alkenyl, 3) C2-8 alkynyl or 4) Cyc3, or C1-8 alkyl, C2-8 alkenyl or C2-8 alkynyl substituted by Cyc3,

Cyc3 has the same meaning as Cyc1, wherein Cyc3 may be optionally substituted by 1-5 of $R^{109}$,

$R^{109}$ has the same meaning as $R^{51}$;

$R^3$ and $R^4$ are each independently

(1) hydrogen,
(2) C1-8 alkyl,
(3) C2-8 alkenyl,
(4) C2-8 alkynyl,
(5) $-COOR^{120}$,
(6) $-CONR^{121}R^{122}$,
(7) Cyc4, or
(8) C1-8 alkyl, C2-8 alkenyl or C2-8 alkynyl substituted by 1-5 of optionally selected from (a) halogen, (b) nitrile, (c) Cyc4, (d) $-COOR^{120}$, (e) $-CONR^{121}R^{122}$, (f) $-OR^{123}$, (g) $-SR^{124}$, (h) $-NR^{125}R^{126}$, (i) $-NR^{127}COR^{128}$, (j) $-SO_2NR^{129}R^{130}$, (k) $-OCOR^{131}$, (l) $-NR^{132}SO_2R^{133}$, (m) $-NR^{134}COOR^{135}$, (n) $-NR^{136}CONR^{137}R^{138}$, (o) $-S-SR^{139}$, (p) $-NHC(=NH)NHR^{140}$, (q) keto, (r) $-NR^{145}CONR^{146}COR^{147}$ or (s) $-N(SO_2R^{133})_2$,

$R^{120}$-$R^{130}$, $R^{132}$, $R^{134}$, $R^{136}$-$R^{138}$, $R^{145}$ and $R^{146}$ are each independently 1) hydrogen, 2) C1-8 alkyl, 3) C2-8 alkenyl, 4) C2-8 alkynyl, 5) Cyc4 or 6) C1-8 alkyl, C2-8 alkenyl or C2-8 alkynyl substituted by Cyc4, halogen, $-OR^{148}$, $-SR^{149}$, $-COOR^{150}$ or-$NHCOR^{141}$,

$R^{121}$ and $R^{122}$, $R^{129}$ and $R^{130}$, $R^{137}$ and $R^{138}$, taken together, are 1) C2-6 alkylene, 2) -(C2-6 alkylene) -O-(C2-6 alkylene)-, 3) -(C2-6 alkylene)-S-(C2-6 alkylene)-or 4) -(C2-6 alkylene)-$NR^{202}$-(C2-6 alkylene)-, wherein $R^{202}$ is hydrogen, C1-8 alkyl, phenyl or C1-8 alkyl substituted by phenyl,

$R^{131}$, $R^{133}$, $R^{135}$, $R^{139}$ and $R^{147}$ are each independently 1) C1-8 alkyl, 2) C2-8 alkenyl, 3) C2-8 alkynyl, 4) Cyc4 or 5) C1-8 alkyl, C2-8 alkenyl or C2-8 alkynyl substituted by Cyc4, halogen, $-OR^{148}$, $-SR^{149}$, $-COOR^{150}$ or $-NHCOR^{141}$,

$R^{140}$ is hydrogen, $-COOR^{142}$ or $-SO_2R^{143}$,

$R^{141}$-$R^{143}$ are each independently 1) C1-8 alkyl, 2) C2-8 alkenyl, 3) C2-8 alkynyl, 4) Cyc4 or 5) C1-8 alkyl, C2-8 alkenyl or C2-8 alkynyl substituted by Cyc4,

$R^{148}$-$R^{150}$ are each independently 1) hydrogen, 2) C1-8 alkyl, 3) C2-8 alkenyl, 4) C2-8 alkynyl, 5) Cyc4 or 6) C1-8 alkyl, C2-8 alkenyl or C2-8 alkynyl substituted by Cyc4,

Cyc4 has the same meaning as Cyc1, wherein Cyc4 may be optionally substituted by 1-5 of $R^{144}$,

$R^{144}$ has the same meaning as $R^{51}$, or

$R^3$ and $R^4$, taken together, are

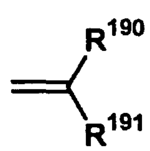

wherein $R^{190}$ and $R^{191}$ are each independently the same meaning as $R^3$ or $R^4$;

$R^5$ is

(1) hydrogen,
(2) C1-8 alkyl,
(3) Cyc5, or
(4) C1-8 alkyl substituted by Cyc5

wherein Cyc5 has the same meaning as Cyc1, and wherein Cyc5 is optionally substituted by 1-5 of $R^{160}$,

$R^{160}$ has the same meaning as $R^{51}$;
a quaternary ammonium salt thereof, an N-oxide thereof, or a non-toxic salt thereof, and
at least one of cytochrome P 450 isozyme inhibitors and / or at least one of P glycoproteins,

**2.** The pharmaceutical composition comprising the combination according to claim 1, wherein the triazaspiro[5.5] undecane derivative, a quaternary ammonium salt thereof, or an N-oxide thereof, or a non-toxic salt thereof and at least one cytochrome P450 isozyme 3A4 inhibitor and/or at least one P-glycoprotein inhibitor as active ingredients are administered as separate pharmaceutical agents.

**3.** The pharmaceutical composition comprising the combination according to claim 1, wherein the thazaspiro[5.5] undecane derivative, a quaternary ammonium salt thereof, or an N-oxide thereof, or a non-toxic salt thereof and at least one cytochrome P450 isozyme 3A4 inhibitor and/or at least one P-glycoprotein inhibitor as active ingredients are contained in a single pharmaceutical preparation.

**4.** The pharmaceutical composition according to claim 1, wherein the cytochrome P 450 isozyme 3A4 inhibitor described in claim 1 is a drug which inhibits cytochrome P 450 isozyme 3A4 and a drug which is to be a substrate for the cytochrome P 450 isozyme 3A4.

**5.** The pharmaceutical composition according to claim 1, wherein the drug which inhibits cytochrome P 450 isozyme 3A4 or the drug which is to be a substrate for the cytochrome P 450 isozyme 3A4 described in claim 4 is an HIV protease inhibitor, a non-nucleoside reverse transcriptase inhibitor, an azole antifungal agent, a macrolide antibiotic, an immunosuppressant, a calcium channel blocker, an antihistaminic, an antiarrhythmic, an antitumor agent, a benzodiazepine drug, an antidepressant, a sex hormone, an adrenocortical hormone, an antihyperlipemic agent, an ergotamine preparation, an anesthetic analgesics, a remedy for asthma, a remedy for peptic ulcer, an activation regulator for enteric movement, a remedy for gout, cardiotonics, an anticoagulant, an analgesic, an antitussive/ expectorant, an agent for schizophrenia, a tranquilizer, an agent for Hansen's disease, a remedy for erectile failure or grapefruit juice.

**6.** The pharmaceutical composition according to claim 5,
wherein the HIV protease inhibitor is indinavir, saquinavir, ritonavir, nelfinavir, amprenavir or lopinavir;
the non-nucleoside reverse transcriptase inhibitor is delavirdine;
the azole antifungal is ketoconazole, itraconazole or miconazole;
the macrolide antibiotic is erythromycin or clarithromycin;
the immunosuppressant is cyclosporine or tacrolimus;
the calcium channel blocker is diltiazem, verapamil or nifedipine;
the antihistaminic is cimetidine, astemizole, terfenadine or lovatidine;
the antiarrhythmic is amiodarone, lidocaine, quinidine, theophylline, disopyramide, propafenone or bepridil;
the antitumor agent is etoposide, flutamide, ifosfamide, toremifene or tamoxifen;
the benzodiazepine drug is alprazolam, midazolam or triazolam;
the antidepressant is imipramine, buprenorphine, amitriptyline or clomipramine;
the sex hormone is testosterone, estradiol or progesterone;
the adrenocortical hormone is budesonide or cortisone;
the antihyperlipemic agent is simvastatin, lovastatin or atorvastatin:
the ergotamine preparation is dihydroergotamine or ergotamine;
the anesthetic analgesic is fentanyl or ethylmorphine;
the remedy for asthma is seratrodast or dextrin;
the remedy for peptic ulcer is omeprazale or lansoprazole;
the activation regulator for enteric movement is cisapride;
the remedy for gout is colchicine;
the cardiotonic is digitoxin;
the anticoagulant is warfarin;
the analgesic is acetaminophen;
the antitussive/expectorant is codeine; and
the agent for schizophrenia is pimozide;
the tranquilizer is diazepam;
the agent for Hansen's disease is diaphenylsulfone;
the remedy for erectile failure is sildenafil; or
other cytochrome P 450 isozyme 3A4 inhibitor is grapefruit juice.

7. The pharmaceutical composition according to claim 1, wherein the P-glycoprotein inhibitor described in claim 1 is a drug which inhibits P-glycoprotein and a drug which is to be a substrate for the P-glycoprotein.

8. The pharmaceutical composition according to claim 1, wherein the drug which inhibits P-glycoprotein or the drug which is to be a substrate for the P-glycoprotein described in claim 7 is an HIV protease inhibitor, an immunosuppressant, an antitumor agent, a steroid, a calcium channel blocker, an azole antifungal agent, a new quinolone antibacterial, a sex hormone, a benzodiazepine drug, an antihyperlipemic agent, an antihistaminic, a cardiotonic, an antiarrhythmic agent, an antihypertensive agent, a coronary vasodilator, a tranquilizer, an antidepressant, an antiemetic agent, an antidiarrheal agent, a narcotic analgesic, a remedy for gout, a macrolide antibiotic substance, a d-α-tocopheryl polyethylene glycol 1000 succinate, a polyethylene glycol, a poloxamer, polyethylene oxide, a polyoxyethylene castor oil derivative, a chrysin, a (+)-taxifolin, a naringenin, diosmin, a quercetin, a bergamottin, LY 335979, GG 918, reversin 121 or 205, rhodamine 123 or a non-toxic salt thereof.

9. The pharmaceutical composition according to claim 8,
wherein the HIV protease inhibitor is indinavir, saquinavir, ritonavir, nelfinavir, amprenavir or lopinavir;
the immunosuppressant is cyclosporine, tacrolimus or PSC 833;
the antitumor agent is tamoxifen, doxorubicin, daunorubicin, vinblastine, vincristine, actinomycin, paclitaxel, teniposide, etoposide, docetaxel or mitomycin;
the steroid is aldosterone, hydrocortisone, cortisol, corticosterone or dexamethasone;
the calcium channel blocker is diltiazem, verapamil, nifedipine, nicardipine, nitrendipine or felodipine;
the azole antifungal is ketoconazole, itraconazole or miconazole;
the new quinolone antibacterial is fleroxacin, enoxacin, levofloxacin or norfloxacin;
the sex hormone is testosterone, estradiol or progesterone;
the benzodiazepine drug is midazolam;
the antihyperlipemic agent is lovastatin or atorvastatin;
the antihistaminic is terfenadine;
the cardiotonic is digoxin;
the antiarrhythmic agent is quinidine, amiodarone, lidocaine, propranolol or bepridil;
the antihypertensive agent is losartan or reserpine;
the coronary vasodilator is dipyridamole;
the tranquilizer is chlorpromazine;
the antidepressant is amitriptyline;
the antiemetic agent is ondansetron or domperidone;
the antidiarrheal agent is loperamide;
the narcotic analgesic is morphine;
the remedy for gout is colchicine;
the macrolide antibiotic substance is erythromycin or clarithromycin; or
other P-gp inhibitor is a d-α-tocopheryl polyethylene glycol 1000 succinate, a polyethylene glycol, a poloxamer, a polyethylene oxide, a polyoxyethylene castor oil derivative, chrysin, (+)-taxifolin, naringenin, diosmin, quercetin, bergamottin, LY 335979, GG 918, reversin 121 or 205, or rhodamine 123.

10. An agent for preventing and/or treating various inflammatory diseases, asthma, atopic dermatitis, urticaria, allergic diseases (allergic bronchopulmonary aspergillosis, allergic eosinophilic gastroenteritis), nephritis, nephropathy, hepatitis, arthritis, chronic articular rheumatism, psoriasis, rhinitis, conjunctivitis, ischemia-reperfusion injury, multiple sclerosis, ulcerative colitis, acute respiratory distress syndrome, shock accompanied by bacterial infection, diabetes mellitus, autoimmune disease, transplant rejection, immunosuppression, metastasis of cancer or acquired immune deficiency syndrome, which comprises the pharmaceutical composition according to claim 1.

11. An agent for preventing and/or treating human immune deficiency virus infection, which comprises the pharmaceutical composition according to claim 1.

## INTERNATIONAL SEARCH REPORT

International application No.
PCT/JP02/02552

A. CLASSIFICATION OF SUBJECT MATTER
Int.Cl[7] A61K31/499, 31/5377, 45/00, C07D471/10, A61P43/00

According to International Patent Classification (IPC) or to both national classification and IPC

B. FIELDS SEARCHED

Minimum documentation searched (classification system followed by classification symbols)
Int.Cl[7] A61K31/499, 31/5377, 45/00, C07D471/10, A61P43/00

Documentation searched other than minimum documentation to the extent that such documents are included in the fields searched

Electronic data base consulted during the international search (name of data base and, where practicable, search terms used)
CAPLUS(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| Y | WO 01/40227 A1 (Ono Pharmaceutical Co., Ltd.), 07 June, 2001 (07.06.01), (Family: none) | 1-11 |
| Y | MAEDA, K. et al., Novel Low Molecular Weight Spirodiketopiperazine Dericatives Potently Inhibit R5 HIV-1 Infection through Their Antagonistic Effects on CCR5, Journal of Biological Chemistry, September 2001, Vol.276, No.37, pages 35194 to 35200 | 1-11 |
| Y | WO 95/20980 A1 (The Regents of the University of California), 10 August, 1995 (10.08.95),Full text & AU 9515629 A & US 5567592 A & EP 742722 A1 & JP 9-508623 A & US 6004927 A & US 6028054 A & EP 1127579 A | 1-11 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 06 June, 2002 (06.06.02) | 18 June, 2002 (18.06.02) |
| Name and mailing address of the ISA/ Japanese Patent Office | Authorized officer |
| Facsimile No. | Telephone No. |

Form PCT/ISA/210 (second sheet) (July 1998)